# EUROPEAN PATENT SPECIFICATION

(11) **EP 1 529 107 B1**
(45) Date of publication and mention of the grant of the patent: **17.10.2012**
(21) Application number: 03813037.3
(22) Date of filing: 21.03.2003
(51) Int. Cl.: C07K 14/175, A61K 39/42

(54) **DNA VACCINES AGAINST HANTAVIRUS INFECTIONS**
DNA-IMPFSTOFFE GEGEN HANTAVIRUSINFEKTIONEN
VACCINS A ADN DIRIGES CONTRE DES INFECTIONS D'HANTAVIRUS

(30) Priority: 22.03.2002 US 367128 P; 26.07.2002 US 398985 P
(43) Date of publication of application: 11.05.2005
(73) Proprietor: U.S. Army Medical Research Institute of Infectious Diseases; Department of the Army, Frederick, MD 21702 (US)
(72) Inventor: HOOPER, Jay, W., New Market, MD 21774 (US); SCHMALJOHN, Connie, S., Frederick, MD 21702 (US); CUSTER, Max, Durham, NC 27713 (US)
(74) Representative: Icely, Dominic Michael
(86) International application number: PCT/US2003/008810
(87) International publication number: WO 2004/058808

(56) References cited:
- HOOPER J W ET AL: "DNA vaccination with the Hantaan virus M gene protects hamsters against three of four HFRS hantaviruses and elicits a high-titer neutralizing antibody response in rhesus monkeys" JOURNAL OF VIROLOGY, vol. 75, no. 18, September 2001 (2001-09), pages 8469-8477, XP002296288 ISSN: 0022-538X
- DATABASE GENESEQ EBI, HINXTON, CAMBRIDGESHIRE, U.K.; 8 November 2000 (2000-11-08), SCHMALJOHN CS & HOOPER JW: "pWRG/SEO-M vaccine construct." XP002296289 Database accession no. AAA47796 -& WO 00/44406 A (SCHMALJOHN CONNIE S ; HOOPER J W (US); US MED RES INST INFECT DISEASE) 3 August 2000 (2000-08-03)

## Description

### INTRODUCTION

Currently, there are four known hantaviruses associated with hemorrhagic fever with renal syndrome (HFRS): Hantaan virus (HTNV), Dobrava-Belgrade virus (DOBV), Puumala virus (PUUV), and Seoul virus (SEOV). Because distinct hantaviruses are usually carried by only one principal rodent host species, their distribution is generally limited to the range of that host (reviewed in Schmaljohn and Hjelle, 1997, Emerg. Infect. Dis. 3, 95-104). HTNV, carried by *Apodemus agrarius*, is found in Asia; DOBV, carried by *Apodemus flavicollis*, and PUUV, carried by *Clethrionomys glareolus*, are found in Europe. SEOV is more widely disseminated than any other recognized hantavirus because its host, the common urban rat (*Rattus norvegicus*), is found throughout the world.

New-World hantaviruses have been associated with outbreaks of a highly lethal disease, hantavirus pulmonary syndrome (HPS), in the Americas (reviewed in Schmaljohn and Hjelle, 1997, Emerg. Infect Dis. 3, 95-104). The disease is characterized by fever and vascular leakage resulting in non-cardiogenic pulmonary edema followed by shock. Case-fatality for HPS caused by the most prevalent North American and
South American hantaviruses, Sin Nombre virus (SNV) and Andes virus (ANDV), respectively is 30-50%.

Viruses in the Hantavirus genus (family Bunyaviridae) are enveloped and contain a genome comprised of three single-stranded RNA segments designated large (L), medium (M), and small (S) based on size (reviewed in Schmaljohn, 1996, In The Bunvaviridae Ed. R. M. Elliott. New York, Plenum Press p. 63-90). The hantavirus L segment encodes the RNA dependent RNA polymerase, M encodes two envelope glycoproteins (G1 and G2), and S encodes the nucleocapsid protein (N).

A number of inactivated HFRS vaccines derived from cell culture or rodent brain were developed and tested in Asia (Lee et al., 1990, Arch. Virol., Suppl. 1, 35-47; Song et al., 1992, Vaccine 10, 214-216; Lu et al., 1996, J. Med. Virol. 49, 333-335). Drawbacks of these traditional killed-virus vaccines include a requirement for appropriate containment for the growth and manipulation of virus. In order to overcome these drawbacks, vaccine approaches involving recombinant DNA technology were developed including: vaccinia-vectored vaccines (Schmaljohn et al. 1990, J. Virol. 64, 3162-3170; Schmaljohn et al. 1992, Vaccine 10, 10-13; Xu et al. 1992, Am. J. Trop. Med. Hyg. 47, 397-404), protein subunit vaccines expressed in bacteria or insect cells (Schmaljohn et *al.* 1990, supra; Yoshimatsu et al., 1993, Arch. Virol. 130, 365-376; Lundkvist et al., 1996, Virology 216, 397-406), and a hepatitis core antigen-based recombinant vaccine (Ulrich et al., 1998, Vaccine 16, 272-280).

Vaccination with vaccinia recombinants expressing the M segment of either HTNV or SEOV elicited neutralizing antibodies and protected rodents against infection with both HTNV and SEOV, suggesting that an immune response to G1-G2 alone can confer protection (Schmaljohn *et al.* 1990, supra; Xu *et al*. 1992, supra; Chu et al. 1995, J. Virol. 69, 6417-6423). Similarly, vaccination with G1-G2 protein expressed in insect cells (baculovirus recombinant virus system) elicited neutralizing antibodies and protected hamsters from infection with HTNV (Schmaljohn *et al.* 1990, supra). In both the vaccinia and baculovirus systems, vaccination with G1-G2 provided more complete protection than G1 or G2 alone (Schmaljohn *et al.* 1990, supra). Neutralizing antibody responses to G1-G2 in the aforementioned vaccine studies correlated with protection, suggesting that neutralizing antibodies play an important role in preventing hantavirus infection. Passive transfer of neutralizing monoclonal antibodies (MAbs) specific to either G1 or G2 protected hamsters against HTNV infection (Schmaljohn *et al*., 1990, supra; Arikawa et al., 1992, J. Gen. Virol. 70, 615-624), supporting the idea that neutralizing antibodies alone can confer protection.

The N protein also plays a role in protecting against hantavirus infection. Vaccination with N expressed in bacteria, insect cells, or as chimeric hepatitis B virus (HBV) core particles protected rodents from hantavirus infection (Schmaljohn *et al*., 1990, supra; Yoshimatsu *et al*. 1993, supra; Lundkvist *et al*., 1996, supra; Ulrich *et al*., 1998, supra). Vaccination with vaccinia recombinants expressing the S segment were less conclusive. A construct expressing the HTNV S segment did not protect hamsters from HTNV infection, possibly due to low N expression levels (Schmaljohn *et al*. 1990, supra); and a construct expressing the S segment of SEOV protected three of four gerbils from SEOV infection (Xu *et al*. 1992, supra).

Similarly, basic research towards a gene-based vaccine that protects against HPS has been ongoing since the isolation of the first HPS-associated hantavirus in the mid 1990s. There are reports that candidate DNA vaccines comprised of around 500 nucleotide stretches of the SNV M gene, or the full-length S gene, are immunogenic in mice (Bharadwaj, et al., 1999, Vaccine 17, 2836,43) and conferred some protection against infection with SNV in a deer mouse infection model (Bharadwaj, et al., 2002, J. Gen. Virol. 83, 1745-1751). The protection was surmised to be cell-mediated because there was no convincing evidence that these constructs elicited a neutralizing, or otherwise protective, antibody response.

Therefore, it remains unclear whether or not G1 alone, G2 alone, or fragments of the glycoproteins can elicit neutralizing antibody and protect against infection. Vaccination with recombinant baculovirus-infected cell lysates containing G1 or G2 alone, and recombinant vaccinia viruses expressing G1 or G2 alone, failed to elicit neutralizing antibody, and exhibited incomplete protection in a hamster infection model (Schmaljohn *et al*., 1990). Even though these vaccinia vaccines showed some potential, recombinant vaccinia virus vaccines and vaccinia-based vaccines present disadvantages including the potential for disseminated infection, especially in immunocompromised individuals, since the vaccines consist of live virus. Also, vaccination with these viruses can result in a lesion (pock) that contains infectious virus. Virus from these lesions can be inadvertently spread to other sites (e.g., eyes) or to other individuals. In addition, vaccinia-vectored vaccine are poorly immunogenic in persons previously vaccinated with smallpox vaccine (McClain et al., 2000, J. Med. Virol. 60, 77-85). Other drawbacks of vaccinia-based vaccines include discomfort due to swollen lymphnodes and scarring at the site of inoculation.

### SUMMARY OF THE INVENTION

The invention provides an isolated nucleotide sequence selected from the group consisting of: nucleotides 1-8 of SEQ ID NO. 6; nucleotides 1-12 of SEQ ID NO. 6; and nucleotides 1-24 of SEQ ID NO. 6. Also provided is a recombinant DNA construct comprising: (i) a vector; (ii) at least one hantavirus Hantaan M gene nucleic acid fragment; and (iii) the present sequence; as well as an immunogenic composition against hantavirus infection, comprising a nucleic acid comprising a promoter operative in the cells of a mammal, the present nucleotide sequence, and a hantavirus polynucleotide M segment encoding G1 and G2 from the Hantaan hantavirus and, optionally, further hantaviruses. A vaccine against hantavirus infection comprising said construct or said composition is also provided. The vaccine may be a multivalent vaccine for protection against infection with more than one hemorrhagic fever with renal syndrome (HFRS) hantavirus. Finally, the invention provides the nucleotide sequence for use in inducing a protective immune response against Hantaan hantavirus infection in a mammal.

WO 00/44406, also by the present Applicants, describes DNA vaccines against Seoul hantavirus infections. The plasmid pWGR/SEO-M is disclosed which consists of 8001 nucleotides. This includes nucleotides 1-12 of SEQ ID NO. 6, but no function is ascribed to this sequence. We have, surprisingly, shown that a non-coding region consisting of nucleotides 1-8, 1-12 or 1-24 of SEQ ID NO. 6 is critical to expression of the G1 glycoprotein. Accordingly, we describe herein a new recombinant DNA vaccine approach that involves vaccination with naked DNA expressing individual hantavirus genome segment cDNAs. Naked DNA vaccination involves delivery of plasmid DNA constructs with a gene(s) of interest into the tissue of the vaccinee (reviewed in Robinson and Torres, 1997, Semin. Immunol. 9, 271-283; and Gregoriadis, 1998, Pharm. Res. 15, 661-670).
Torres, 1997, Semin. Immunol. 9, 271-283; and Gregoriadis, 1998, Pharm. Res. 15, 661-670).

This vaccine approach is advantageous over subunit vaccines which do not elicit a cytotoxic response necessary to prevent the establishment of infection or disease. DNA vaccination mimicks the de novo antigen production and MHC class I-restricted antigen presentation obtainable with live vaccines, without the risks of pathogenic infection. Also, this DNA vaccine approach allows delivery to mucosal tissues which may aid in conferring resistance to viral introduction since entry of the virus may be through mucosal tissues.

The gene(s) of interest, in our case, a hantavirus genome segment, is controlled by a mammalian or virus promoter (e.g., the cytomegalovirus immediate early promoter) that facilitates expression of the naked DNA gene product(s) within the vaccinee's cells. This intracellular expression can elicit both humoral and cell-mediated immune responses (Robinson and Torres, 1997, supra; and Gregoriadis, 1998, supra). Methods of DNA delivery include needle inoculation, oral or pulmonary delivery, and inoculation by particle bombardment (i.e., gene gun). DNA vaccination by each of these methods elicits protective immunity against many different pathogens including numerous viruses (Robinson and Torres, 1997,supra; and Gregoriadis, 1998, supra). However, neither an immune response against hantaviruses nor protection against hantavirus infection have so far been demonstrated using a DNA vaccine.

In this report, we demonstrate that naked DNA vaccination with the SEOV M or S genome segment elicits SEOV-specific antibody responses in rodents. More importantly, we demonstrate that DNA vaccination with the SEOV M segment elicits neutralizing antibodies and protects hamsters against SEOV infection.

Also in this application we report the development of a HTNV M DNA vaccine. This vaccine expresses the G1 and G2 proteins of HTNV, elicits neutralizing antibodies in hamsters, and protects hamsters against infection with HTNV, SEOV, and DOBV. Furthermore, we demonstrate that the SEOV M, and HTNV M DNA vaccine elicit high-titer neutralizing antibody responses in nonhuman primates.

Additionally, we describe the development and testing of the first ANDV M gene-based DNA vaccine that elicits a neutralizing, or otherwise protective, antibody response. This was an unexpected result since the vaccine is not immunogenic nor protective in hamsters but is immunogenic and protective in rhesus monkeys. In addition, cross protection was evident against two other HPS-associated hantaviruses: Sin Nombre virus and Black Creek Canal virus.

Also described in this application is a Hantaan/Andes dual-M gene hantavirus DNA vaccine containing both the HTNV M gene and the ANDV M gene. This vaccine showed immunogenicity and protection in non-human primates. This is the first DNA vaccine designed to protect against all hantaviruses, both HPRS-associated and HPS-associated, that cause severe disease. The Hantaan/Andes dual-M gene hantavirus DNA vaccine, pWRG/HA-M, was tested for immunogenicity in rhesus macaques. The vaccine elicited an antibody response that neutralized Hantaan virus and Andes virus (see Table 8). In a single hamster experiment, we found that this plasmid was similar to the Andes virus plasmid, in that it did not elicit an antibody response in hamsters. However, the fact it elicits neutralizing antibodies in monkeys suggests it could elicit neutralizing antibodies in humans.

Furthermore, this application also describes a Hantavirus immunoglobulin composition which can be used as a prophylactic or therapeutic effective in preventing onset of hantavirus infection after exposure to hantavirus, and/or in treating hantavirus disease. There is presently no specific drug or immunotherapeutic to treat HPS or HFRS disease or to administer to people with possible hantavirus exposure, or hantavirus disease. The hantavirus immunoglobulin composition of the present invention is composed of polyclonal antiserum from a population of animals/humans vaccinated with a DNA vaccine comprised of a plasmid expressing the Andes virus M gene and/or the Hantaan virus M gene. The polyclonal serum would contain neutralizing antibodies against Andes virus and/or against Hantaan virus. Unlike conventional polyclonal immune serum products, the process used to make this invention (DNA vaccination of primate antibody producing vaccinees) does not involve live virus and does not require the identification of patients who have survived hantavirus disease.

Therefore, it is one object of the present invention to provide a hantavirus DNA vaccine comprising a hantavirus genome segment. We describe a SEOV hantavirus DNA vaccine comprising the SEOV M genome segment specified in SEQ ID NO:1 or comprising the SEOV S segment specified in SEQ ID NO:2. We also describe a HTNV hantavirus DNA vaccine comprising the HTNV M genome segment specified in extending from 2 to 3565 of genbank sequence accession number M14627, and an ANDV DNA vaccine comprising the ANDV M genome segment extending from 2 to 3671 of genbank sequence accession number AF291703. We also describe a HTNV/ANDV DNA vaccine comprising the HTNV M genome segment and the ANDV M genome segment. The hantavirus DNA segments described above can be presented to a subject as part of a DNA vaccine alone, or in combination with another Hantavirus DNA segment, whether the combination is on the same construct or on a different construct.
Also disclosed is a method for eliciting in a subject an immune response against hantavirus, the method comprising administering to a subject a DNA fragment comprising a genome segment of hantavirus. More specifically, we disclose a method for eliciting an immune response against SEOV hantavirus by providing the M or S genome segment of SEOV, a method for eliciting an immune response against HTNV hantavirus by providing the M genome segment of HTNV, a method for eliciting an immune response against ANDV hantavirus by providing the M genome segment of ANDV. Cross protection against other hantaviruses such as Hantaan virus and Dobrava or Sin Nombre and Black Creek Canal are also an object of this invention.

Also disclosed is a DNA vaccine which elicits an immune response against both HFRS and HFS hantavirus and protects against all the hantaviruses causing severe disease by providing to a subject a DNA vaccine comprising a Hantaan M gene DNA vaccine in combination with an Andes M gene DNA vaccine such that each M gene is expressed in the subject. The Hantaan M gene or the Andes M gene may be administered separately, i.e. on seperate vectors, or may be combined on the same vector as is described in one aspect of this invention. In addition, other M gene segments from other hantaviruses, e.g. Puumula, may be combined with the Hantaan DNA vaccine and the Andes DNA vaccine in order to produce a multivalent vaccine which can provide additional protection against hantavirus infection.
The DNA vaccine may be delivered by coating a small carrier particle with the DNA vaccine and delivering the DNA-coated particle into an animal's epidermal tissue via particle bombardment. This method may be adapted for delivery to either epidermal or mucosal tissue, or delivery into peripheral blood cells, and thus may be used to induce humoral, cell-mediated, and secretory immune responses in the vaccinated individual.

The DNA vaccine is inherently safe, is not painful to administer, and should not result in adverse side effects to the vaccinated individual. In addition, it does not require growth or use of hantavirus, which may be spread by aerosol transmission.

Also disclosed is a composition of primate polyclonal serum containing neutralizing antibodies directed against at least one, preferably two or more, hantavirus M segments, e.g. Andes virus and/or against Hantaan virus.

Antibodies may be provided that are functionally equivalent to the antibodies listed above. These functionally equivalent antibodies substantially share at least one major functional property with an antibody listed above and herein described comprising: immunoreactivity in vitro, protection against hantavirus challenge when administered prophylactically or therapeutically, competition for same binding site on G1 and/or G2. The antibodies can be of any class such as IgG, IgM, or IgA or any subclass such as IgG1, IgG2a, and other subclasses known in the art. Further, the antibodies can be produced by any method, such as phage display, or produced in any organism or cell line, including bacteria, insect, mammal or other type of cell or cell line which produces antibodies with desired characteristics, such as humanized antibodies. The antibodies can also be formed by combining an Fab portion and a Fc region from different species.

Hantavirus infection may be treated or prevented by administering a therapeutically or prophylactically effective amount of serum or a mixture of antibodies to a subject in need of such treatment.

Passive vaccines may be provided for treating or preventing hantavirus infections comprising a therapeutically or prophylactically effective amount of the antibodies which protect against hantavirus disease in combination with a pharmaceutically acceptable carrier or excipient.

A method for diagnosis of hantavirus infection may comprise assaying for the presence of hantavirus in a sample using the antibodies described.

Novel immunoprobes and test kits may be provided for detection of hantavirus infection comprising antibodies. For immunoprobes, the antibodies are directly or indirectly attached to a suitable reporter molecule, e.g., and enzyme or a radionuclide. The test kit includes a container holding one or more antibodies and instructions for using the antibodies for the purpose of binding to hantavirus to form an immunological complex and detecting the formation of the immunological complex such that presence or absence of the immunological complex correlates with presence or absence of hantavirus.

### BRIEF DESCRIPTION OF THE DRAWINGS

These and other features, aspects, and advantages of the present invention will become better understood with reference to the following description, appended claims, and accompanying drawings where:

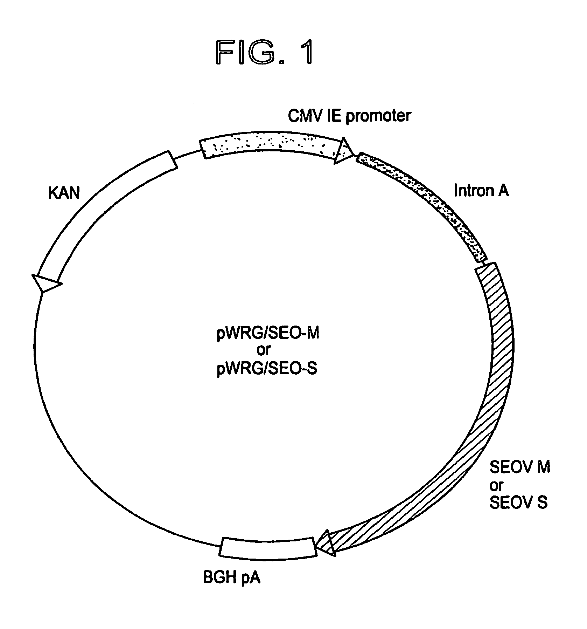

**Fig. 1**. SEOV naked DNA expression constructs. The SEOV M or S genome segment was amplified by RT-PCR and cloned into *Not*I and *Bam*HI sites of pWRG7077 (PowderJect Vaccines, Inc., Madison, Wisconsin, described in Schmaljohn et al., 1997, supra). Characteristics of pWRG7077 are similar to those of pWRG1602 described previously (Dimmock, N. J., 1995, Med. Virol. 5: 165) and include a human cytomegalovirus early promoter (CMV IE promoter) and intron A, a bovine growth hormone transcription terminator and polyadenylation signal (BGH pA), and a kanamycin resistance gene. CMV IE promoter: cytomegalovirus immediate early promoter and intron A, BGH pA: Bovine growth hormone polyadenylation signal, KAN: kanamycin antibiotic resistance gene.

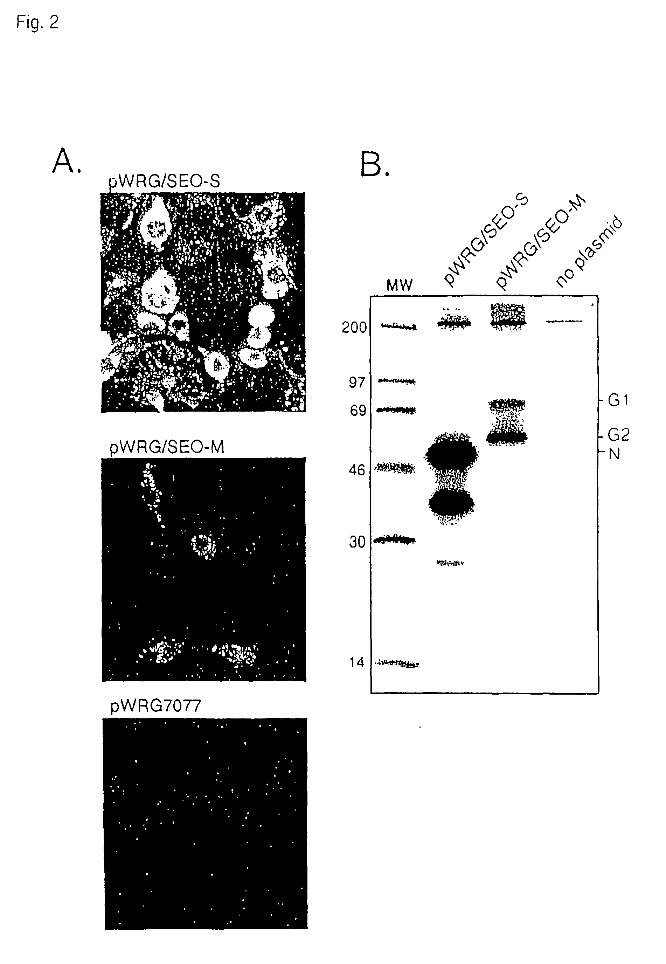

**FIG. 2A and 2B**. Transient expression from pWRG/SEO-M (SEQ ID NO:3) and pWRG/SEO-S (SEQ ID NO:4). A) IFAT were performed on COS cell monolayers transfected with the SEOV M or S constructs. pWRG/7077-transfected monolayers served as negative controls. Anti-SEOV polyclonal rabbit antisera was used as the primary antibody. B) COS cells were transfected with the indicated plasmid and radiolabeled expression products were immunoprecipitated with anti-SEOV polyclonal rabbit serum. Molecular size markers (MW) in kDa are shown on the left, and the position of the SEOV G1, G2, and N proteins are shown on the right.

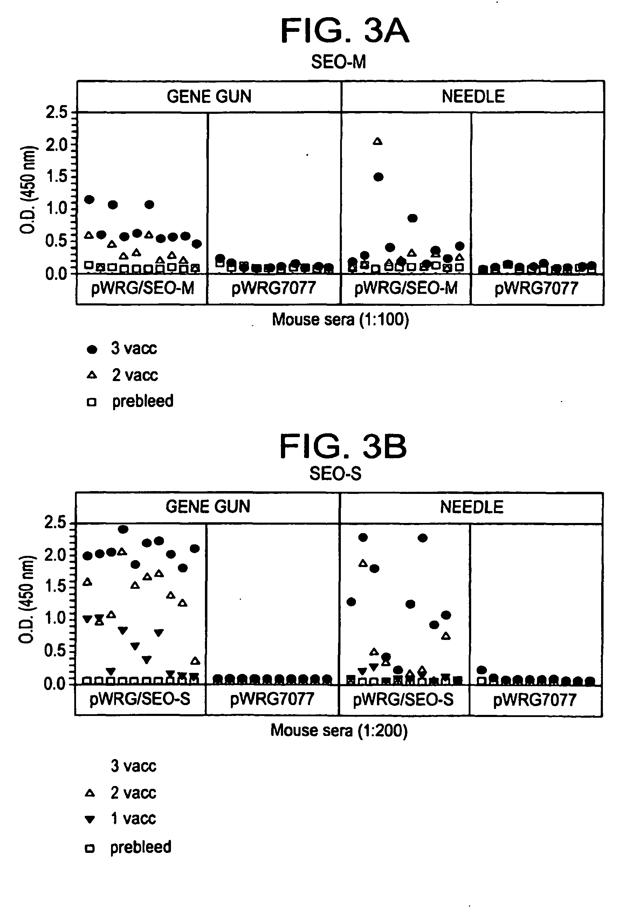

**Fig. 3A and 3B**. Antibody responses (ELISA) of mice vaccinated with SEOV naked DNA by gene gun or intramuscular needle inoculation. The antibody responses to vaccination with pWRG/SEO-M (panel A) or pWRG/SEO-S (panel B) by gene gun or intramuscular needle inoculation were evaluated by ELISA. Mice vaccinated with negative control plasmid pWRG7077 were evaluated in both ELISAs. Prebleed sera and sera collected 4 weeks after the first vaccination (1 vacc), 4 weeks after the second vaccination (2 vacc), 3 weeks after the final vaccination (3 vacc) were all tested in a single assay. Symbols represent the average values of individual mice serum samples assayed in duplicate.

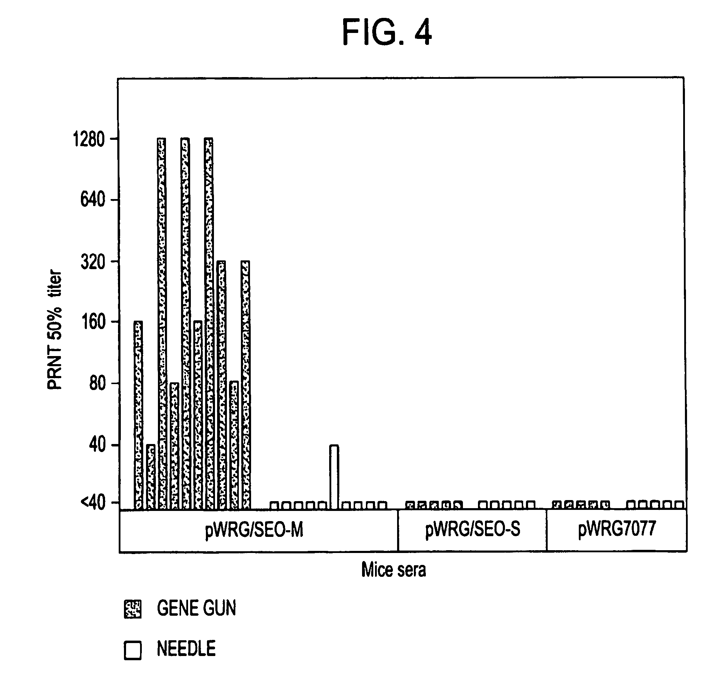

**Fig. 4**. Neutralizing antibody responses of mice vaccinated with SEOV naked DNA. PRNT were performed on mice sera collected 3 weeks after the third vaccination by either gene gun or intramuscular needle injection (needle). Bars represent sera from individual mice vaccinated with the indicated immunogen. Sera were heat inactivated (56°C, 30 min) and the assay was performed in the presence of 5% guinea pig complement. PRNT_{50%} titers are expressed as reciprocal of the highest serum dilution resulting in a 50% reduction in plaque number.

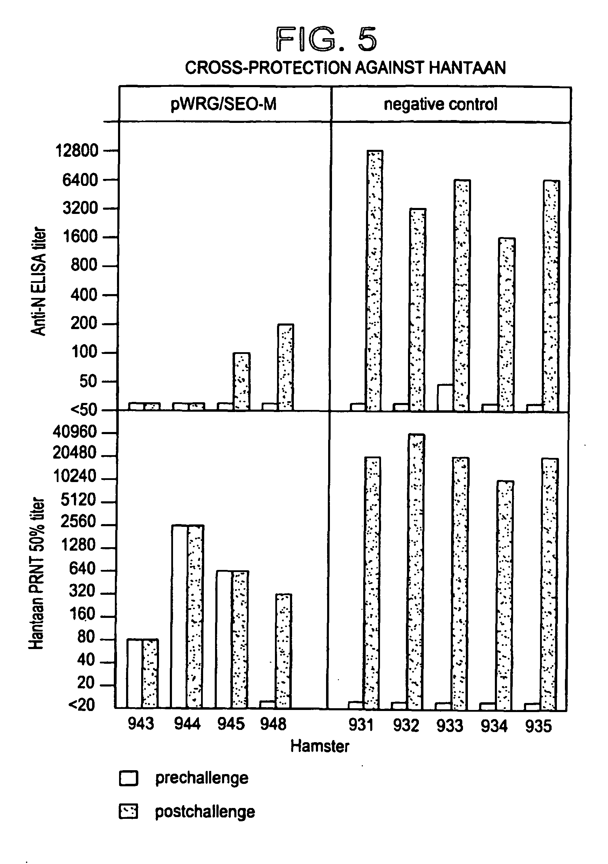

**Fig. 5**. Vaccination with pWRG/SEO-M cross-protects against infection with Hantaan virus. Groups of 4-5 hamsters were vaccinated with either pWRG/SEO-M or negative control plasmid (pWRG7077) as described in Methods below. Three weeks after the final vaccination, prechallenge serum samples were obtained and the hamsters were challenged with 1,000 PFU of Hantaan virus. Twenty-eight days after challenge postchallenge serum samles were obtained. Pre and postchallenge serum samples were evaluated by anti-N ELISA to detect antibody to nucleocapsid, and by a Hantaan plaque reduction neutralization test (PRNT) to detect Hantaan neutralizing antibodies. ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum.

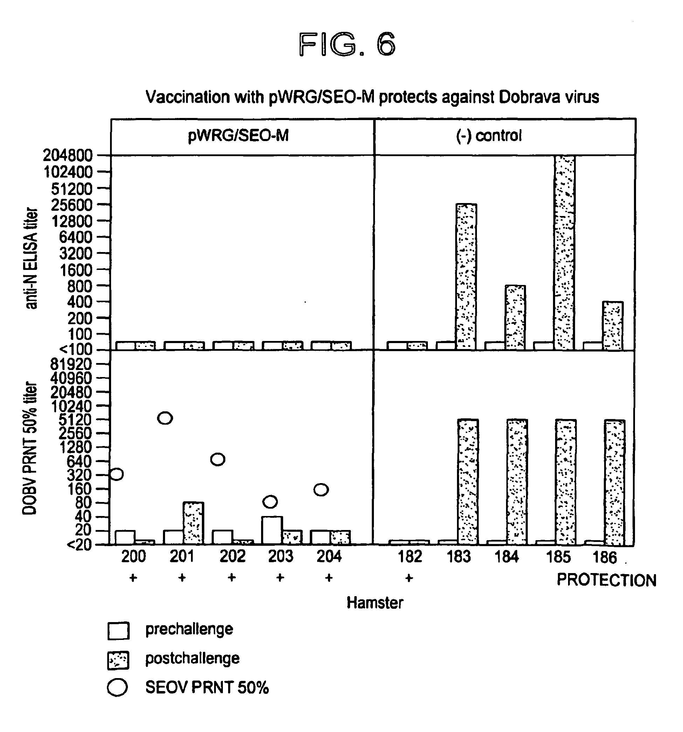

**Fig. 6**. Vaccination with pWRG/SEO-M cross-protects against infection with Dobrava virus. Groups of 5 hamsters were vaccinated with either pWRG/SEO-M or negative control plasmid (pWRG7077) as described in Methods. Three weeks after the final vaccination, prechallenge serum samples were obtained and the hamsters were challenged with 1,000 PFU of Dobrava virus. Twenty-eight days after challenge postchallenge serum samples were obtained. Pre and postchallenge serum samples were evaluated by anti-N ELISA to detect antibody to nucleocapsid, and by a Dobrava plaque reduction neutralization test (PRNT) to detect Dobrava neutralizing antibodies. ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum. The Seoul neutralizing prechallenge antibody titers (elicted by the vaccine) were also measured. The Seoul virus PRNT80% values are shown as empty circles.

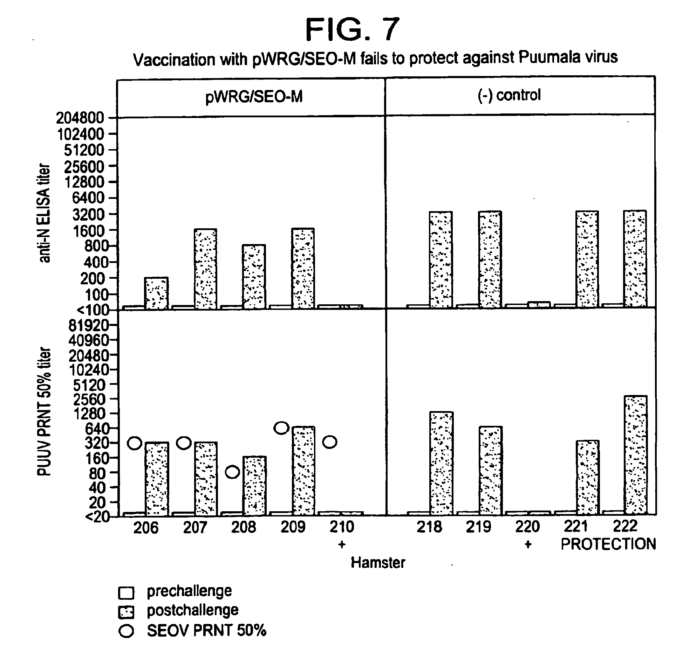

**Fig. 7**. Vaccination with pWRG/SEO-M fails to cross-protect against infection with Puumala virus. Groups of 5 hamsters were vaccinated with either pWRG/SEO-M or negative control plasmid (pWRG7077) as described in Methods. Three weeks after the final vaccination, prechallenge serum samples were obtained and the hamsters were challenged with 1,000 PFU of Puumala virus. Twenty-eight days after challenge postchallenge serum samples were obtained. Pre and postchallenge serum samples were evaluated by anti-N ELISA to detect antibody to nucleocapsid, and by a Puumala plaque reduction neutralization test (PRNT) to detect Puumala neutralizing antibodies . ELISA titers represent the lowest reciprocal dilution that resulted in an O.D. value that was greater than the background O.D. plus three standard deviations. PRNT50% titers represent the lowest reciprocal dilution that resulted in a 50% reduction in plaque number in the absence of serum. The Seoul neutralizing prechallenge antibody titers (elicted by the vaccine) were also measured. The Seoul virus PRNT80% values are shown as empty circles.

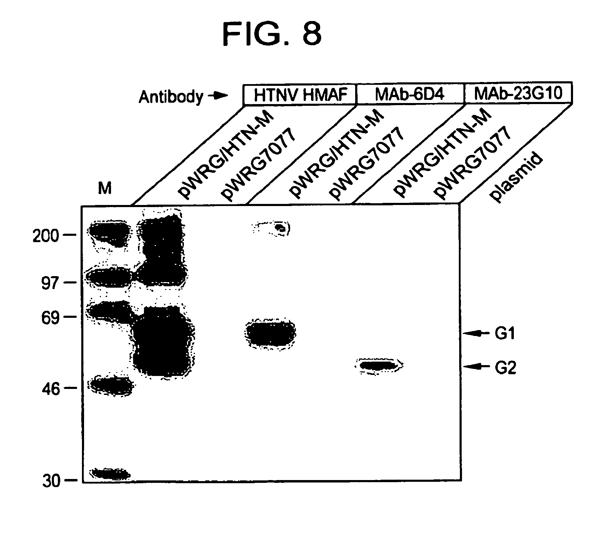

**Fig. 8****. Transient expression of HTNV G1 and G2**. Cos cells were transfected with pWRG/HTN-M or a negative control plasmid (pWRG7077) and, after 24 hr, radiolabeled cell lysates were prepared for analysis by RIPA. Expression products were immunoprecipitated with a polyclonal mouse hyperimmune ascitic fluid against HTNV (HTN HMAF), a G1-specific MAb (MAb 6D4), or a G2-specific MAb (Mab-23G10). Molecular sizes (M) are shown at left in kDa, and the position G1 and G2 are shown at the right.

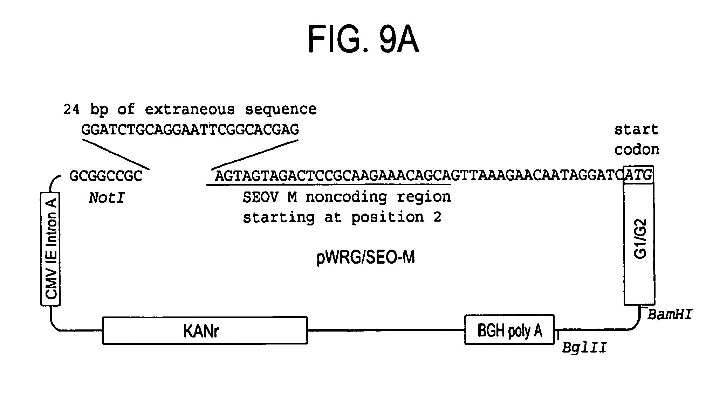

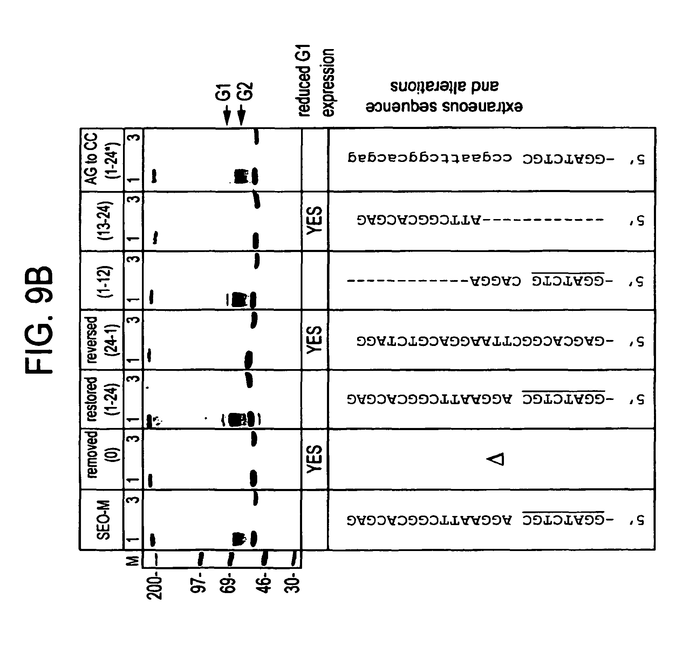

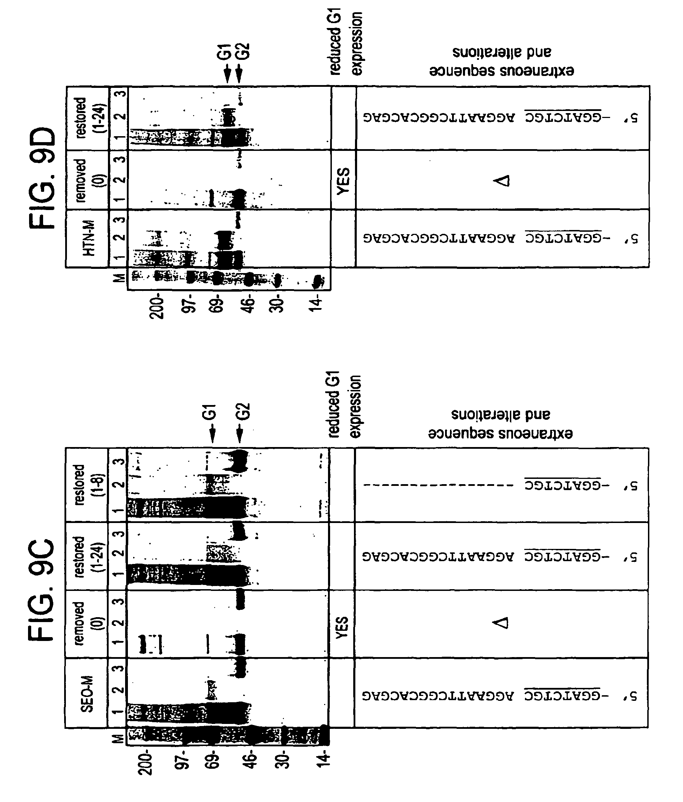

**Fig. 9A****,** **9B****,** **9C, 9D****. Efficient expression of G1 requires upstream extraneous sequence**. (A) Schematic diagram of SEOV M gene DNA vaccine plasmid, pWRG/SEO-M, showing nucleotide sequence between NotI site of the vector and the SEOV G1 start codon [SEQ ID NO:5]. The NotI site is followed by 24 extraneous nucleotides arising from a previous cloning procedure [SEQ ID NO:6], which is followed by SEOV M antigenome 5' noncoding region starting at position 2. The hantavirus M genes were cloned into either the NotI-BamHI site, or the NotI-BglII site of pWRG7077. G1/G2, open reading frame encoding the G1 and G2 glycoproteins; CMV IE intron A, cytomegalovirus immediate-early promoter followed by intron A sequence; KANr, kanamycin resistance gene; BGH poly A, bovine growth hormone polyadenylation signal. (B) Alterations in the extraneous sequence affect SEOV G1 expression. COS cells were transfected with pWRG/SEO-M (SEO-M) (sequence in this column corresponds to SEQ ID NO:6), or a plasmid with the indicated alteration: extraneous sequence removed (0); extraneous sequence restored (1-24) (sequence in this column corresponds to SEQ ID NO:6) ; extraneous sequence reversed (24-1) (sequence in this column corresponds to SEQ ID NO:6 reversed); 3' half of extraneous sequence removed (1-12) (sequence in this column corresponds to nucleotides 1-12 of SEQ ID NO:6); 5' half of extraneous sequence removed (13-24) (sequence in this column corresponds to nucleotides 13-24 of SEQ ID NO:6); substitution of AG with CC at position 9,10 of extraneous sequence (1-24* (sequence in this column corresponds to SEQ ID NO:6 with CC to AG substitution at position 9, 10 of sequence). Immunoprecipitations were performed using polyclonal rabbit anti-SEOV antibody containing G1- and G2 specific antibodies (1), or a pool of G2-specific monoclonal antibodies (3). The sequence between the NotI site of the vector and position 2 of the SEOV M gene nontranslated region are shown. Δ, indicates extraneous sequence is deleted. The underlined GGATCTGC (nucleotides 1-8 of SEQ ID NO:6) is the minimal unaltered sequence associated with efficient expression of G1. (C) Efficient G1 expression is restored if nucleotides 1-8 of the extraneous sequence, GGATCTGC (nucleotides 1-8 of SEQ ID NO:6), are included between the NotI site and the SEOV M noncoding region. COS cells were transfected with pWRG/SEO-M (SEO-M), or a plasmid with the extraneous sequence removed (0); extraneous sequence restored (1-24) (SEQ ID NO:6); or a shortened version of the extraneous sequence (1-8) (nucleotides 1-8 of SEQ ID NO:6). Immunoprecipitations were performed using polyclonal rabbit anti-SEOV antibody containing G1- and G2 specific antibodies (1), a G1-specific MAb pool (2), or a G2-specific MAb-11EI0 (3) (D) Deletion of the extraneous sequence affects HTNV G1 expression. COS cells were transfected with the HTNV M gene DNA vaccine plasmid, pWRG/HTN-M(x)(HTN-M) (sequence in this column corresponds to SEQ ID NO:6), or a plasmid with the indicated alteration: extraneous sequence removed (0); extraneous sequence restored (1-24) (sequence in this column corresponds to SEQ ID NO:6). Immunoprecipitations were performed using anti-HTNV mouse hyperimmune ascitic fluid containing G1- and G2-specific antibodies (1), G1-specific MAb pool (2), or a G2-specific MAb-11E10 (3). Molecular sizes (M) are shown at the left in kDa, and the position of G1 and G2 are shown at the right.

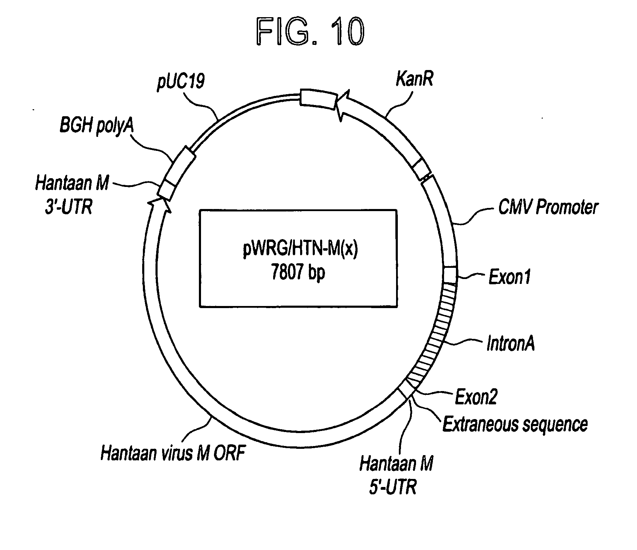

**Fig. 10****: Schematic of pWRG/HTNV-M(x) plasmid expressing G1 and G2**. pWRG/HTN-M(x) employs the hCMV immediate early promoter and 5' noncoding sequences from exons 1 and 2 with the native intervening Intron A. The Hantaan virus M gene open reading frame (ORF) is preceded by the Hantaan virus M untranslated region (UTR) and followed by sequence from the Hantaan M gene 3'-UTR. Extraneous sequence that we have found is required for efficient G1 expression is between the Intron A and Hantaan virus 5' UTR. Transcription termination is facilitated by a bovine growth hormone polyadenylation site (BGH polyA). The plasmid backbone is pUC19 and a kanamycin resistance gene (KanR) confers antibiotic resistance.

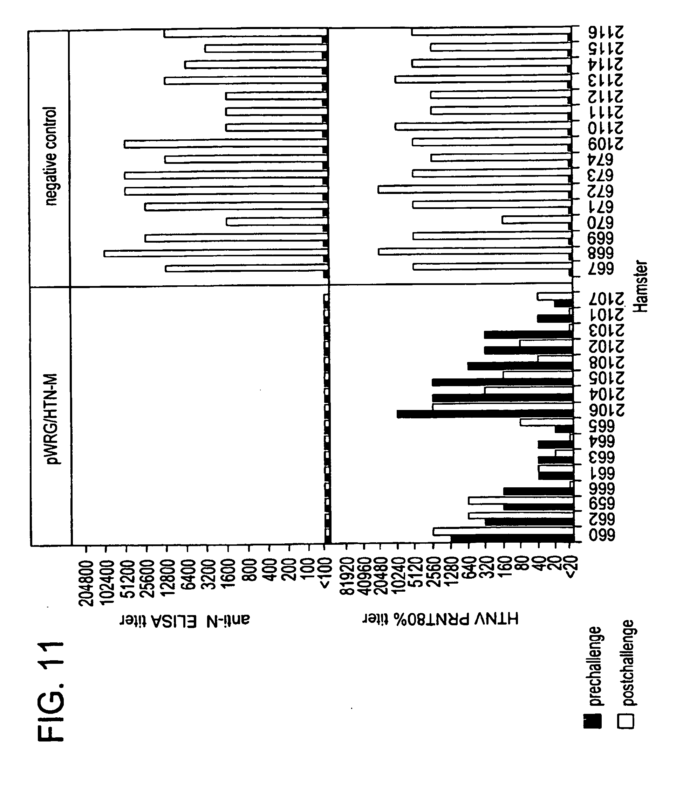

**Fig. 11****: DNA vaccination with plasmid expressing HTNV G1 and G2 protects against HTNV infection**. The results of two independent experiments are combined in this figure. In the first experiment, one group of hamsters (659-666) was vaccinated with pWRG/HTN-M, and a second negative control group (667-674) was vaccinated with the vector plasmid, pWRG7077. In the second experiment one group of hamsters (2101-2108) was vaccinated with pWRG/HTN-M(x) and a second negative control group (2109-2116) remained unvaccinated. Three weeks after the final vaccination, prechallenge serum samples were obtained and the hamsters were challenged with HTNV. Postchallenge serum samples were obtained 28 days after challenge. The pre- and postchallenge serum samples were tested for N-specific antibodies by anti-N ELISA, and neutralizing antibodies by PRNT. The pre- and postchallenge, end-point antibody titer for each hamster is shown. For each experiment, the prechallenge homotypic PRNT_{80%} titers were sorted from highest to lowest, left to right.

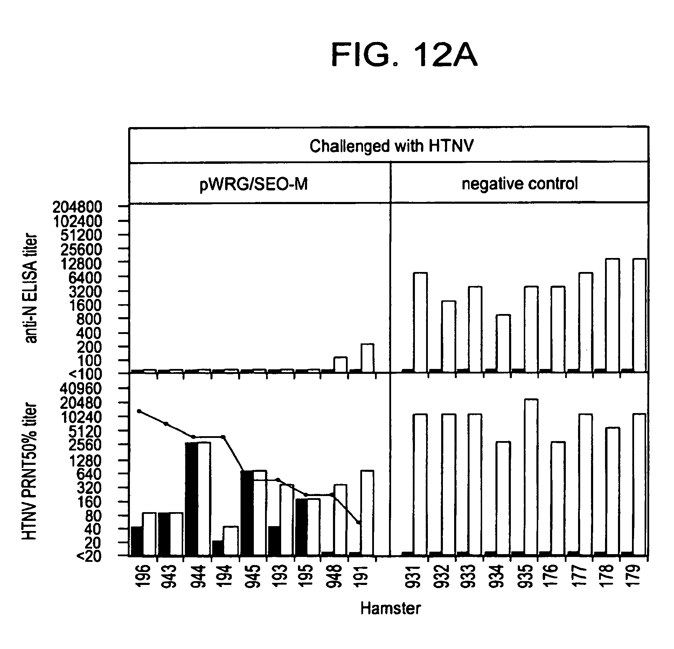

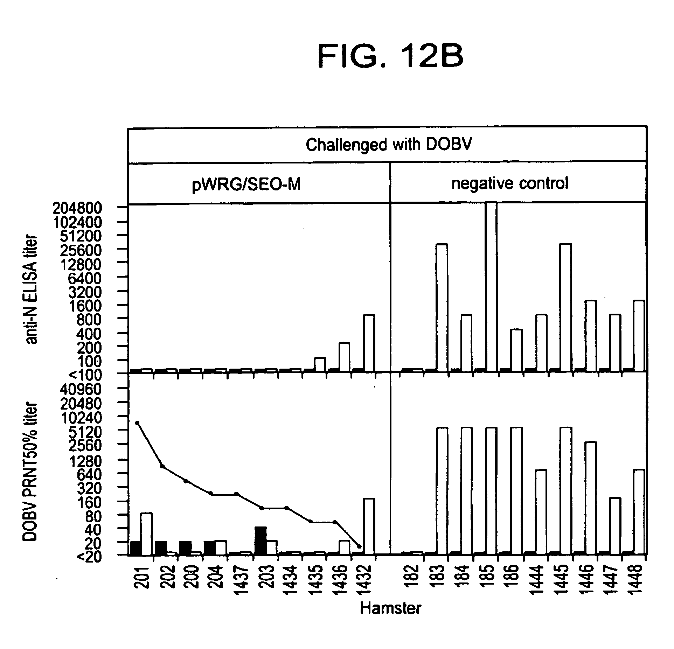

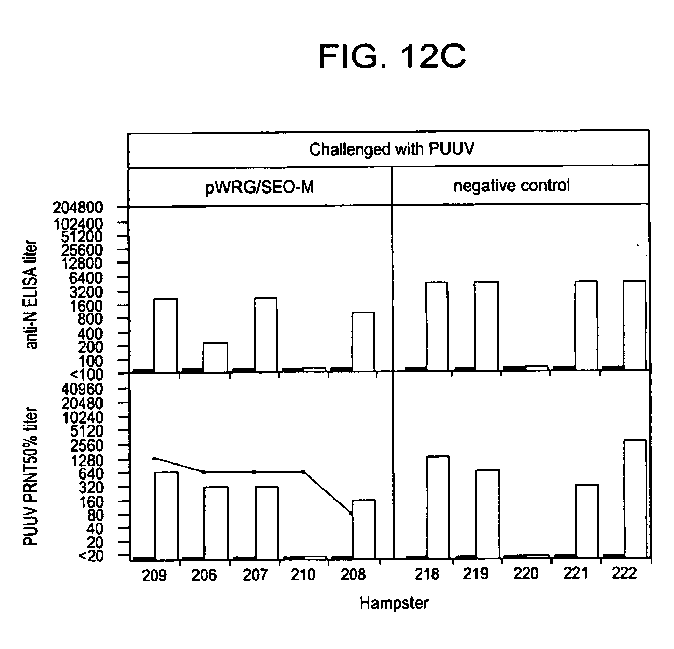

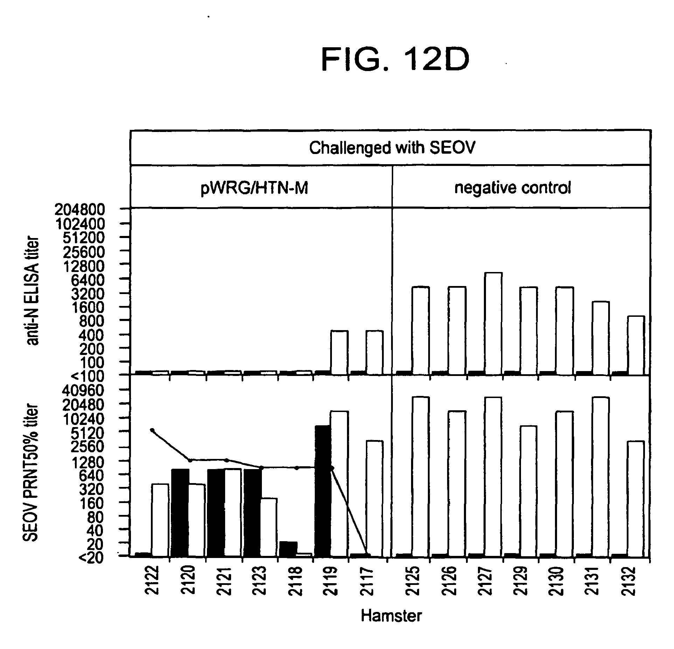

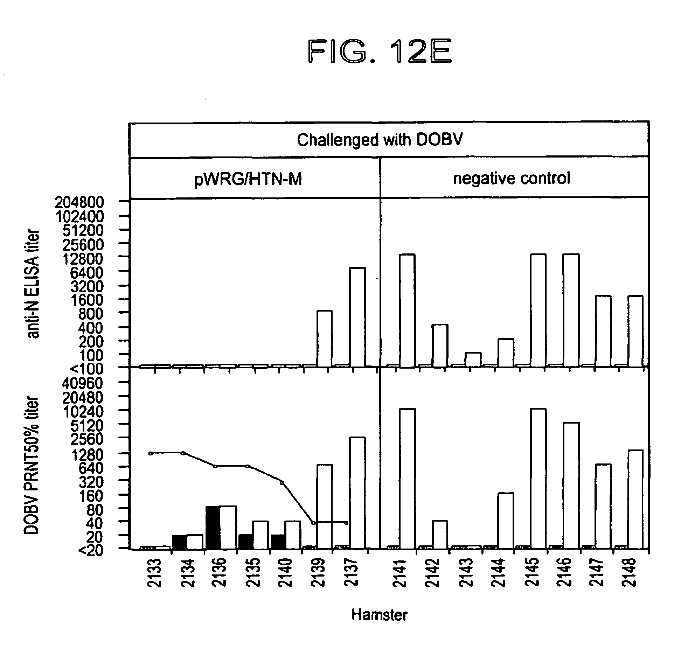

**Fig. 12A****,** **12B****,** **12C****,** **12D****, and** **12E****: Cross-protection**. Hamsters were vaccinated with the indicated plasmid (pWRG/SEO-M, pWRG/HTN-M(x) containing the extraneous sequence, or a negative control), and then challenged with the indicated virus. The negative control hamsters in panels A, B, and C were vaccinated with a pWRG7077-based plasmid; and the negative control hamsters in panels D and E remained unvaccinated. Pre- and postchallenge serum samples were tested for anti-N antibodies by anti-N ELISA, and neutralizing antibodies by PRNT. PRNT_{80%} titers for homotypic virus, and PRNT_{50%} titers for heterotypic virus, were determined. The prechallenge and postchallenge end-point antibody titers for each hamster are shown. Prechallenge homotypic PRNT_{80%} titers (sorted from highest to lowest, left to right) are shown as lines with symbols. The identification code for each hamster is shown on the x-axis. The HTNV PRNT, and anti-N ELISA data for hamsters 943, 944,945, and 948 was published previously (Kamrud et al., 1999, Virology, 263, 209-219).

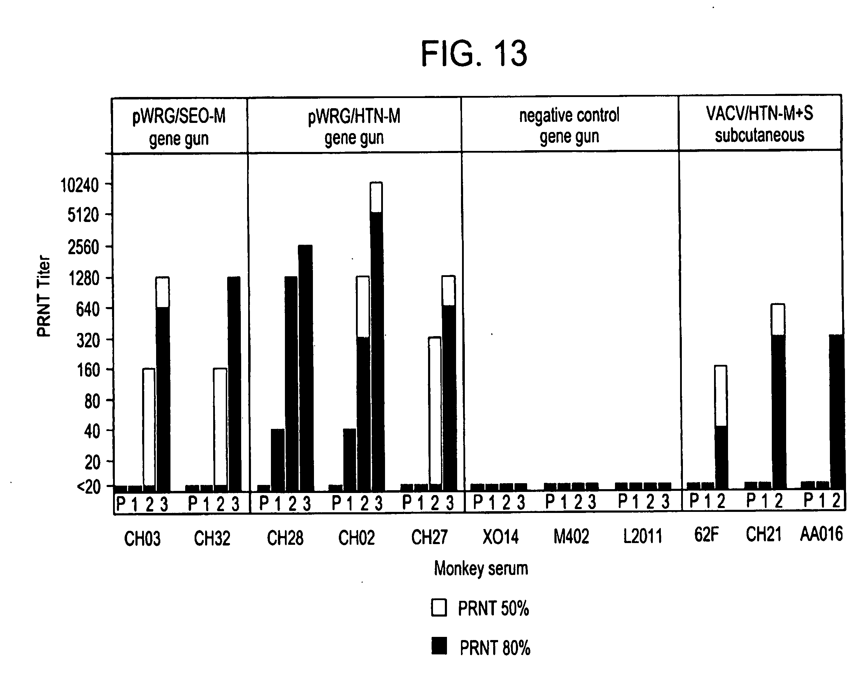

**Fig. 13****: DNA vaccination with plasmid expressing SEOV or HTNV G1 and G2 elicits high-titer neutralizing antibody responses in rhesus monkeys**. Rhesus monkeys were vaccinated with either pWRG/SEO-M(x), pWRG/HTN-M(x), or rVV/HTN-M+S by the indicated route as described in Methods. Serum samples were obtained before vaccination (P), and then 3 weeks after the first (1), second (2) and third vaccination (3). Serum was also collected 2 months (a) 4 months (b), and 6 months (c) after the final vaccination. PRNT titer represents the reciprocal serum dilution that neutralized virus plaque number 80% or 50%. The identification code for each monkey is shown below its respective plot.

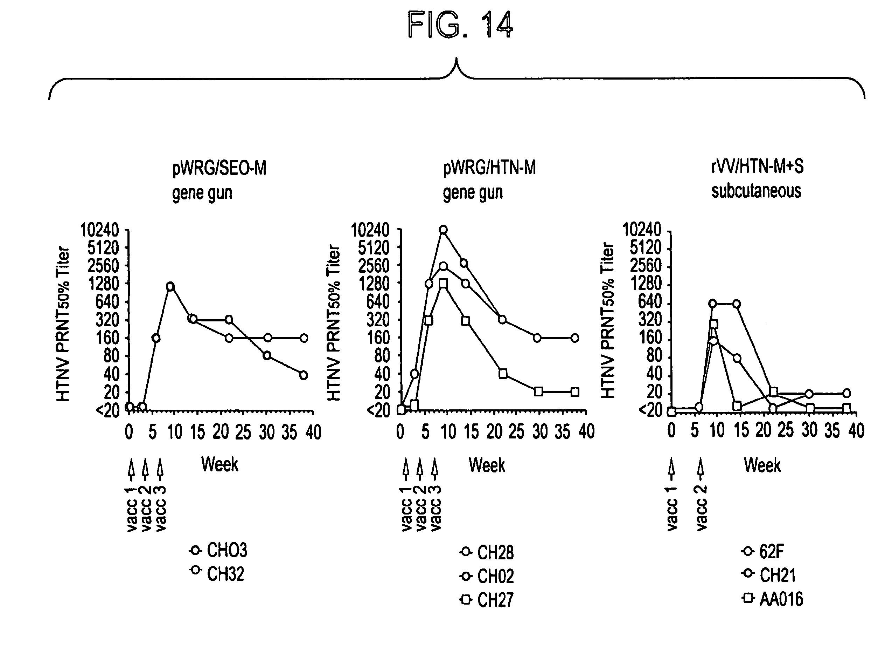

**Fig. 14****: Duration of Neutralizing antibody response in monkeys**. Neutralizing antibodies elicited by DNA vaccination are still detected in rhesus monkeys 8 months after the final vaccination. Rhesus monkeys vaccinated with the indicated vaccine were bled 3 weeks after each vaccination and then at 2, 4, 6, and 8 months after the final vaccination (weeks 14, 22, 30, and 38, respectively). The homologous neutralizing antibody response for the indicated week after the first vaccination (week 0) was evaluated by PRNT. Each line represents an individual monkey. The week 9 data are also presented in Fig. 13.

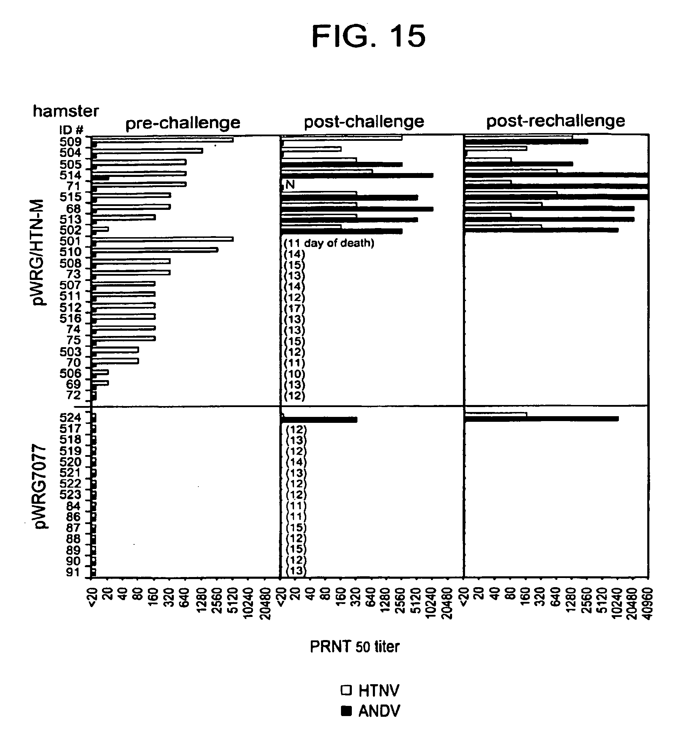

**Fig. 15****: Evaluation of HFRS DNA vaccine in hamster/ANDV lethal disease model**. Hamsters were vaccinated with pWRG/HTN-M(x) or negative control plasmid pWRG7077 and then challenged with ANDV. For animals that succumbed, the day-of-death is shown in parentheses. Animals that survived the first challenge were rechallenged with ANDV. Serum drawn on the day of challenge (prechallenge), 4-6 weeks after challenge (postchallenge), and 28-48 days after a second challenge (post-rechallenge) was tested for HTNV- and ANDV-specific NAbs by PRNT. Bars represent PRNT titers. Hamster identification numbers (ID#) 68-91 and 501-523 represent two independent experiments; N, not done.

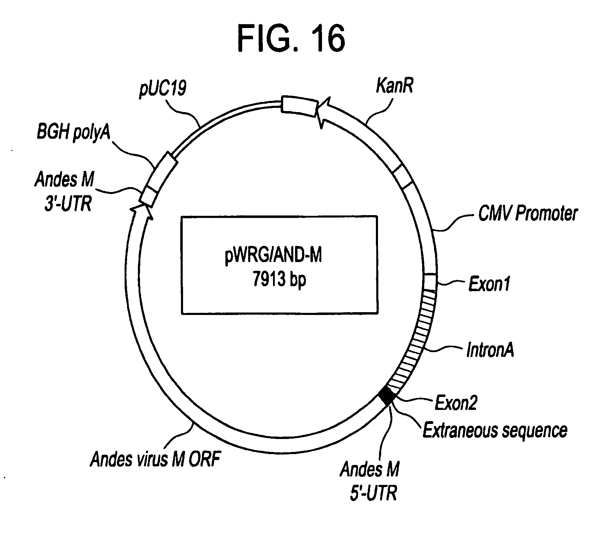

**Figure 16****: Schematic of pWRG/AND-M plasmid.** pWRG/AND-M employs the hCMV immediate early promoter and 5' noncoding sequences from exons 1 and 2 with the native intervening Intron A. The Andes virus M gene open reading frame (ORF) is preceded by the Andes virus M untranslated region (UTR) and followed by sequence from the Andes M gene 3'-UTR. Extraneous sequence that we have found is required for efficient G1 expression is between the Intron A and Andes virus 5'-UTR. Transcription termination is facilitated by a bovine growth hormone polyadenylation site (BGH polyA). The plasmid backbone is pUC19 and a kanamycin resistance gene (KanR) confers antibiotic resistance.

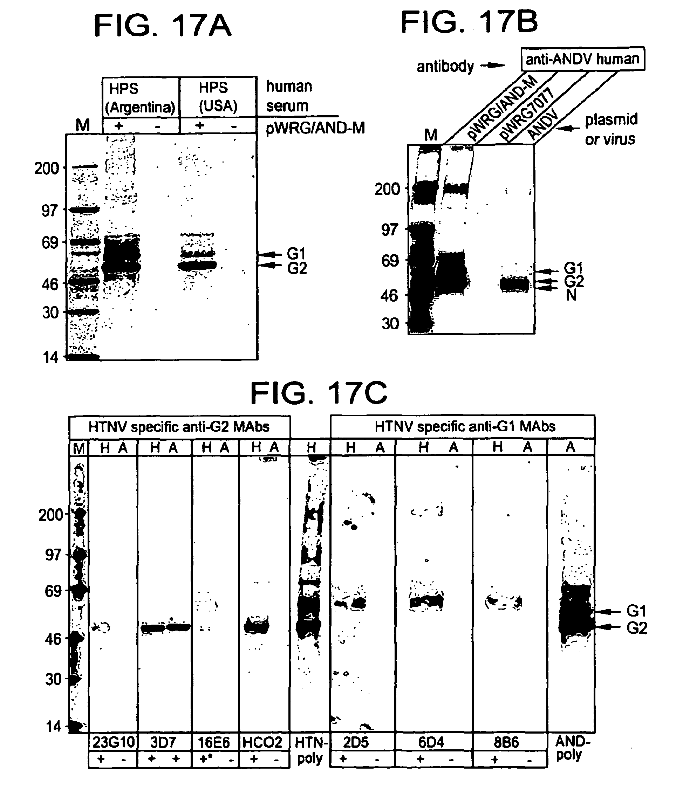

**Fig. 17A, 17B, and 17C****: Expression from pWRG/AND-M**. A) HPS convalescent serum from Argentina or the U.S. were used to immunoprecipitate radio-labeled proteins from COS cells transfected with pWRG/AND-M (+), or empty vector plasmid pWRG7077 (-). B) HPS convalescent serum from Argentina (anti-ANDV human) was used to immunoprecipitate proteins from COS cells transfected with pWRG/AND-M, pWRG7077, or Vero E6 cells infected with ANDV. C) The indicated HTNV specific MAbs, HTNV-specific mouse hyperimmune ascitic fluid (HTN-poly), or HPS convalescent serum form Argentina (AND-poly) were used to immunoprecipitate proteins from COS cells transfected with pWRG/HTN-M(x) (H) or pWRG/AND-M (A). + indicates G1 or G2 were immunoprecipitated, - indicates no protein was immunoprecipitated, and * indicates both G1 and G2 were immunoprecipitated. Molecular size marker (M) sizes in kDa are shown at the left and the position of G1, G2, and N are shown at the right.

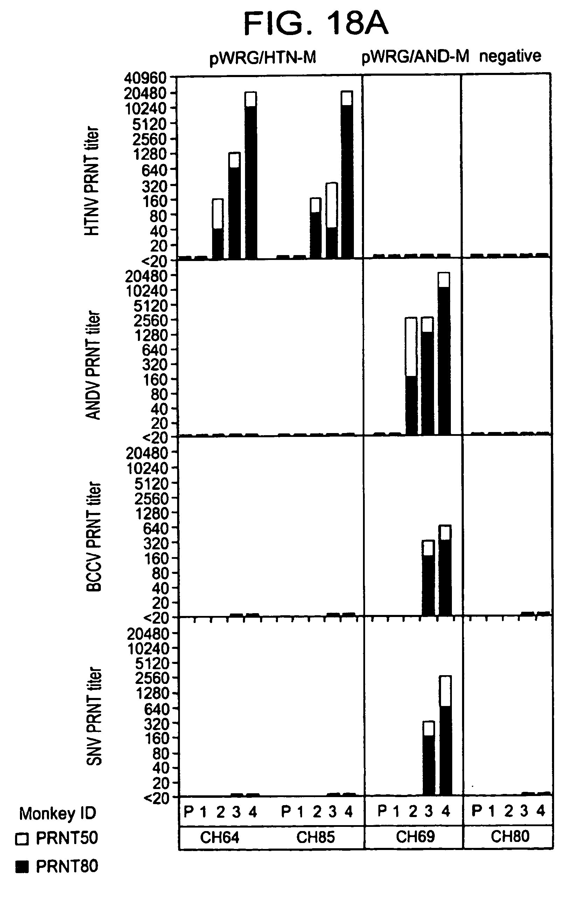

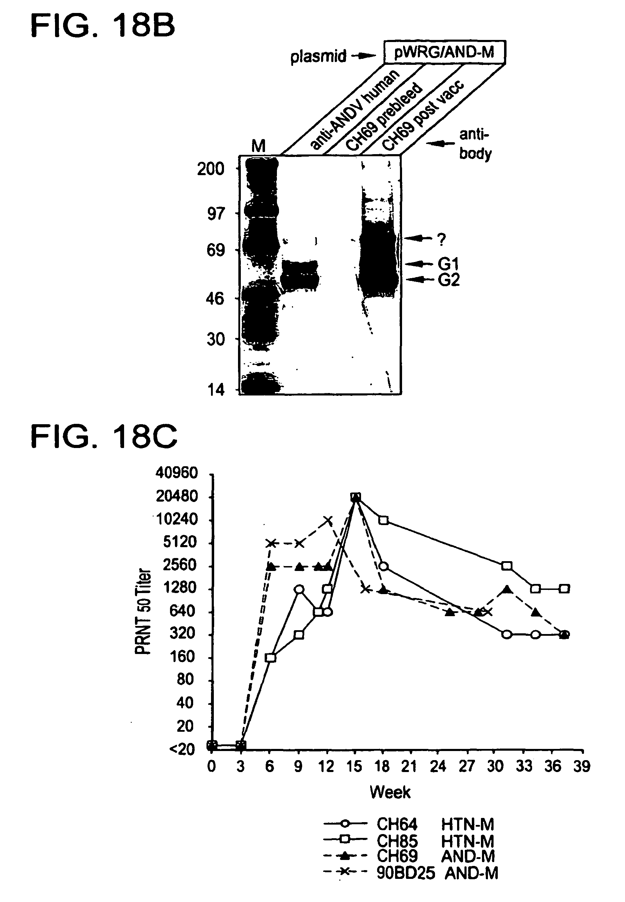

**Fig. 18A****,** **18B, and 18C****: Immunogenicity of hantavirus DNA vaccines in nonhuman primates**. A) Rhesus macaques were vaccinated with pWRG/HTN-M(x), pWRG/AND-M, or a negative control plasmid. Serum was collected before the first vaccination (P), and 3 weeks after the first, second, third, and fourth vaccination, 1, 2, 3, 4, respectively. HTNV-, ANDV-, BCCV-, and SNV-specific PRNT were performed and the endpoint 50% and 80% titers were determined. B) Serum from monkey CH69 collected before (prebleed) and after (postvaccination) four vaccinations with pWRG/AND-M was tested by RIPA for G1- and G2-specific antibodies using radio-labeled lysates from cells transfected with pWRG/AND-M. Human HPS-patient convalescent serum (anti-ANDV human) was used as a positive control for anti-ANDV antibodies. Molecular size marker (M) sizes in kDa are shown at the left and the position of G1, G2, and a -70 kDa protein (identity unknown) are shown at the right. C) The NAb responses in monkeys vaccinated with either pWRG/HTN-M(x) or pWRG/AND-M were determined after each vaccination and then at the indicated weeks after the first vaccination (week 0). CH64, CH85, and CH69 were vaccinated on week 0, 3, 6, and 12; and monkey 90BD25 was vaccinated on week 0, 3, 6, and 9. NAb titers to HTNV (solid line) or ANDV (dashed line) were determined by PRNT.

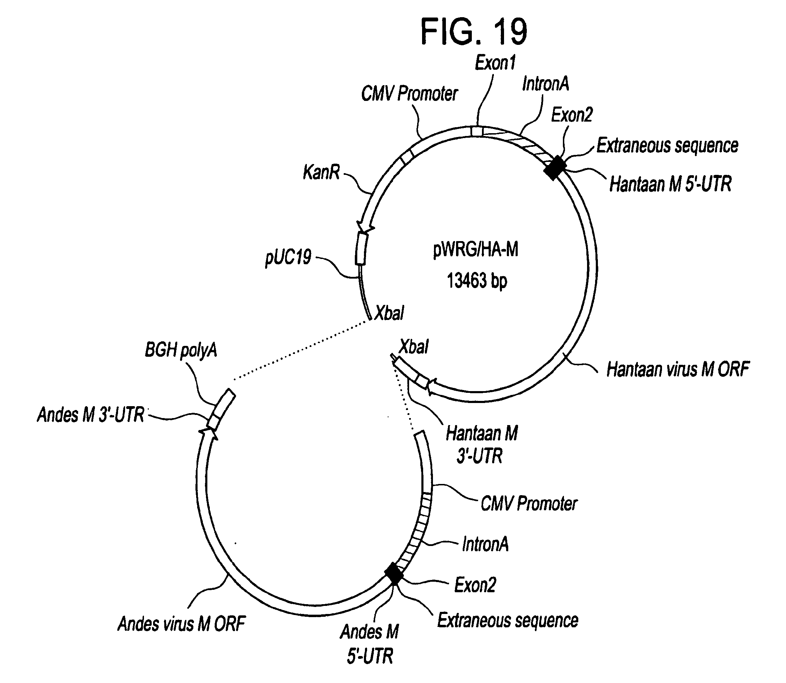

**Fig. 19****:Schematic of pWRG/HA-M plasmid.** pWRG/HA-M was constructed by PCR amplifying a region from pWRG/AND-M and inserting it into the XbaI site of pWRG/HTN-M(x). The region amplified (using primers that created XbaI sites) included the CMV promoter, Intron A, Exon 2, extraneous sequence, Andes M 5'-UTR, Andes virus M ORF, Andes 3'-UTR and BGH poly A. See previous figures for other abbreviations

### DETAILED DESCRIPTION

In this application is described a nucleotide sequence, a construct, a composition and uses therefor in methods for the vaccination of individuals against hantavirus. The method comprises delivery of a DNA encoding a hantavirus antigen to cells of an individual such that the antigen is expressed in the cell and an immune response is induced in the individual.

DNA vaccination involves administering antigen-encoding polynucleotides *in vivo* to induce the production of a correctly folded antigen(s) within the target cells. The introduction of the DNA vaccine will cause to be expressed within those cells the structural protein determinants associated with the pathogen protein or proteins. The processed structural proteins will be displayed on the cellular surface of the transfected cells in conjunction with the Major Histocompatibility Complex (MHC) antigens of the normal cell. Even when cell-mediated immunity is not the primary means of preventing infection, it is likely important for resolving established infections. Furthermore, the structural proteins released by the expressing transfected cells can also be picked up by antigen-presenting cells to trigger systemic humoral antibody responses.

In order to achieve the immune response sought, a DNA vaccine construct capable of causing transfected cells of the vaccinated individual to express one or more major viral antigenic determinant is necessary.
This can be done by identifying regions of the viral genome which code for viral glycoproteins or capsid components, and joining such coding sequences to promoters capable of expressing the sequences in cells of the vaccinee. Alternatively, the viral genome itself, or parts of the genome, can be used.

We disclose a DNA or cDNA segment which encodes an antigen from a hantavirus. By hantavirus is meant any of the Hemmorhagic fever with renal syndrome (HFRS) hantavirus such as Hantaan virus (HTNV), Dobrava-Belgrade virus (DOBV), Puumala virus (PUUV), and Seoul virus (SEOV), as well as hantavirus pulmonary syndrome (HPS) hantaviruses such as Sin Nombre virus (SNV), Black Creek Canal virus (BCCV), Bayou virus (BAYV), New York virus (NYV), Andes virus (ANDV), Laguna Negra virus (LNV), and any other hantavirus known to cause disease in humans.

More specifically, a hantavirus genome M segment encoding two envelope glycoproteins (G1 and G2) (SEQ ID NO:1), and a hantavirus genome S segment encoding nucleocapsid protein (SEQ ID NO:2) were deduced from the SEOV, strain SR-11 (Kitamura, T. et al. 1983, Jpn. J. Med. Sci. Biol. 36, 17-25) viral genome (Arikawa, J. et al. 1990, Virology 176, 114-125). The M segment (specified in SEQ ID NO:1), deposited in GeneBank accession no. M34882, corresponds to Seoul (SR-11) M gene from nucleotide 2 to 3602. The S segment (SEQ ID NO:2), having Genebank accession no. M34881, corresponds to Seoul (SR-11) S gene from nucleotide 2 to 1699.

The Hantaan hantavirus (HTNV) M segment encoding G1/G2, published Hantaan M sequence accession number M14627, corresponds to Hantaan (strain 76-118) nucleotides 2 to 3565. The Andes M segment encoding
G1/G2, deposited in GeneBank accession number AF291703, corresponds to Andes strain Chile-9717869 extending from 2 to 3671.

DNA or polynucleotide sequences which may be used in combination with the invention include fragments of the M or S gene segment from other Hantaviruses containing protective epitopes or antigenic determinants. Such epitopes, which may be conformational, may include sequences from G1 and/or G2 since monoclonal antibodies to both G1 and G2 have been shown to neutralize virus and protect rodents in passive protection experiments. Additionally, the amino terminus of N is highly immunogenic and others have shown that other methods of vaccination with the amino terminal region can confer protection (Lundvist, 1996, supra; Ulrich, 1998, supra).

The derived sequence is not necessarily physically derived from the nucleotide sequence itself, but may be generated in any manner, including for example, chemical synthesis or DNA replication or reverse transcription or transcription, which are based on the information provided by the sequence bases in the region(s) from which the polynucleotide is derived. In addition, combinations of regions corresponding to that of the designated sequence may be modified in ways known in the art to be consistent with an intended use. The sequences can be used in diagnostic assays such as hybridization assays and polymerase chain reaction (PCR) assays for the detection of Hantavirus.

RT-PCR cloning of the M and S genome segments of SEOV, strain SR-11, was described previously (Arikawa et al., 1990, Virology 176, 114-125). A DNA fragment containing Seoul (SR-11) M gene from nucleotide 2 to 3602 was excised from YMIS/SR 11-M (Arikawa, 1990, supra) using Bgl II and Bam HI. This fragment was ligated into the Bam HI site of pWRG7077 resulting in pWRG/SEO-M (SEQ ID NO:3). pWRG/SEO-M contains additional nucleotides derived from cloning vector baculovirus transfer vector YMIS.

A DNA fragment containing Seoul (SR-11) S gene from nucleotide 2 to 1699 was excised from pBSKS+/SR11-S (Arikawa, 1990, supra) using EcoRI. This fragment was subjected to Klenow to blunt the restriction sites and then ligated into Klenow-blunted Not I site of pWRG7077 (provided by Powderject, Inc., Madison, Wisconsin) resulting in pWRG/SEO-S (SEQ ID NO:4). pWRG/SEO-S contains additional nucleotides derived from cloning vector pBSKS+.

The HTNV M DNA vaccine plasmid, pWRG/HTN-M(x) (SEQ ID NO:7), was constructed essentially as follows. First, DNA encoding the HTNV G1/G2 was cut from pTZ19RHTNMm (Schmaljohn et al., 1989, In: Genetics and pathogenicity of negative strand viruses, D. Kolokofsky and B. Mahy, Eds., pp. 58-66. Elsevier Press, Amsterdam) as a *BglII* fragment and ligated into *BamHI*-cut pWRG7077 vector. This plasmid expressed G2 but not G1. We solved this problem after discovering that the SEOV M plasmid pWRG/SEO-M, failed to efficiently express G1 if a 24 base pair (bp) sequence of extraneous DNA (SEQ ID NO:6), found between the vector Not I cloning site and the SEOV M noncoding region (Fig. 9A), was removed. This extraneous DNA originated from procedures used during earlier cloning and subcloning of the SEOV gene. The SEOV M construct with the 24 bp sequence removed failed to express G1, but did express G2 (Fig. 9B). Expression of G2 was restored if the 24 bp sequence was engineered back into the construct in the forward but not reverse orientation (Fig 9B). This result indicated that the nucleotide sequence, not nucleotide number, affected G1 expression. To map the sequence that allowed efficient G1 expression, we made constructs which included either nucleotides 1-12 or 13-24 of the extraneous sequence. G1 was expressed when nucleotides 1-12 but not 13-24 was present in the plasmid (Fig. 9B). We noted that nucleotides 4-10 of the DNA (TCTGCAG) were identical to the last seven nucleotides of intron A (*i.e*., splice acceptor site)(Stenberg et al., 1984, J. Virol. 49, 190-199). Mutating the putative splice acceptor dinucleotide AG to CC did not influence the expression of G1, suggesting that, if this region is involved in splicing, it is not dependent on maintaining the exact sequence of the intron A splice site (Fig. 9B). We made a construct containing nucleotides 1-8 of the extraneous DNA and found this was sufficient to restore efficient expression of G1 (Fig. 9C). Thus, we determined that the sequence GGATCTGC, located between the *Not*I cloning site and the 5' noncoding region of the SEOV M gene, was required for efficient expression of G1 from pWRG7077-based DNA vaccines.

Based on the SEOV M findings, we postulated that including the extraneous sequence upstream of the HTNV M gene might allow us to solve our inability to express both HTNV G1 and G2 from a DNA vaccine plasmid. We constructed a plasmid that included the extraneous sequence upstream of the HTNV M gene and, in doing so, obtained a clone that expressed both G1 and G2, pWRG/HTN-M (x) (Fig. 9D and Fig. 10). When the extraneous sequence was removed, G1 was not expressed, and when it was restored, G1 was efficiently expressed (Fig. 9D). These data confirmed that the extraneous sequence was not only required for successful expression of SEOV G1, but also of HTNV G1. This sequence had no influence on expression of G2 from either SEOV or HTNV (Fig. 9). The mechanism by which the extraneous sequence affects G1 expression but not G2 expression remains unknown.

To construct the ANDV M gene-based DNA vaccine plasmid, pWRG/AND-M (SEQ ID NO:8), viral RNA was isolated from ANDV-infected Vero E6 and reverse transcribed. Forward and reverse primers based on the published SNV and PUUV sequences, respectively, were included in the reverse transcription reaction. The forward primer included a *Not* I restriction site (underlined) and 24 nucleotides upstream of the M gene noncoding region that we previously found were important for expressing G1 in pWRG/HTN-M as described above. cDNA was purified and used as template in a PCR reaction. The PCR product was cut with *NotI* and *BamHI* and then ligated into *NotI*-*BglII*-cut pWRG7077 vector to produce pWRG/AND-M. The ANDV M gene sequence was unknown, so we sequenced the M gene, and vector/insert junctions. The full-length M gene of ANDV, strain Chile-9717869, was RT-PCR cloned into pWRG7077 to yield pWRG/AND-M. We sequenced the entire M gene open reading frame. The sequence of our cloned M gene was almost identical to the published M gene sequence of ANDV, GeneBank accession number AF291703, which is not surprising because the viral isolates were from the same rodent specimen (Meisner et al., 2002, Virus Res. 89, 131-143). There were two adenine to guanine nucleotide changes. The change at position 1504 was silent and the change at position 1840 resulted in a threonine to alanine substitution at amino acid 597.

A construct comprising both the Hantaan M gene and the Andes M gene was prepared. The Hantaan M gene and the Andes M gene were cloned into pWRG7077 to produce pWRG/HA-M (SEQ ID NO: 9, Fig. 19). Each M gene was flanked by a cytomegalovirus promoter and intron A (CMV intron A) and a bovine growth hormone poly adenylation site. The entire Hantaan virus and Andes virus M gene open reading frames as well as most of the 5' and 3' noncoding sequences are included in the construct. In addition, the 24 bp sequence positioned between each hantavirus M sequence and its respective CMV intron A sequence. As discussed above, we have found, for unknown reasons, this 24 bp sequence (or a portion thereof) is required for expression of G1 glycoprotein. When pWRG/HA-M is introduced into mammalian cells, the Hantaan virus and Andes virus M genes are expressed. The expression products consist of the Hantaan virus G1 and G2 glycoproteins and the Andes virus G1 and G2 glycoproteins.

It is understood in the art that certain changes to the nucleotide sequence employed in a genetic construct have little or no bearing on the proteins encoded by the construct, for example due to the degeneracy of the genetic code. Such changes result either from silent point mutations or point mutations that encode different amino acids that do not appreciably alter the behavior of the encoded protein. It is also understood that portions of the coding region can be eliminated without affecting the ability of the construct to achieve the desired effect, namely induction of a protective immune response against hantavirus. It is further understood in the art that certain advantageous steps can be taken to increase the antigenicity of an encoded protein by modifying its amino acid composition. Such changes in amino acid composition can be introduced by modifying the genetic sequence encoding the protein. It is contemplated that all such modifications and variations of the M and S segments of hantavirus can be used in combination with the nucleotides of the present invention.

The DNA encoding the desired antigen can be introduced into the cell in any suitable form including, a linearized plasmid, a circular plasmid, a plasmid capable of replication, an episome, RNA, etc. Preferably, the gene is contained in a plasmid. In a particularly preferred embodiment, the plasmid is an expression vector. Individual expression vectors capable of expressing the genetic material can be produced using standard recombinant techniques. Please see e.g., Maniatis et al., 1985 Molecular Cloning: A Laboratory Manual or DNA Cloning, Vol. I and II (D. N. Glover, ed., 1985) for general cloning methods.

Therefore, in another embodiment, the present invention relates to a recombinant DNA molecule that includes a vector and the present nucleotdie sequence. The vector can take the form of a plasmid such as pCRII (Invitrogen) or pJW4303 (Konishi, E. et al., 1992, Virology 188:714), or any expression vector such as viral vectors e.g. adenovirus or Venezuelan equine encephalitis virus and others known in the art. Preferably, a promoter sequence operable in the target cell is operably linked to the DNA sequence. Several such promoters are known for mammalian systems which may be joined 5', or upstream, of the coding sequence for the encoded protein to be expressed. A suitable promoter is the human cytomegalovirus immediate early promoter. A downstream transcriptional terminator, or polyadenylation sequence, such as the polyA addition sequence of the bovine growth hormone gene, may also be added 3' to the protein coding sequence.

A suitable construct for use with the present invention is pWRG7077 (4326 bp)(PowderJect Vaccines, Inc., Madison, WI), Figure 1. pWRG7077 includes a human cytomegalovirus (hCMV) immediate early promoter (IE) and a bovine growth hormone polyA addition site. Between the promoter and the polyA addition site is Intron A, a sequence that naturally occurs in conjunction with the hCMV IE promoter that has been demonstrated to increase transcription when present on an expression plasmid. Downstream from Intron A, and between Intron A and the polyA addition sequence, are unique cloning sites into which the hantavirus M or S DNA can be cloned. Also provided on pWRG7077 is a gene that confers bacterial host-cell resistance to kanamycin. Any of the fragments that encode hantavirus G1 and/or G2 or nucleocapsid peptides can be cloned into one of the cloning sites in pWRG7077, using methods known to the art.

Host cells may be stably transformed or transfected with the above-described recombinant DNA constructs. The host cell can be prokaryotic such as *Bacillus* or *E. coli*, or eukaryotic such a *Saccharomyces* or *Pichia*, or mammalian cells or insect cells. The vector containing the hantavirus sequence is expressed in the bacteria and the expressed product used for diagnostic procedures or as a vaccine. Please see e.g., Maniatis et al., 1985 Molecular Cloning: A Laboratory Manual or DNA Cloning, Vol. I and II (D. N. Glover, ed., 1985) for general cloning methods. The DNA sequence can be present in the vector operably linked to a highly purified IgG molecule, an adjuvant, a carrier, or an agent for aid in purification of hantavirus proteins or peptides.
The transformed or transfected host cells can be used as a source of DNA sequences described above. When the recombinant molecule takes the form of an expression system, the transformed or transfected cells can be used as a source of the protein or peptide encoded by the DNA. The DNA can be used as circular or linear, or linearized plasmid as long as the hantavirus sequences are operably linked to a promoter which can be expressed in the transfected cell.

In this application we describe the elicitation of protective immunity to hantaviruses by DNA vaccines. The DNA can be delivered by injection into the tissue of the recipient, oral or pulmonary delivery and inoculation by particle bombardment (i.e., gene gun). Any of these methods can be used to deliver DNA as long as the DNA is expressed and the desired antigen is made in the cell. Two methods are exemplified in this application, both shown to be successful in eliciting a protective immune response in the vaccinee.

To deliver DNA vaccines by particle bombardment, we chose to use the *PowderJect-XR*™ gene gun device described in WO 95/19799, 27 July 1995. Other instruments are available and known to people in the art. This instrument, which delivers DNA-coated gold beads directly into epidermal cells by high-velocity particle bombardment, was shown to more efficiently induce both humoral and cell-mediated immune responses, with smaller quantities of DNA, than inoculation of the same DNAs by other parenteral routes (Eisenbraun, M. et al., 1993, DNA Cell. Biol. 12: 791; Fynan, E. F. et al., 1993, Proc. Natl. Acad. Sci. USA 90: 11478; Haynes, J. R. et al., 1994, AIDS Res. Hum. Retroviruses 10: Suppl. 2:S43; Pertmer, T. M. et al., 1995, Vaccine 13: 1427). Epidermal inoculation of the DNA candidate vaccines also offers the advantages of gene expression in an immunologically active tissue that is generally exfoliated within 15 to 30 days, and which is an important natural focus of viral replication after tick-bite (Bos, J. D., 1997, Clin. Exp. Immunol. 107 Suppl. 1:3; Labuda, M. et al., 1996, Virology 219:357; Rambukkana, A. et al., 1995, Lab. Invest. 73:521; Stingl, G., 1993, Recent Results Cancer Res. 128:45).

Candidate vaccines include particles having a suitable promoter, the present sequence and a hantavirus M genome segment, encoding G1 and/or G2, from the Hantaan hantavirus and, optionally one or more different HFRS-associated viruses such as Seoul virus, Hantaan virus, Pumuula virus, and Dobrava virus, as well as one or more HPS-associated virus such as Sin Nombre virus, Black Creek Canal virus, Bayou virus, New York virus, Andes virus, and Laguna Negra virus, or any combination thereof. The DNA segments from different viruses can be on different particles or on the same particle, whichever results in the desired immune response. In addition, the present invention relates to a vaccine comprising the present sequence and two or more DNAs from different hantavirus M segments, at least one of which is a Hantaan hantavirus, each M segment encoding G1 and G2 from its respective hantavirus, operatively linked to a promoter active in cells of a mammal. Such a vaccine is referred to as a multivalent vaccine. The vaccine is designed to protect against pathologies resulting from exposure to one or several hantaviruses. The vaccine can also be combined with reagents which increase the antigenicity of the vaccine, or reduce its side effects.

The technique of accelerated particles gene delivery or particle bombardment is based on the coating of DNA to be delivered into cells onto extremely small carrier particles, which are designed to be small in relation to the cells sought to be transformed by the process. The DNA sequence containing the desired gene can be simply dried onto a small inert particle. The particle may be made of any inert material such as an inert metal (gold, silver, platinum, tungsten, etc.) or inert plastic (polystyrene, polypropylene, polycarbonate, etc.). Preferably, the particle is made of gold, platinum or tungsten. Most preferably, the particle is made of gold. Suitably, the particle is spherical and has a diameter of 0.5 to 5 microns, preferably 1 to 3 microns.

The DNA sequence containing the desired gene prepared in the form suitable for gene introduction can be simply dried onto naked gold or tungsten pellets. However, DNA molecules in such a form may have a relatively short period of stability and may tend to degrade rather rapidly due to chemical reactions with the metallic or oxide substrate of the particle itself. Thus, if the carrier particles are first coated with an encapsulating agent, the DNA strands have greatly improved stability and do not degrade significantly even over a time period of several weeks. A suitable encapsulating agent is polylysine (molecular weight 200,000) which can be applied to the carrier particles before the DNA molecules are applied. Other encapsulating agents, polymeric or otherwise, may also be useful as similar encapsulating agents, including spermidine. The polylysine is applied to the particles by rinsing the gold particles in a solution of 0.02% polylysine and then air drying or heat drying the particles thus coated. Once the metallic particles coated with polylysine were properly dried, DNA strands are then loaded onto the particles.

The DNA is loaded onto the particles at a rate of between 0.5 and 30 micrograms of DNA per milligram of gold bead spheres. A preferable ratio of DNA to gold is 0.5-5.0 ug of DNA per milligram of gold. A sample procedure begins with gamma irradiated (preferably about 30 kGy) tefzel tubing. The gold is weighed out into a microfuge tube, spermidine (free base) at about 0.05 M is added and mixed, and then the DNA is added. A 10% CaCl solution is incubated along with the DNA for about 10 minutes to provide a fine calcium precipitate. The precipitate carries the DNA with it onto the beads. The tubes are microfuged and the pellet resuspended and washed in 100% ethanol and the final product resuspeded in 100% ethanol at 0.0025mg/ml PVP. The gold with the DNA is then applied onto the tubing and dried.

The general approach of accelerated particle gene transfection technology is described in U.S. Patent No. 4,945,050 to Sanford. An instrument based on an improved variant of that approach is available commercially from PowderJect Vaccines, Inc., Madison Wisconsin, and is described in WO 95/19799. All documents cited herein *supra* and infra are hereby incorporated in their entirety by reference thereto. Briefly, the DNA-coated particles are deposited onto the interior surface of plastic tubing which is cut to a suitable length to form sample cartridges. A sample cartridge is placed in the path of a compressed gas (e.g., helium at a pressure sufficient to dislodge the particles from the cartridge e.g., 350-400 psi). The particles are entrained in the gas stream and are delivered with sufficient force toward the target tissue to enter the cells of the tissue. Further details are available in the published apparatus application.

The coated carrier particles are physically accelerated toward the cells to be transformed such that the carrier particles lodge in the interior of the target cells. This technique can be used either with cells in vitro or in vivo. At some frequency, the DNA which has been previously coated onto the carrier particles is expressed in the target cells. This gene expression technique has been demonstrated to work in prokaryotes and eukaryotes, from bacteria and yeasts to higher plants and animals. Thus, the accelerated particle method provides a convenient methodology for delivering genes into the cells of a wide variety of tissue types, and offers the capability of delivering those genes to cells in situ and in vivo without any adverse impact or effect on the treated individual. Therefore, the accelerated particle method is also preferred in that it allows a DNA vaccine capable of eliciting an immune response to be directed both to a particular tissue, and to a particular cell layer in a tissue, by varying the delivery site and the force with which the particles are accelerated, respectively. This technique is thus particularly suited for delivery of genes for antigenic proteins into the epidermis.

A DNA vaccine can be delivered in a non-invasive manner to a variety of susceptible tissue types in order to achieve the desired antigenic response in the individual. Most advantageously, the genetic vaccine can be introduced into the epidermis. Such delivery, it has been found, will produce a systemic humoral immune response.

To obtain additional effectiveness from this technique, it may also be desirable that the genes be delivered to a mucosal tissue surface, in order to ensure that mucosal, humoral and cellular immune responses are produced in the vaccinated individual. There are a variety of suitable delivery sites available including any number of sites on the epidermis, peripheral blood cells, i.e. lymphocytes, which could be treated in vitro and placed back into the individual, and a variety of oral, upper respiratory, and genital mucosal surfaces.

Gene gun-based DNA immunization achieves direct, intracellular delivery of DNA, elicits higher levels of protective immunity, and requires approximately three orders of magnitude less DNA than methods employing standard inoculation.

Moreover, gene gun delivery allows for precise control over the level and form of antigen production in a given epidermal site because intracellular DNA delivery can be controlled by systematically varying the number of particles delivered and the amount of DNA per particle. This precise control over the level and form of antigen production may allow for control over the nature of the resultant immune response.

The term transfected is used herein to refer to cells which have incorporated the delivered foreign DNA vaccine, whichever delivery technique is used.

It is herein disclosed that when inducing cellular, humoral, and protective immune responses after DNA vaccination the preferred target cells are epidermal cells, rather than cells of deeper skin layers such as the dermis. Epidermal cells are preferred recipients of DNA vaccines because they are the most accessible cells of the body and may, therefore, be immunized non-invasively. Secondly, in addition to eliciting a humoral immune response, DNA immunized epidermal cells also elicit a cytotoxic immune response that is stronger than that generated in sub-epidermal cells. Delivery to epidermis also has the advantages of being less invasive and delivering to cells which are ultimately sloughed by the body.

Although it can be desirable to induce an immune response by delivering genetic material to a target animal, merely demonstrating an immune response is not necessarily sufficient to confer protective advantage on the animal. What is important is to achieve a protective immune response that manifests itself in a clinical difference. That is, a method is effective only if it reduces the severity of the disease symptoms. It is preferred that the immunization method be at least 20% effective in preventing death in an immunized population after challenge with hantavirus. More preferably, the vaccination method is 50% or more effective, and most preferably 70-100% effective, in preventing death in an immunized population. The vaccination method is shown herein to be 100% effective in the hamster model for hantavirus. Hamsters have been used extensively as the laboratory model of choice for assessment of protective immune responses to hantaviruses. In contrast, unimmunized animals are uniformly infected by challenge with hantavirus. Our results indicate that vaccination with SEOV M genome segment protects against infection with Seoul virus Hantaan virus (HTNV), and Dobrava virus (DBOV). Vaccination with HTNV M genome segment protects against infection Hantaan virus, Seoul virus and Dobrava virus. Vaccination with ANDV M genome segment protects against infection with ANDV, Sin Nombre Virus and Black Creek Canal virus. Vaccination with ANDV-M/HTNV-M segments protects against infection with SEOV, HTNV, DBOV, ANDV, Sin Nombre, and Black Creek virus among other hantaviruses.

Generally, the DNA vaccine administered may be in an amount of about 1-8 ug of DNA per dose and will depend on the subject to be treated, capacity of the subject's immune system to develop the desired immune response, and the degree of protection desired. Precise amounts of the vaccine to be administered may depend on the judgement of the practitioner and may be peculiar to each subject and antigen.

The vaccine for eliciting an immune response against one or more viruses, may be given in a single dose schedule, or preferably a multiple dose schedule in which a primary course of vaccination may be with 1-8 separate doses, followed by other doses given at subsequent time intervals required to maintain and or reinforce the immune response, for example, at 1-4 months for a second dose, and if needed, a subsequent dose(s) after several months. Examples of suitable immunization schedules include: (i) 0, 1 months and 6 months, (ii) 0, 7 days and 1 month, (iii) 0 and 1 month, (iv) 0 and 6 months, or other schedules sufficient to elicit the desired immune responses expected to confer protective immunity, or reduce disease symptoms, or reduce severity of disease.

Reagents useful for carrying out the present process may comprise a DNA fragment containing M or S or both gene segments from one or more hantavirus, and a small, inert, dense particle. The DNA fragment, and dense particle are those described above.

The DNA may be frozen or lyophilized, and the small, inert, dense particle is in dry powder. If a coating solution is used, the dry ingredients for the coating solution may be premixed and premeasured and contained in a container such as a vial or sealed envelope.

Kits may also be useful for use with the present invention. Such kits may comprise a first container means containing the above-described frozen or lyophilized DNA. Such kits may also comprise a second container means which contains the coating solution or the premixed, premeasured dry components of the coating solution. Such kits may also comprise a third container means which contains the small, inert, dense particles in dry powder form or suspended in 100% ethanol. These container means can be made of glass, plastic or foil and can be a vial, bottle, pouch, tube, bag, etc. Such kits may also contain written information, such as procedures for carrying out the present invention or analytical information, such as the amount of reagent (e.g. moles or mass of DNA) contained in the first container means. The written information may be on any of the first, second, and/or third container means, and/or a separate sheet included, along with the first, second, and third container means, in a fourth container means. The fourth container means may be, e.g. a box or a bag, and may contain the first, second, and third container means.

Also disclosed are polyclonal antibodies from vaccinees receiving the DNA vaccines described above. The term "antibody" is art-recognized terminology and is intended to include molecules or active fragments of molecules that bind to known antigens. These active fragments can be derived from an antibody by a number of techniques. For further description of general techniques for the isolation of active fragments of antibodies, see for example, Khaw, B. A. et al. J. Nucl. Med. 23:1011-1019 (1982). The term "antibody" also includes bispecific and chimeric antibodies.

The polyclonal antibodies described in the Examples below are characterized in that the antibody binds to the appropriate immunogen, i.e. G1 and G2, as measured by assays such as ELISA, immunoprecipitation, or immunofluorescence. Also, the antibodies must neutralize hantavirus as measured by plaque reduction neutralization test (PRNT). The polyclonal antibody may be concentrated, irradiated, and tested for a capacity to neutralize Andes virus and Hantaan virus, among other hantaviruses of interest. Serum lots with sufficiently high neutralizing antibody titers, i.e., high enough to give a detectable response in the recipient after transfer can be pooled. The product can then be lyophilized for storage and reconstituted for use.

As described in greater detail in the examples, the present inventors have found that serum from a vaccinee immunized with a DNA vaccine comprising the M segment of a hantavirus, e.g. Hantaan or Andes, contains antibodies able to neutralize hantavirus and display in vitro and in vivo hantavirus neutralization properties. Significantly, the reactivity of the antibodies is applicable against a broad variety of different hantavirus, both HFRS hantavirus and HPS hantavirus.

Given these results, polyclonal antibodies are suitable both as therapeutic and prophylactic agents for treating or preventing hantavirus infection or disease in susceptible hantavirus-exposed subjects. Subjects include rodents such as mice or guinea pigs, birds or avian, and mammals, including humans.

In general, this will comprise administering a therapeutically or prophylactically effective amount of polyclonal antibodies to a subject after possible exposure to hantavirus or to a subject exhibiting hantavirus symptoms. Any active form of the antibodies can be administered. Antibodies can be produced in any system, including insect cells, baculovirus expression systems, chickens, rabbits, goats, cows, or plants such as tomato, potato, banana or strawberry. Methods for the production of antibodies in these systems are known to a person with ordinary skill in the art. Preferably, the antibodies used are compatible with the recipient species such that the immune response to the antibodies does not result in clearance of the antibodies before virus can be controlled, and the induced immune response to the antibodies in the subject does not induce "serum sickness" in the subject.

Treatment of individuals having hantavirus infection may comprise the administration of a therapeutically effective amount of anti-hantavirus antibodies. The antibodies can be provided in a kit as described below. In providing a patient with antibodies, or fragments thereof, capable of binding to hantavirus, or an antibody capable of protecting against hantavirus in a recipient patient, the dosage of administered agent will vary depending upon such factors as the patient's age, weight, height, sex, general medical condition, previous medical history, etc.

In general, it is desirable to provide the recipient with a dosage of antibody which is in the range of from about 1 pg/kg-100 pg/kg, 100 pg/kg-500 pg/kg, 500 pg/kg-1 ng/kg, 1 ng/kg-100 ng/kg, 100 ng/kg-500 ng/kg, 500 ng/kg- 1 ug/kg, 1 ug/kg- 100 ug/kg, 100 ug/kg-500 ug/kg, 500 ug/kg- 1 mg/kg, 1 mg/kg-50 mg/kg, 50 mg/kg-100 mg/kg, 100 mg/kg-500 mg/kg, 500 mg/kg-1 g/kg, 1 g/kg-5 g/kg, 5 g/kg-10 g/kg (body weight of recipient), although a lower or higher dosage may be administered.

The antibodies capable of protecting against hantavirus are intended to be provided to recipient subjects in an amount sufficient to effect a reduction in the hantavirus infection symptoms. An amount is said to be sufficient to "effect" the reduction of infection symptoms if the dosage, route of administration, etc. of the agent are sufficient to influence such a response. Responses to antibody administration can be measured by analysis of subject's vital signs.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient.

The sequences of the present invention can be formulated according to known methods to prepare pharmaceutically useful compositions, whereby these materials are combined in admixture with a phamaceutically acceptable carrier vehicle. Suitable vehicles and their formulation, inclusive of other human proteins, e.g., human serum albumin, are described, for example, in Remington's Pharmaceutical Sciences (16th ed., Osol, A. ed., Mack Easton Pa. (1980)). In order to form a pharmaceutically acceptable composition suitable for effective administration, such compositions will contain an effective amount of the present sequences together with a suitable amount of carrier vehicle.

Additional pharmaceutical methods may be employed to control the duration of action. Control release preparations may be achieved through the use of polymers to complex or absorb the compounds. The controlled delivery may be exercised by selecting appropriate macromolecules (for example polyesters, polyamino acids, polyvinyl, pyrrolidone, ethylenevinylacetate, methylcellulose, carboxymethylcellulose, or protamine sulfate) and the concentration of macromolecules as well as the method of incorporation in order to control release. Another possible method to control the duration of action by controlled release preparations is to incorporate the present sequences of the present invention into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, interfacial polymerization, for example, hydroxymethylcellulose or gelatin-microcapsules and poly(methylmethacylate)-microcapsules, respectively, or in colloidal drug delivery systems, for example, liposomes, albumin microspheres, microemulsions, nanoparticles, and nanocapsules or in macroemulsions. Such techniques are disclosed in *Remington's Pharmaceutical Sciences* (1980).

Administration of the antibodies disclosed herein may be carried out by any suitable means, including parenteral injection (such as intraperitoneal, subcutaneous, or intramuscular injection), *in ovo* injection of birds, orally, or by topical application of the antibodies (typically carried in a pharmaceutical formulation) to an airway surface. Topical application of the antibodies to an airway surface can be carried out by intranasal administration (e.g., by use of dropper, swab, or inhaler which deposits a pharmaceutical formulation intranasally). Topical application of the antibodies to an airway surface can also be carried out by inhalation administration, such as by creating respirable particles of a pharmaceutical formulation (including both solid particles and liquid particles) containing the antibodies as an aerosol suspension, and then causing the subject to inhale the respirable particles. Methods and apparatus for administering respirable particles of pharmaceutical formulations are well known, and any conventional technique can be employed. Oral administration may be in the form of an ingestable liquid or solid formulation.

The treatment may be given in a single dose schedule, or preferably a multiple dose schedule in which a primary course of treatment may be with 1-10 separate doses, followed by other doses given at subsequent time intervals required to maintain and or reinforce the response, for example, at 1-4 months for a second dose, and if needed, a subsequent dose(s) after several months. Examples of suitable treatment schedules include: (i) 0, 1 month and 6 months, (ii) 0, 7 days and 1 month, (iii) 0 and 1 month, (iv) 0 and 6 months, or other schedules sufficient to elicit the desired responses expected to reduce disease symptoms, or reduce severity of disease.

Also disclosed herein is a method for detecting hantavirus in a sample suspected of containing hantavirus. The method includes contacting the sample with polyclonal antibodies of the present invention which bind hantavirus antigens, allowing the antibody to bind to the hantavirus antigen(s) to form an immunological complex, detecting the formation of the immunological complex and correlating the presence or absence of the immunological complex with the presence or absence of hantavirus antigen in the sample. The sample can be biological, environmental or a food sample.

The language "detecting the formation of the immunological complex" is intended to include discovery of the presence or absence of hantavirus antigen in a sample. The presence or absence of hantavirus antigen can be detected using an immunoassay. A number of immunoassays used to detect and/or quantitate antigens are well known to those of ordinary skill in the art. See Harlow and Lane, Antibodies: A Laboratory Manual (Cold Spring Harbor Laboratory, New York 1988 555-612). Such immunoassays include antibody capture assays, antigen capture assays, and two-antibody sandwich assays. These assays are commonly used by those of ordinary skill in the art. In an antibody capture assay, the antigen is attached to solid support, and labeled antibody is allowed to bind. After washing, the assay is quantitated by measuring the amount of antibody retained on the solid support. A variation of this assay is a competitive ELISA wherein the antigen is bound to the solid support and two solutions containing antibodies which bind the antigen, for example, serum from a hantavirus virus vaccinee and the polyclonal antibodies of the present invention, are allowed to compete for binding of the antigen. The amount of polyclonal antibody bound is then measured, and a determination is made as to whether the serum contains anti hantavirus antigen antibodies. This competitive ELISA can be used to indicate immunity to known protective epitopes in a vaccinee following vaccination.

In an antigen capture assay, the antibody is attached to a solid support, and labeled antigen is allowed to bind. The unbound proteins are removed by washing, and the assay is quantitated by measuring the amount of antigen that is bound. In a two-antibody sandwich assay, one antibody is bound to a solid support, and the antigen is allowed to bind to this first antibody. The assay is quantitated by measuring the amount of a labeled second antibody that can bind to the antigen.

These immunoassays typically rely on labeled antigens, antibodies, or secondary reagents for detection. These proteins can be labeled with radioactive compounds, enzymes, biotin, or fluorochromes. Of these, radioactive labeling can be used for almost all types of assays and with most variations. Enzyme-conjugated labels are particularly useful when radioactivity must be avoided or when quick results are needed. Biotin-coupled reagents usually are detected with labeled streptavidin. Streptavidin binds tightly and quickly to biotin and can be labeled with radioisotopes or enzymes. Fluorochromes, although requiring expensive equipment for their use, provide a very sensitive method of detection. Antibodies useful in these assays include monoclonal antibodies, polyclonal antibodies, and affinity purified polyclonal antibodies. Those of ordinary skill in the art will know of other suitable labels which may be employed in accordance with the present invention. The binding of these labels to antibodies or fragments thereof can be accomplished using standard techniques commonly known to those of ordinary skill in the art. Typical techniques are described by Kennedy, J. H., et al., 1976 (Clin. Chim. Acta 70:1-31), and Schurs, A. H. W. M., et al. 1977 (Clin. Chim Acta 81:1-40). Coupling techniques mentioned in the latter are the glutaraldehyde method, the periodate method, the dimaleimide method, and others, all of which are incorporated by reference herein.

The language "biological sample" is intended to include biological material, e.g. cells, tissues, or biological fluid. By "environmental sample" is meant a sample such as soil and water. Food samples include canned goods, meats, and others.

A kit for detecting hantavirus in a biological sample may include a container holding one or more polyclonal antibodies which bind a hantavirus antigen and instructions for using the antibody for the purpose of binding to hantavirus antigen to form an immunological complex and detecting the formation of the immunological complex such that the presence or absence of the immunological complex correlates with presence or absence of hantavirus in the sample. Examples of containers include multiwell plates which allow simultaneous detection of hantavirus in multiple samples.

The following materials and method were used in the examples below.

### MATERIALS AND METHODS

### Viruses, cells, and medium

HTNV, strain 76-118 (Lee, et al., 1978, J. Infect. Dis. 137, 298-308), ANDV strain Chile-9717869 (Hooper et al., 2001, Virology 289, 6-14), BCCV (Rollin et al., 1995, J. Med. Virol. 46, 35-39), and SNV strain CC107 (Schmaljohn et al., 1995, Virology 206, 963-972) were propagated in Vero E6 cells (Vero C1008; ATCC CRL 1586). Transient expression experiments were performed with COS cells (COS-7; ATTC CRL1651). Both cell types were maintained in Eagle's minimal essential medium with Earle's salts (EMEM) containing 10% fetal bovine serum (FBS), 10 mM HEPES pH 7.4, and antibiotics (penicillin [100 U/ml], streptomycin [100 µg/ml], and gentamicin sulfate [50 µg/ml])(cEMEM) at 37°C in a 5% CO₂ incubator.

HTNV G2-specific monoclonal antibodies (MAbs): MAb-23G10, MAb-11E10, MAb-3D7, MAb-16E6, and MAb-HC02; and HTNV G1-specific MAbs: MAb-2D5, MAb-6D4, MAb-8B6, MAb-3D5, and MAb-10F11 were described previously (Arikawa, et al., 1989, J. Gen. Virol. 70, 615-24, 24). MAb-HC02 and MAbE606 was provided by Dr. J. McCormick (Centers for Disease Control, Atlanta, Georgia, Ruo et al., 1991, Arch. Virol. 119, 1-11).

**Construction of SEOV M and S naked DNA plasmids.** RT-PCR cloning of the M and S genome segments of SEOV, strain SR-11, was described previously (Arikawa et al., 1990, Virology 176, 114-125). The cDNA representing each genome segment was subcloned into the *Not* I or *Bam* HI site of a previously described naked DNA vector pWRG7077 (Schmaljohn et al., 1997, J. Virol. 64, 3162-3170). Plasmid DNA was purified using Qiagen maxiprep DNA purification kits according to the manufacturer's directions (Cat. no. 12163, Qiagen). The DNA we used for both intramuscular needle inoculation and epidermal gene gun inoculation was not prepared using endo-free qiagen DNA purification kits, and therefore, the DNA was not endotoxin-free (Qiagen plasmid kits yield -9.3 endotoxin units per µg DNA [QIAGEN Plasmid Purification Handbook 01.97]). To control for possible immunostimulatory effects of endotoxin, our negative control plasmid DNA was prepared in precisely the same was as the vaccine candidates.

### Construction of hantavirus M gene DNA vaccine plasmids

Modified versions of pWRG/SEO-M were made as follows.

SEOV M DNA was amplified by polymerase chain reaction (PCR) from pWRG/SEO-M by using a forward primer [primer0, 5'-GCGCGCGGCCGCAGTAGTAGACTCCGCAAGAAAC (SEQ ID NO:10)], and a reverse primer [BACK, 5'-GCGCGGATCCCGGGTACCGGGCCCCCCCTCG (SEQ ID NO:11)]. PCR product was cut with *NotI* and *BamHI* and then ligated into *NotI*-*BamHI*-cut pWRG7077 vector to create pWRG/SEO-M(0).

SEOV M DNA was amplified by PCR from pWRG/SEO-M(0) by using a forward primer [primer1-24, 5' GGCCGCGGCCGCGGATCTGCAGGAATTCGGCACGAGAGTAGTAGACTCCGCAAG AAACAGCA (SEQ ID NO:12], or [primer24-1, 5'-GGCCGCGGCCGCGAGCACGGCTTAAGGACGTCTAGGAGTAGTAGTCTCCGCAAG AAACAGCA (SEQ ID NO:13)], or [primer1-12, 5'-GGCCGCGGCCGCATTCGGCACGAGAGTAGTAGACTCCGCAAGAAACAGCA (SEQ ID NO:14], or [primer13-24, 5' GGCCGCGGCCGCGGATCTGCAGGAAGTAGTAGACTCCGCAAGAAACAGCA (SEQ ID NO:15] or [primer1-24*, 5' GGCCGCGGCCGCGGATCTGCCCGAATTCGGCACGAGAGTAGTAGACTCCGCAAG AAACAGCA (SEQ ID NO:16] or [primer1-8, 5'GGCCGCGGCCGCGGATCTGCAGTAGTAGACTCCGCAAGAAACAGCA (SEQ ID NO:17] and the reverse primer BACK. PCR products were cut with *NotI* and *BamHI* and then ligated into *NotI*-*BamHI*-cut pWRG7077 vector to create, pWRG/SEO-M(1-24), pWRG/SEO(24-1), pWRG/SEO-M(1-12), pWRG/SEO-M(13-24), pWRG/SEO-M(1-24*), and pWRG/SEO-M(1-8), respectively.

To make pWRG/SEO-M(x), DNA encoding the SEOV G1/G2 proteins was amplified by PCR from pWRG/SEO-M by using primer1-24 and a reverse primer [SEOMX, 5'-GCGCGGATCCAGATTGGGAGATAGAAGAGAG (SEQ ID NO:18] . PCR product was cut with *NotI* and *BamHI* and then ligated into *NotI*-*BglII*-cut pWRG7077 vector. This clone was made to remove undesirable cloning artifact DNA (~100 nucleotides of the simian immunodeficiency virus nef gene) found between the *BamHI* and *BglII* sites of pWRG7077. Removing this sequence had no effect on expression of cloned genes (data not shown).

The HTNV M DNA vaccine plasmid, pWRG/HTN-M, was constructed essentially as follows. First, DNA encoding the HTNV G1/G2 was cut from pTZ19RHTNMm (Schmaljohn *et al*., 1989, supra) as a *BglII* fragment and ligated into *BamHI*-cut pWRG7077 vector. This plasmid expressed G2 but not G1 (data not shown). Next, the *NotI-PshAI* fragment of this plasmid, which contained the 5' end of the M gene, was excised and replaced with DNA amplified by PCR (from a puc18 plasmid containing a full-length HTNV M gene cloned by reverse transcriptase-PCR cloning from viral RNA), using primer1-24 (see above) and a reverse primer [M5B, 5'-TCAGGACTCCTGTCATGCAATAAGATCTC (SEQ ID NO:19]. The reverse primer included silent nucleotide changes in the HTNV M gene that created a *BglII* site used for diagnostic purposes. The PCR product was cut with *NotI* and *PshAI* and ligated into the *NotI*-*PshAI*-cut plasmid to create pWRG/HTN-M.

pWRG/HTN-M(x) was constructed by using primer1-24 and a reverse primer [HTNMX, 5'-GCGCGGATCCGTTTGTGGTTAGAAAGCTAC (SEQ ID NO:20] to PCR amplify the HTNV M gene from pWRG/HTN-M. PCR product was cut with *NotI* and *BamHI* and ligated into the *NotI-BglII*-cut pWRG7077 vector. This clone was identical to pWRG/HTN-M; however, a portion of the 3' nontranslated region of the gene, and the vector sequence between *NotI* and *BglII* was removed.

pWRG/HTN-M(0) was constructed by using primer0 and reverse primer HTNMX to PCR amplify the HTNV M gene from pWRG/HTN-M(x). PCR product was cut with *NotI* and *BamHI* and ligated into the *NotI-BamHI* site of pWRG7077.

pWRG/HTN-M(1-24) was constructed by using primer1-24 and reverse primer HTNMX to PCR amplify the HTNV M gene from pWRG/HTN-M(0). PCR product was cut with *NotI* and *BamHI* and ligated into the *NotI-BamHI* site of pWRG7077.

Plasmid DNA was purified by using Qiagen maxiprep DNA purification kits according to the manufacturer's directions.

To construct the ANDV M gene-based DNA vaccine plasmid, pWRG/AND-M, viral RNA was isolated from ANDV-infected Vero E6 cells using TRIzol (Invitrogen, Carlsbad, Calif.) by standard methods. The RNA was reverse transcribed using Superscript II reverse transcriptase (Invitrogen) at 50°C for 50 min, then inactivated by incubation at 70°C 15 min. RNA was removed by digestion with RNaseH 37°C for 20 min. Forward and reverse primers based on the published SNV and PUUV sequences, respectively, were included in the reverse transcription reaction: forward primer [SN-Fj, 5'-GGCCGCGGCCGCGGATCTGCAGGAATTCGGCACGAGAGTAGTAGACTCCGCACG AAGAAGC (SEQ ID NO:21)] and reverse primer [PUUM-R, 5'-GCGCGGATCCTAGTAGTATGCTCCGCAGGAAC (SEQ ID NO:22)]. The forward primer included a *Not* I restriction site (underlined) and 24 nucleotides upstream of the M gene noncoding region that we previously found were important for expressing G1 in pWRG/HTN-M(x). The reverse primer included a *BamH* I restriction site (underlined). cDNA was purified by a PCR purification kit (Qiagen) and used as template in a PCR reaction. Primers SN-Fj and PUUM-R were included in the PCR reaction which included Platinum Taq High Fidelity DNA polymerase (Invitrogen): one 3-min cycle at 94°C followed by 30 cycles of 94°C 30 s, 68°C 8 min. The PCR product was cut with *NotI* and *BamHI* and then ligated into *NotI-BglII-cut* pWRG7077 vector to produce pWRG/AND-M(1.1), hitherto referred to as pWRG/AND-M (SEQ ID NO:8). Plasmid DNA was purified using Qiagen maxiprep DNA purification kits according to the manufacturer's directions. At the time this plasmid was constructed, the ANDV M gene sequence was unknown, so we sequenced the M gene, and vector/insert junctions, using a primer walking technique and an ABI 3100 genetic analyzer.

### Indirect fluorescent antibody test (IFAT)

IFAT was a modification of a previously described procedure (Kamrud et al., 1995, Exp. Parasitol. 81, 394-403). COS cells grown on 15mm glass cover slips in 12-well cell culture plates were transfected with 1 µg of plasmid DNA using Fugene6 (Boehringer Mannheim) as described by the manufacturer. Twenty-four hr posttransfection, cover slips were rinsed once with PBS (pH 7.4), fixed with acetone for 10 min at room temperature, and then reacted with anti-SEOV polyclonal antiserum (rabbit), or sera from vaccinated animals. Antibodies were diluted in PBS containing 3% fetal bovine serum (FBS) and then incubated on transfected cells for 30 min at 37°C. Cover slips were rinsed three times with PBS and incubated for 30 min at 37°C with biotinylated donkey anti-rabbit followed by streptavidin conjugated fluorescein (Amersham), or fluorescein-labeled goat anti-hamster antibody (Kirkegaard & Perry Laboratories) diluted in PBS,3% FBS. Evans blue (Fisher) was included in the secondary antibody solution as a counter stain. Cover slips were rinsed three times with PBS and once with deionized water and then were placed on a drop of fluorescence mounting medium (DAKO) on glass slides. Cells were observed with a Zeiss Axioplan fluorescence microscope.

For HTNV, Hamster sera were diluted 1:200 in PBS containing 3% FBS and then incubated on transfected cells for 1 h at 37°C. Cover slips were rinsed three times with PBS and incubated for 30 min at 37°C with a fluorescein-labeled goat anti-hamster IgG antibody (Kirkegaard & Perry Laboratories). Hoechst stain (1 ug per ml) was included in the secondary antibody solution as a counter stain. Cover slips were rinsed three times with PBS, once with deionized water, and then placed on a drop of fluorescence mounting medium (DAKO) on glass slides. Cells were observed with a Nikon E600 fluorescence microscope.

### Immunoprecipitation

COS cells grown in T-25 cell culture flasks were transfected with 5-8 µg of plasmid DNA with Fugene6 (Boehringer Mannheim). After 24 hr, expression products were radio-labeled with Promix ([³⁵S]-methionine and [³⁵S]-cysteine, Amersham) and immunoprecipitated as described previously (Schmaljohn et al., 1997, Emerg. Infect. Dis. 3, 95-104). Reduced samples were run on 4-12% Bis-Tris SDS PAGE gradient gels with 3-(N-morpholino) propane sulfonic acid (MOPS) running buffer (NuPage), at 200V constant voltage.

### Vaccination with the gene gun

Cartridges for the gene gun were prepared as described previously (Eisenbraun et al., 1993, DNA Cell. Biol. 12, 791-797; Schmaljohn et al. 1997, supra). Briefly, ~1.5 µg of DNA was precipitated onto 0.5 mg of - 2 µm diameter gold beads (Degussa). DNA-coated gold beads were used to coat the inner surface of Tefzel tubing (McMaster-Carr). Cartridges were made by cutting the DNA-gold-coated Tefzel tubing into -0.5-inch sections, each containing ∼0.5 mg of gold, coated with ∼0.75 µg of DNA (-50% of the precipitated DNA bound to the gold as determined by elution and fluorometric assay). To vaccinate animals, abdominal fur was removed with clippers and DNA-coated gold was administered to nonoverlapping sites on the abdominal epidermis by using the gene gun (Powderject-XR delivery device, Powderject Vaccines, Inc.) as described previously (Pertmer et al., 1995, Vaccine 13, 1427-1430). BALB/c mice (National Cancer Institute) and outbred Syrian hamsters (Charles River) were vaccinated with two or four cartridges, respectively, under 400 p.s.i. of helium pressure. This research was conducted in accordance with procedures described in the Guide for the Care and Use of Laboratory Animals (National Institute of Health, 1996). The facilities are fully accredited by the American Association for Accreditation of Laboratory Animal Care.

For HTNV, gene gun cartridges (-0.5 µg of plasmid DNA coated on 0.5 mg of gold) were prepared, and outbred golden Syrian hamsters were gene gun-vaccinated as described above. Hamsters were vaccinated three times at 3 week intervals. Rhesus macaques were vaccinated with the same cartridges and the same gene gun conditions used to vaccinate the hamsters; however, the monkeys received eight administrations per vaccination, rather than four.

Rhesus macaques were vaccinated with a recombinant vaccinia virus, rVV/HTN-M+S, by the method used to vaccinate humans in a Phase II clinical trial (McClain *et al.,* 2000, supra). The vaccine (3.4 x 10⁷ PFU in a 0.5 ml of PBS) was injected subcutaneously into the right lateral upper arm with a 26G 3/8 inch needle. After 42 days, the monkeys received a identical boost vaccination on the left arm.

For ANDV, plasmid DNA was precipitated onto gold beads (3 µg of DNA per mg gold) and then the DNA-coated gold beads were coated on tubing. Gene gun cartridges consisting of ∼0.75 µg of plasmid DNA coated on 0.5 mg of gold were prepared and stored at 4(C, desiccated, until use. Syrian hamsters were vaccinated with the Powderject XR1 particle-mediated epidermal delivery device (gene gun) (Powderject Vaccines, Inc., Madison, Wis.), four administrations per vaccination, at non-overlapping sites on the shaved abdominal epidermis using 400 p.s.i. of helium pressure. Rhesus macaques were vaccinated with the similar cartridges and the same gene gun conditions used to vaccinate the hamsters; however, the monkeys received eight administrations (four on abdomen and four over inguinal lymph nodes) per vaccination. Hamsters and monkeys were anesthetized during the non-painful gene gun procedure. The only visible effect was mild erythema at the sites of vaccination.

**Vaccination by intramuscular needle inoculation.** Plasmid DNA (25 µg) suspended in 50 µl of PBS (pH 7.4) was injected into the gastrocnemius muscle of anesthetized mice with a 28.5-gauge needle.

**ELISA**. To detect SEOV G1 and G2, ELISA plates (Costar) were coated with SEOV-infected Vero E6 cell lysates [⁻Co-irradiated (3 million rads) to inactivate virus] diluted in carbonate buffer (pH. 9.6) overnight at 4°C. Plates were blocked with PBS, 3% skim milk, 0.05% Tween-20 (blocking solution) for 1 hr at 37°C and then washed once with PBS, 0.05% Tween-20 (wash solution). Mouse or hamster sera diluted in blocking solution were added to the wells, and plates were incubated as before. Plates were washed four times with wash solution and then incubated for 1 hr with horseradish peroxidase (HRP)-conjugated goat antimouse IgG (Cat. no. A-4416, Sigma) at a dilution of 1/1,000 or peroxidase-labeled goat anti-hamster antibody (Cat. no. 14-22-06, Kirkegaard & Perry Laboratories) diluted 1/2,000 in blocking solution. Plates were washed as before and then incubated for 10 min at room temperature with tetra-methylbenzidine substrate (TMB, Cat. no. 50-76-04, Kirkegaard & Perry Laboratories). The colorimetric reaction was stopped by adding Stop solution (Cat. no. 50-85-04, Kirkegaard & Perry Laboratories) and the optical density (O.D.), 450 nm, was determined. The method for detecting SEOV N-specific antibodies was adapted from a previously described technique (Elgh et al., 1997, J. Clin. Microbiol. 35, 1122-1130). Amino acids 1-117 of the nucleocapsid protein of SEOV (strain SR-11) were expressed as a histidine-tagged fusion protein by using the pRSET plasmid (Invitrogen) in *Escherichia coli* BL21 (DE3) (Novagen, Inc.) and purified by affinity chromatography on Ni-NTA columns (Qiagen). Antigen was diluted in carbonate buffer (pH 9.6), added to 96-well microtiter plates (Maxisorp; NUNC) (0.2 µg in 100 µl per well), and incubated overnight at 4°C. Plates were washed in deionized water and incubated with blocking solution for 30 min at room temperature. After plates were washed in deionized water, 100 µl of serum (diluted in blocking buffer containing *E*. *coli* antigen extract [0.6 mg/ml]) was added to duplicate wells. Plates were incubated at 37°C for 1 h and again washed as before. Addition of HRP secondary antibody and colorimetric detection were added as described above for the SEOV G1-G2 ELISA. End-point titers were determined as the highest dilution with an O.D. greater than the mean O.D. value of serum samples from negative control plasmid (pWRG7077)-vaccinated animals plus three standard deviations. The SEOV N antigen was used to detect HTNV N-specific antibodies and the PUUV N was used to detect ANDV N-specific antibodies.

**Plaque reduction neutralization tests (PRNT).** Neutralization assays were performed essentially as previously described (Chu et al., 1995, J. Virol. 69, 6417-6423). Serum samples were diluted in cEMEM and then combined with an equal volume (111 µl) of cEMEM containing -75 PFU of SEOV. This serum-virus mixture was incubated overnight at 4°C and then 200 µl/well was added to 6well plates containing Vero E6 monolayers 3-7 days old. After a 1-hr adsorption at 37°C, 3 ml of overlay medium (Earl's basal minimal essential medium (EBME), 10 mM HEPEs, 0.6% agarose [Sea Kem ME agarose], 8 mM L-glutamine, antibiotics) containing 10% FBS and 1x nonessential amino acid mixture (GIBCO BRL) was added to each well. Plates were incubated at 37°C for 7 days and then stained by adding 2 ml/well of overlay medium containing 5% FBS and 5% neutral red solution (GIBCO BRL). Plaques were counted after 2 days at 37°C. Serum samples were heat inactivated (56°C, 30 min) before assay. In experiments where complement was used, serum and virus were incubated overnight in the presence of 5% guinea pig complement (Cat. no. ACL-4051, Accurate Chemical and Scientific Corporation). HTNV,SEOV, ANDV, and BCCV PRNT were stained with neutral red after 1 week and PUUV, DOBV, and SNV PRNT were stained after 9 days. Plaques were counted 2-3 days (37°C) after staining.

**Challenge with SEOV.** Syrian hamsters were injected intramuscularly (caudal thigh) with a 25-gauge needle with 10³ PFU of SEOV diluted in 0.2 ml sterile PBS (pH 7.4). The 10³ PFU dose was based on previously pulished work that reported 100% infection of hamsters after this challenge dose and route (Schmaljohn *et al.,* 1990, supra; Chu *et al.,* 1995, supra). Twenty-eight days after challenge, the hamsters were anesthetized and exsanguinated by cardiac puncture. Postchallenge serum was evaluated for the presence of anti-SEOV antibody by IFAT, ELISA, and PRNT. A dramatic rise in postchallenge antibody titer to viral proteins other than the immunogens indicated that the hamster was infected with SEOV.

For HTNV and ANDV, pre- and postchallenge serum was evaluated for the presence of N-specific antibody by ELISA, and neutralizing antibody by PRNT. Detecting postchallenge N-specific antibody indicated that the hamster was infected with the challenge virus.

The challenge dose for HTNV was 2,000 PFU, which is -1000 LD₅₀ (unpublished (data). The challenge dose for ANDV was 2,000 PFU, which is 250 LD₅₀. Work involving ANDV-infected hamsters was performed in a biosafety level 4 (BSL-4) laboratory. Work involving HTNV-infected hamsters was performed while wearing a 3M-RACAL hood and TYVEK suit in a biosafety level 3 (BSL-3) laboratory. The effect of vaccine on survival outcome was assessed using a logistic regression model. Analysis was conducted using SAS Version 8.2 (SAS Institute Inc., SAS OnlineDoc, Version 8, Cary, NC 2000).

All animal research was conducted in accordance with procedures described in the Guide for the Care and Use of Laboratory Animals (National Institutes of Health, Bethesda, MD, 1996). The facilities are fully accredited by the American Association of Acccreditation of Laboratory Animal Care.

**Cross protection assay.** Groups of 4-5 hamsters were vaccinated with either pWRG/SEO M or negative control plasmid (pWRG7077). Vaccinations consisted of four gene gun administrations (-1.5 ug DNA per administration) three times at three week intervals. Three weeks after the final vaccination, prechallenge serum samples were obtained and the hamsters were challenged with 1,000 PFU of Hantaan virus, 1,000 PFU of Dobrava virus, or 1,000-2,000 PFU of Puumala virus. Twenty-eight days after challenge postchallenge serum samples were obtained. Pre and postchallenge serum samples were evaluated by anti-N ELISA to detect antibody to nucleocapsid, by a Hantaan, Dobrava, or Puumula plaque reduction neutralization test (PRNT) to detect the resective neutralizing antibodies depending on the challenge virus.

**Antibody injections.** Lyophilized human convalescent plasma from a human HPS patient was resuspended in water and recalcified before use by adding CaCl₂ to 0.1 M, incubation at 37°C for 4 h, at 4°C overnight, and at -20°C for 1 h. The treated plasma was thawed, centrifuged at 10,000 x G for 20 min and the supernatant was collected. Monkey serum from DNA-vaccinated monkeys and human serum (PEL-FREEZ Biologics, Rogers, Ariz.) were heat inactivated (56°C 30 min). One ml of serum or plasma was injected intraperitoneally (i.P.) into hamsters using a 1-ml syringe with a 25-ga, 5/8-inch needle.

### EXAMPLE 1

### Transient expression of hantavirus genes.

The cDNA representing the M (SEQ ID NO:1) or S (SEQ ID NO:2) gene segment of the SR-11 strain of SEOV was subcloned into the naked DNA expression vector pWRG7077 downstream of the cytomegalovirus immediate early promoter to yield pWRG/SEO-M (SEQ ID NO:3) and pWRG/SEO-S (SEQ ID NO:4), respectively (Fig. 1). To assess expression by the M and S constructs, these plasmids were transfected into COS cells and the expressed proteins were either immunostained or immunoprecipitated. The presence of stained cells in transfected monolayers incubated with anti-SEOV polyclonal sera indicated that SEOV reactive protein(s) were expressed from both pWRG/SEO-M and pWRG/SEO-S (Fig. 2A). Radiolabeled expression products were immunoprecipitated with anti-SEOV polyclonal antisera and analyzed by SDS-PAGE (Fig. 2B). Immunoprecipitated proteins had apparent molecular sizes expected for G1, G2, and N (80 kDa, 56 kDa, and 48 kDa) (Elliot et al. 1984, J. Gen. Virol. 65, 1285-1293; Schmaljohn et al. 1986, Virology 155, 633-643). Additional protein bands with apparent molecular masses of -37 kDa and -28 kDa were coprecipitated with N. These polypeptides were immunoprecipitated by a N amino-terminus-specific monoclonal antibody, and could be detected by Western blot, suggesting they represent truncated forms of N (data not shown).

### EXAMPLE 2

**Vaccination of mice with pWRG/SEO-M or pWRG/SEO-S.** To determine if either the M or S construct could elicit an antibody response in mice, groups of 10 6- to 8-week-old female BALB/c mice were vaccinated with pWRG/SEO-M, pWRG/SEO-S, or negative control DNA (pWRG7077) by two different routes: inoculation of the epidermis by gene gun, or inoculation of the gastrocnemius muscle by needle injection. Mice received a priming vaccination followed by two boosts at 4 week intervals. Blood samples collected before each vaccination and 3 weeks after the final boost were screened for a SEOV-specific antibody response by ELISA. Both the M and S constructs elicited a SEOV-specific antibody response by both routes of DNA administration (Fig. 3). Most mice vaccinated with SEOV S by gene gun exhibited specific antibodies after only one vaccination, and the antibody responses increased after subsequent vaccinations (Fig. 3B). To compare the relative antibody responses, end-point ELISA titers were determined for the final blood samples (Table 1). Gene gun vaccination resulted in higher seroconversion frequencies and geometric mean titers (GMT) than intramuscular vaccination with either construct.

**Table 1: ELISA Titers in Mice Vaccinated with SEOV Naked DNA Vaccines**

| Inoculated DNA | Vaccine route^{a} | Positive/ total | End point titer range | GMT^{b} |
|---|---|---|---|---|
| pWRG/SEO-M | g.g. | 9/10 | 200-1600 | 303 |
| pWRG/SEO-M | i.m. | 7/10 | 100-400 | 132 |
| pWRG/SEO-S | g.g. | 10/10 | 6400-12800 | 7352 |
| pWRG/SEO-S | i.m. | 9/10 | 800-6400 | 1213 |

| | | | | |
|---|---|---|---|---|
| Note. Mice were vaccinated three times at 4-week intervals. Titers were determined for sera collected three weeks after the final boost. ^{a}g.g. indicates gene gun; i.m., intramuscular needle inoculation. ^{b}Geometric mean titer of all animals in group. | | | | |

To determine if naked DNA vaccination elicited neutralizing antibodies, we performed plaque reduction neutralization tests (PRNT) on sera collected 3 weeks after the final boost. All (10/10) mice that were vaccinated with pWRG/SEO-M by gene gun exhibited PRNT50% titers ranging from 40 to 1280 (Fig. 4). Only 1/10 mice vaccinated with pWRG/SEO-M by needle injection exhibited a detectable neutralizing antibody response (PRNT50% = 40). Mice vaccinated with pWRG/SEO-S or pWRG7077 by either gene gun or needle inoculation did not develop neutralizing antibodies (Fig. 4).

### EXAMPLE 3

**Vaccination and challenge of hamsters.** To study the protective efficacy of the naked DNA hantavirus vaccines, groups of five hamsters were vaccinated with either pWRG/SEO-M, pWRG/SEO-S, or negative control plasmid pWRG7077 by gene gun (a priming vaccination followed by two boosts at 4-week intervals) and then challenged with SEOV 6 weeks after the final boost. IFAT, ELISA, and PRNT were performed on serum samples taken before vaccination, on the day of challenge, and 4 weeks after challenge. Because there are no disease models for hantavirus in adult rodents, we evaluated whether or not our DNA vaccines could protect against infection rather than prevent disease.

IFAT and ELISA results demonstrated that before vaccination, none of the hamsters was positive for either SEOV G1-G2 or N protein (data not shown). After vaccination (prechallenge sera drawn on day of challenge), five of five hamsters vaccinated with pWR.G/SEO-M developed anti-G1-G2 antibodies, and four of five hamsters vaccinated with the pWRG/SEO-S exhibited anti-N antibodies by IFAT (Table 2). The prechallenge ELISA results detected similar antibody responses except that only four of five pWRG/SEO-M vaccinated hamsters were positive in the anti-SEOV ELISA. The pWRG7077 vaccinated hamsters failed to develop both G1-G2- and N-specific antibodies by IFAT and ELISA before challenge.

After challenge, serum samples from hamsters previously vaccinated with pWRG/SEO-M remained negative for anti-nucleocapsid antibody by IFAT indicating the animals were not infected with SEOV. In contrast, postchallenge serum samples from pWRG/SEO-S and pWRG7077 vaccinated hamsters were positive for both G1-G2 and N antibodies, indicating that the animals were infected when challenged with SEOV. Postchallenge ELISA results were similar to the IFAT results; however, three of five of the pWRG/SEO-M vaccinated hamsters exhibited a weak anti-N response after challenge indicating that, although the IFAT were negative, the hamsters had developed antibodies to the challenge virus.

Before vaccination, none of the hamsters had SEOV neutralizing antibody titers (data not shown). After vaccination, all hamsters vaccinated with pWRG/SEO-M developed neutralizing antibodies with PRNT80% titers ranging from 160 to 640 (Table 2). Neutralizing antibody was not detected in pWRG/SEO-S and pWRG7077 prechallenge sera from vaccinated hamsters. After SEOV challenge, neutralizing antibody titers of the pWRG/SEO-M immunized animals remained essentially the same as before challenge (plus or minus twofold). In contrast, the pWRG7077 and pWRG/SEO-S postchallenge neutralizing antibody titers increased significantly from <20 to between 1,280 and >81,920.

**Table 2. Serological responses and protection of hamsters vaccinated with naked DNA vaccines.**

| | | IFAT_{b} | | | | | | |
|---|---|---|---|---|---|---|---|---|
| | | anti-N | | anti-G1-G2 | | PRNT_{50%C} | Protection | |
| Vaccineₐ | Hamster | pre | post | pre | post | pre | post | |
| pWRG/SEO-M | 1 | - | - | + | + | 320(20) | 640 | yes |
| | 2 | - | - | + | + | 320(40) | 640 | yes |
| | 3 | - | - | + | + | 320(20) | 1280 | yes |
| | 4 | - | - | + | + | 80(<20) | 160 | yes |
| | 5 | - | - | + | + | 640(20) | 1280 | yes |
| | | | | | | | | |
| PWRG/SEO-S | 1 | + | + | - | + | <20(<20) | >640 | no |
| | 2 | + | + | - | + | <20(<20) | >640 | no |
| | 3 | + | + | - | + | <20(<20) | >640 | no |
| | 4 | + | + | - | + | <20(<20) | >640 | no |
| | 5 | - | + | - | + | <20(<20) | >640 | no |
| | | | | | | | | |
| PWRG7077 | 1 | - | + | - | + | <20(<20) | >640 | no |
| | 2 | - | + | - | + | <20(<20) | >640 | no |
| | 3 | - | + | - | + | <20(<20) | >640 | no |
| | 4 | - | + | - | + | <20(<20) | >640 | no |
| | 5 | - | + | - | + | <20(<20) | >640 | no |

| | | | | | | | | |
|---|---|---|---|---|---|---|---|---|
| ^{a} Three vaccinations consisting of 4 gene gun cartridges (about 1.5 ug/cartridge) administered at 4 week intervals. ^{b} Sera were screened at 1:200 in IFAT sing pWRG/SEO-S or pWRG/SEO-M trasnfected cells as target antigen for anti-n and anti-G1-G2, respectively. - indicates no reactivity; + indicates reactivity. c PRNT values are reciprocal serum dilutions reducing plaque number by 50%. Assays were performed in the presence of 5% guinea pig complement. Numbers in parenthesis are PRNT values of heated serum in the absence of complement. pre, prechallenge serum samples from day of challenge; post, poschallenge serum samples from 4 weeks post challenge. | | | | | | | | |

During the course of this study, we observed that PRNT titers decreased dramatically if sera were heat-inactivated (56°C, 30 min). To test the possibility that the neutralizing activity was complement-enhanced, we performed PRNT assays in the presence of 5% guinea pig complement and found that adding complement increased the PRNT titers of heated serum 4- to 32-fold (Table 2). Complement alone had no effect on SEOV plaque number (data not shown).

### Example 4

### Vaccination with pWRG/SEO-M cross-protects against infection with Hantaan virus

Hamsters vaccinated with pWRG/SEO-M had no detectable anti-N antibody prior to challenge, and the postchallenge anti-N response was undetectable or very low (<= 1:200) (Fig. 6). In contrast, hamsters vaccinated with the negative control plasmid exhibited a drammatic rise in anti-N antibody (-2 log increase over background). The PRNT indicated that vaccination with pWRG/SEO-M elicited detectable levels of cross neutralizing antibodies in 3 of 4 hamsters. The PRNT titers of these hamsters did not rise after challenge. In the one hamster with undetectable (<1:20) Hantaan neutralizing antibody, the PRNT titer did rise; however, the increase was at least 32-fold lower than that seen in the hamsters vaccinated with the negative control plasmid.

The low or absent increase in antibody titers to Hantaan structural proteins (as measured by ELISA and PRNT) after challenge with Hantaan virus indicates that vaccination with the Seoul virus M gene (pWRG/SEO-M) confers protection against infection with Hantaan virus.

### Example 5

### Vaccination with pWRG/SEO-M cross-protects against infection with Dobrava virus.

Hamsters vaccinated with pWRG/SEO-M had no detectable anti-N antibody prior to challenge, and the postchallenge anti-N response was undetectable. In contrast, all but one of the hamsters vaccinated with the negative control plasmid exhibited a drammatic rise in anti-N antibody (postchallenge anti-N titers of between 1:200 and 1:204,800). The PRNT indicated that vaccination with pWRG/SEO-M elicited low levels of cross neutralizing antibodies in 5 of 5 hamsters. The PRNT titers of these hamsters did not rise above 1:80 after challenge. In contrast, all but one hamster vaccinated with the negative control plasmid exhibited a high postchallenge PRNT (1:5120) indicating the hamsters were infected with Dobrava virus. It remains unclear why animal #182 was not infected.

The low or absent increase in antibody titers to Dobrava structural proteins (as measured by ELISA and PRNT) after challenge with Dobrava virus indicates that vaccination with the Seoul virus M gene (pWRG/SEO-M) confers protection against infection with Dobrava virus.

### Example 6

### Vaccination with pWRG/SEO-M fails to cross-protect against infection with Puumala virus.

Hamsters vaccinated with pWRG/SEO-M had no detectable anti-N antibody prior to challenge. After challenge, all but one hamster had postchallenge anti-N response greater than or equal to 1:200 indicating the hamsters were infected with Puumala virus. The negative control hamsters had similar pre and postchallenge anti-N titers. The PRNT resulsts indicated that, although vaccination with pWRG/SEO-M elicited neutralizing antibodies against Seoul virus (empty circles), these antibodies did not cross-neutralizing Puumala virus, and failed to protect against infection. The postchallenge Puumala virus PRNT titers of the pWRG/SEO-M vaccinated and negative control vaccinated hamsters were all similar (greater than or equal to 1:160), exept one negative control hamster that was not infected. It remains unclear why negative control animal #220 was not infected.

The increase in antibody titers to Puumala virus structural proteins (as measured by ELISA and PRNT) after challenge with Puumala virus indicates that vaccination with the Seoul virus M gene (pWRG/SEO-M) fails to confers protection against infection with Puumala virus. It should be noted that the hamster with the highest prechallenge anti-Seoul virus PRNT titer was 1:640 (animal #209). It is possible that animals with higher prechallenge neutralizing antibody titers might be cross-protected against Puumala virus.

### DISCUSSION

We report that naked DNA shows promise as a way to vaccinate against hantaviruses. Our results demonstrate that vaccination with cDNA representing either the SEOV M or S genome segment elicited antibody responses in BALB/c mice and Syrian hamsters. Hamsters vaccinated with DNA expressing the M segment, but not S segment, developed neutralizing antibodies and were protected against infection with SEOV.

In our initial immunogenicity experiments, gene gun vaccination of BALB/c mice resulted in higher seroconversion rates than intramuscular needle inoculation for both pWRG/SEO-M and pWRG/SEO-S, as measured by ELISA and PRNT. These preliminary data prompted us to focus on the gene gun rather than the intramuscular route of DNA delivery. However, because only one intramuscular DNA dose was tested (25 µg per animal), it is possible that the seroconversion rate and/or titers could be increased by adjusting parameters such as the amount of DNA injected, site of injection, or vaccination schedule. It is also possible that needle inoculation elicited a predominantly cell-mediated rather than humoral response to the DNA vaccination, as has been seen by others (Feltquate et al., 1997, J. Immunol. 158, 2278-2284; Robinson and Torres, 1997, Semin. Immunol. 9, 271-283; Gregoriadis, 1998, Pharm. Res. 15, 661-670).

Gene gun vaccination with pWRG/SEO-M protected all hamsters against infection with SEOV as measured by 1) undetectable, or barely detectable, postchallenge anti-N antibody response and, 2) PRNT titers that remained essentially unchanged after SEOV challenge. Although the IFAT results indicated that none of the pWRG/SEO-M vaccinated hamsters developed anti-N antibodies after challenge, the more sensitive anti-N ELISA revealed that three hamsters had a weak anti-N antibody response. Because the postchallenge anti-N ELISA GMT of these hamsters (GMT = 50) was more than 50 times lower than the negative control pWRG7077 vaccinated hamsters (GMT > 3,195), it is likely the anti-N antibodies represent an antibody response to either the input SEOV antigen (no replication), or to a limited infection (e.g., one round of replication). The PRNT data provided additional evidence that vaccination with pWRG/SEO-M, but not pWRG/SEO-S, protected hamsters from SEOV infection. Whereas the negative control pWRG7077-vaccinated hamsters' PRNT titers increased from <20 to between 10,280 and 81,920 after challenge, the pWRG/SEO-M-vaccinated hamster PRNT titers remained within twofold of prechallenge levels. Moreover, the postchallenge PRNT titers of the pWRG/SEO-M-vaccinated hamsters (GMT = 368) remained well below the postchallenge PRNT titers of the pWRG7077-vaccinated hamsters (GMT > 27,024). Hamsters vaccinated with pWRG/SEO-S were infected with SEOV after challenge. The pWRG/SEO-S-vaccinated hamsters' postchallenge PRNT titers increased from <20 to between 2,560 and 20,480. Although the postchallenge PRNT titers of the pWRG/SEO-S were high (GMT = 5,885), it is noteworthy that these titers were approximately fivefold lower than the negative control (pWRG7077) postchallenge titers (GMT > 27,024).

The absence of a neutralizing antibody response in mice and hamsters vaccinated with pWRG/SEO-S, with or without complement, is consistent with published data that monoclonal antibodies to G1 and G2, but not N, have neutralizing activity (Dantas et al., 1986, Virology 151, 379-384; Arikawa et al., 1989, J. Gen. Virol. 70, 615-624; Schmaljohn et al., 1990, J. Virol. 64, 3162-3170; Arikawa et al., 1992, Arch. Virol. 70, 615-624), and that vaccination with vaccinia recombinants expressing G1 and/or G2, but not N, elicit a neutralizing response (Pensiero et al., 1988, J. Virol. 62, 696-702; Schmaljohn et al. 1992, Vaccine 10, 10-13; Xu et al., 1992, Am. J. Trop. Med. Hyg. 47, 397-404; Chu *et al.,* 1995, supra).

Failure of an anti-N immune response to protect against infection was unexpected because others found that vaccination with recombinant N expressed in insect cells, bacteria, or as chimeric HBV core particles, protect against infection (Schmaljohn *et al*., 1990, supra; Yoshimatsu et al., 1993, Arch. Virol. 130, 365-376; Lundkvist et al., 1996, Virology 216, 397-406; Ulrich et al., 1998, Vaccine 16, 272-280). Also, vaccination with recombinant vaccinia viruses expressing the SEOV S segment partially protected gerbils from SEOV infection (Xu *et al*. 1992, supra). Failure of naked DNA vaccination with the S. segment to protect hamsters in our experiments may be related to a quantitative rather than qualitative deficiency. Although four of five hamsters vaccinated with pWRG/SEO-S developed a detectable antibody response by IFAT and ELISA, the titers were relatively low (ELISA titers less than or equal to 1:1,600). The reason for these low anti-N antibody titers in hamsters, but not mice, remains unclear. Fault cannot lie with the gene gun cartridges as the same lot was used to vaccinate both hamsters and mice on the same day; and the mouse seroconversion rate was 90%, with an ELISA GMT of over 7,000. Low antibody titers may reflect a poor overall immune response to vaccination with pWRG/SEO-S in hamsters or, alternatively, it is possible that epidermal administration of S DNA with the gene gun results in a predominantly cell-mediated, rather than humoral, response to N. Nevertheless, our findings demonstrate that there was a detectable anti-N immune response in four of five hamsters after naked DNA gene gun vaccination with pWRG/SEO-S, and that this response, whether humoral and/or cell-mediated, was insufficient to protect against SEOV infection. The -fivefold difference in postchallenge PRNT titers of pWRG/SEO-S versus pWRG7077 vaccinated hamsters suggests pWRG/SEO-S vaccination may nonetheless limit SEOV infection.

We found that SEOV neutralizing activity elicited in mice and hamsters was reduced 4- to 32-fold if the sera was heat inactivated (56°C, 30 min). This loss in neutralizing activity could be completely reversed if guinea pig complement was included in the PRNT. These data are consistent with those of others who reported that hantavirus neutralizing activity in sera from humans, mice, and especially rats, was enhanced by complement (Takenaka et al., 1985, Arch. Virol. 84, 197-206; Asada et al., 1989, J. Gen. Virol. 70, 819-825). The observation that complement plays a major role in hantavirus neutralization is notable because PRNT titers are commonly used to assess the immunogenicity and predicted efficacy of hantavirus vaccines. Conditions that deplete or add complement, such as heat inactivation or addition of exogenous complement (e.g., as a purified protein fraction or as fresh animal sera), could alter PRNT titers and, therefore, could alter the assessment of candidate vaccines.

### Example 7

**Expression of G1 and G2 from HTNV M DNA vaccine.** cDNA representing the HTNV M genome segment was cloned into a cytomegalovirus (CMV) promoter-based expression plasmid, pWRG7077, to create pWRG/HTN-M. Radio-immunoprecipitation assay (RIPA) experiments using polyclonal and monoclonal antibodies (MAbs) indicated that both the G1 and G2 proteins were transiently expressed in cells transfected with pWRG/HTN-M (Fig. 8).

Earlier attempts to develop a functional HTNV M DNA vaccine were unsuccessful (*i.e*., G2 but not G1 was expressed) (unpublished data). We solved this problem after discovering that our SEOV M plasmid (pWRG/SEO-M) failed to efficiently express G1 if a 24 base pair (bp) sequence of extraneous DNA, found between the vector *NotI* cloning site and the SEOV M noncoding region (Fig. 9A), was removed. This extraneous DNA originated from procedures used during earlier cloning and subcloning of the SEOV M gene. The SEOV M construct with the 24 bp sequence removed failed to express G1, but did express G2 (Fig. 9B). Expression of G1 was restored if the 24 bp sequence was engineered back into the construct in the forward but not reverse orientation (Fig. 9B). This result indicated that the nucleotide sequence, not nucleotide number, affected G1 expression. To map the sequence that allowed efficient G1 expression, we made constructs which included either nucleotides 1-12 or 13-24 of the extraneous sequence. G1 was expressed when nucleotides 1-12 but not 13-24 was present in the plasmid (Fig. 9B). We noted that nucleotides 4-10 of the DNA (TCTGCAG) were identical to the last seven nucleotides of intron A (*i.e*., splice acceptor site)(Stenberg *et al*., 1984, supra). Mutating the putative splice acceptor dinucleotide AG to CC did not influence the expression of G1, suggesting that, if this region is involved in splicing, it is not dependent on maintaining the exact sequence of the intron A splice site (Fig. 9B). We made a construct containing nucleotides 1-8 of the extraneous DNA and found this was sufficient to restore efficient expression of G1 (Fig. 9C). Thus, we determined that the sequence GGATCTGC, located between the *Not*I cloning site and the 5' noncoding region of the SEOV M gene, was required for efficient expression of G1 from pWRG7077-based DNA vaccines.

Based on the SEOV M findings, we postulated that including the extraneous sequence upstream of the HTNV M gene might allow us to solve our inability to express both HTNV G1 and G2 from a DNA vaccine plasmid. We constructed a plasmid that included the extraneous sequence upstream of the HTNV M gene and, in doing so, obtained a clone that expressed both G1 and G2, pWRG/HTN-M (Fig. 9D). When the extraneous sequence was removed, G1 was not expressed, and when it was restored, G1 was efficiently expressed (Fig. 9D). These data confirmed that the extraneous sequence was not only required for successful expression of SEOV G1, but also of HTNV G1. This sequence had no influence on expression of G2 from either SEOV or HTNV (Fig.9). The mechanism by which the extraneous sequence affects G1 expression but not G2 expression remains unknown.

### Example 8

### DNA vaccination with pWRG/HTN-M elicits neutralizing antibody and protects hamsters against infection with HTNV

To determine if the HTNV M DNA vaccine plasmid was immunogenic, we used a gene gun to vaccinate hamsters with either pWRG/HTN-M (pWRG/HTN-M or pWRG/HTN-M(x), see Methods), or a negative control. Three weeks after the final vaccination, the hamsters were bled and sera were evaluated for neutralizing antibody by a plaque-reduction neutralization test (PRNT). In two separate experiments, all of the hamsters vaccinated with pWRG/HTN-M developed HTNV-neutralizing antibody responses (Fig. 11). Titers (PRNT_{80%}) ranged from 20-1280 with a geometric mean titer (GMT) = 104 in the first experiment and 20-10240, GMT = 493, in the second experiment. Negative control groups remained seronegative. Thus, gene gun vaccination with pWRG/HTN-M was immunogenic in hamsters.

To determine the protective efficacy of pWRG/HTN-M we used an infection model described previously (Hooper *et al*., 1999, supra). The model involves challenging vaccinated hamsters with virus, and after 4 weeks, using serological assays to detect evidence of infection. Specifically, if a challenged hamster developed antibodies to hantavirus N protein (which is not a component of the vaccine), then that hamster was considered to be infected. On the other hand, if a challenged hamster failed to develop a N-specific antibody response, then that hamster was not infected (*i.e*, protected against infection). A greater than fourfold increase in the neutralizing antibody response after challenge also served as a marker for evidence of infection.

Vaccinated hamsters were challenged with 2,000 plaque-forming units (ID₅₀ is approximately 2 plaque-forming units [PFU], unpublished data) of HTNV intramuscularly (i.m.). Four weeks after challenge, blood samples were collected and sera were tested for N-specific antibody by ELISA, and for neutralizing antibody by PRNT. The pre- and postchallenge anti-N titers and PRNT titers are shown in (Fig. 11). All of the hamsters that were vaccinated with pWRG/HTN-M were protected against infection as defined by an absence of a postchallenge N-specific antibody response. In addition, the pre- and postchallenge PRNT titers differed ≤ fourfold. In contrast, all of the negative control hamsters, whether they were vaccinated with pWRG7077 or remained unvaccinated, were infected as evidenced by the development of N-specific antibodies and neutralizing antibodies postchallenge. Thus, gene gun vaccination with pWRG/HTN-M (or pWRG/HTN-M(x)) protected against productive infection with HTNV, even when the prechallenge PRNT_{80%} titer was as low as 20.

### Example 9

### DNA vaccination with either pWRG/SEO-M or pWRG/HTN-M cross-protects against challenge with heterotypic hantaviruses

Having determined that our SEOV M and HTNV M gene-based DNA vaccines were capable of protecting hamsters against infection with homotypic virus, we wanted to determine if either of these vaccines could cross-protect against other HFRS-associated hantaviruses. We first measured the cross-neutralizing activities of sera from HFRS hantavirus-infected hamsters, and hamsters vaccinated with either the SEOV M or HTNV M vaccine (Table 3). We found that sera from SEOV-infected hamsters had a low level of HTNV neutralizing activity, and no detectable DOBV or PUUV neutralizing activity. Sera from HTNV- or DOBV-infected hamsters exhibited a low level of neutralizing antibody against SEOV, DOBV, and PUUV. Sera from PUUV-infected hamsters failed to neutralize HTNV or SEOV, and had a barely detectable DOBV-neutralizing activity. The sera from vaccinated hamsters exhibited greater levels of cross-neutralizing activity than sera from the infected hamsters. Vaccination with either pWRG/SEO-M or pWRG/HTN-M elicited an antibody response that cross-neutralized SEOV, HTNV, and DOBV, but not PUUV.

**Table 3. Cross-neutralization activity in serum from infected, or DNA-vaccinated hamsters**

| | PRNT | | | | | | | |
|---|---|---|---|---|---|---|---|---|
| | | SEOV | | HTNV | | DOBV | | PUUV |
| Serum source^{a} | 80% | 50% | 80% | 50% | 80% | 50% | 80% | 50% |
| Infected^{b} with SEOV | **640** | **5120** | * | 80 | * | * | * | * |
| Infected with HTNV | * | 160 | **1280** | **5120** | 40 | 320 | * | 20 |
| Infected with DOBV | 20 | 320 | 20 | 160 | **2560** | **10240** | * | 40 |
| Infected with PUUV | * | * | * | * | * | 20 | **5120** | **20480** |
| Vaccinated^{c} with pWRG/SEO-M | **5120** | **10240** | 320 | 320 | 160 | 320 | * | * |
| Vaccinated with pWRG/HTN-M | 40 | 80 | **5120** | **5120** | 160 | 640 | * | * |

| | | | | | | | | |
|---|---|---|---|---|---|---|---|---|
| ^{a} Pool of serum from three to four hamsters with homotypic titer of at least 160. Serum was collected 4 weeks after infection, or 3 weeks after the final DNA vaccination. ^{b} Hamsters injected i.m. with 2,000 PFU of the indicated virus. ^{c} Hamsters gene gun-vaccinated three times at 3 week intervals with the indicated DNA vaccine plasmid. ^{d} PRNT values are the reciprocal serum dilution that neutralizes the indicated virus plaque number by 80% or 50%. Homotypic titer are shown in bold. *, titer less than 20 | | | | | | | | |

We previously demonstrated that the SEOV M DNA vaccine protected most hamsters not only from SEOV but also from challenge with HTNV (Kamrud et al., 1999, Virology 263, 209-219). To confirm those findings, and to further evaluate the capacity of the SEOV M vaccine to cross-protect against other hantaviruses, we tested pWRG/SEO-M for protective efficacy against HTNV, DOBV, and PUUV. The results of this study indicated that hamsters vaccinated with pWRG/SEO-M were protected against HTNV and DOBV but not PUUV as determined by the absence of anti-N antibody response after challenge (Fig. 12). A homotypic PRNT_{80%} titer >160 protected against HTNV, and a titer (80 protected against DOBV, but a titer as high as 1280 failed to protect against PUUV (Fig. 12A, 12B and 12C). One vaccinated and one control hamster failed to respond to the PUUV challenge, probably because the PUUV challenge dose was 10 ID₅₀, whereas it was 1000 ID₅₀ for the other viruses (unpublished data).

We tested the capacity of pWRG/HTN-M to cross-protect against SEOV or DOBV. The results indicated that vaccination with pWRG/HTN-M elicited cross-protective immunity against both SEOV and DOBV (Fig. 12D and 12E). A homotypic PRNT_{80%} titer (640 was associated with protection of hamsters against SEOV, and a titer (320 protected against DOBV. We did not measure the capacity of vaccination with pWRG/HTN-M to protect against PUUV infection because of our findings that the SEOV DNA vaccine did not protect (Fig. 12C), and because a vaccinia-vectored HTNV vaccine did not protect hamsters against PUUV infection (Schmaljohn et al., 1995, Virology 206, 963-972). Together, these data indicated that DNA vaccination with either pWRG/SEO-M or pWRG/HTN-M cross-protected against three of the four HFRS-associated hantaviruses: SEOV, HTNV, and DOBV.

### Example 10

### DNA vaccination with pWRG/SEO-M or pWRG/HTN-M elicits a high-titer neutralizing antibody response in nonhuman primates

Our vaccination data with plasmids expressing the SEOV M gene (Hooper *et al*., 1999, supra) or HTNV M gene suggests that these vaccines might be efficacious in humans. As a further step toward clinical development of these vaccines, we tested their capacity to elicit antibody responses in nonhuman primates. Two rhesus macaques were vaccinated with the SEOV M DNA vaccine, and three rhesus macaques were vaccinated with the HTNV M DNA vaccine. As negative controls, three monkeys were vaccinated with pWRG7077 expressing irrelevant genes; and, as positive controls, three monkeys were vaccinated with a recombinant vaccinia virus expressing the HTNV M and S genes, rVV/HTN-M+S (Schmaljohn et al., 1992, Vaccine 10, 10-13). rVV/HTN-M+S was previously shown to elicit HTNV-specific immunity, including neutralizing antibodies, in hamsters (Chu et al., 1995, J. Virol. 69, 6417-6423) and to elicit neutralizing antibodies in humans (McClain et al., 2000, J. Med. Virol. 60, 77-85). The DNA vaccines were administered three times at three week intervals. The rW/HTN-M+S vaccine was administered by the dose and schedule used in the human phase II trials (*i.e*., a primary subcutaneous vaccination followed by a boost at day 42).

Three weeks after the first vaccination, two of the three monkeys vaccinated with pWRG/HTN-M(x) demonstrated neutralizing antibodies (Fig. 13). Three weeks after the second gene gun vaccination, all of the monkeys vaccinated with either pWRG/SEO-M(x) or pWRG/HTN-M(x) demonstrated detectable levels of neutralizing antibody. Three weeks after the third vaccination, the neutralizing antibody titers in all of the monkeys vaccinated with pWRG/SEO-M(x) or pWRG/HTN-M(x) were impressively high. Negative control-vaccinated monkeys never developed neutralizing antibodies (data not shown). Monkeys vaccinated with rW/HTN-M+S failed to develop a neutralizing antibody response after one vaccination, but did develop neutralizing antibodies after the 42-day boost. Three weeks after the final vaccination, the PRNT_{80%} GMT of the monkeys vaccinated with pWRG/SEO-M(x), pWRG/HTN M(x), or rVV/HTN-M+S, were 905, 2032, and 160, respectively. These data demonstrated, for the first time, that DNA vaccines expressing the hantavirus M gene products are immunogenic in nonhuman primates and elicit relatively high levels of neutralizing antibodies.

To evaluate the duration of immunity elicited by the DNA vaccine and the recombinant vaccinia virus vaccine, serum from vaccinated monkeys was collected 2, 4, and 6 months after the final vaccination, and tested for neutralizing activity. Two months after the final vaccination, the neutralizing antibody titers in sera from DNA-vaccinated hamsters dropped two- to fourfold (Fig. 14, Table 9 and 10). At 4 months postvaccination, monkeys vaccinated with pWRG/SEO-M(x) or pWRG/HTN-M(x) had PRNT_{80%} GMT = 160 and 80, respectively. After 6 months, all of the monkeys vaccinated with pWRG/SEO-M(x) or pWRG/HTN-M(x) still had detectable levels of neutralizing antibody, PRNT_{80%} GMT = 113 and 63, respectively. After 8 months one of the HTNV M DNA vaccinated monkeys (CH27) had a PRNT_{80%} titer <20, however there was still a detectable neutralizing antibody response, PRNT_{50%} = 20. In contrast, monkeys vaccinated with the positive control vaccine, rVV/HTN-M+S, exhibited little or no detectable neutralizing antibody 4 months after the final vaccination.

### Example 11

### DNA vaccination of nonhuman primates with pWRG/SEO-M or pWRG/HTN-M vaccines elicit antibody that cross-neutralizes DOBV

We tested the sera from the vaccinated monkeys for cross-neutralizing activity by PRNT (Table 4). All of the monkeys vaccinated with either pWRG/SEO-M(x) or pWRG/HTNV-M(x) had antibodies that cross-neutralized DOBV. rVV/HTN-M+S vaccinated monkeys also had DOBV-cross neutralizing antibodies, albeit at lower titer. Surprisingly, monkeys vaccinated with pWRG/SEO-M(x) had little or no HTNV-neutralizing antibody; and monkeys vaccinated with pWRG/HTN-M(x) had little or no SEOV-neutralizing antibody. Only one monkey (monkey CH32), which was DNA vaccinated with pWRG/SEO-M(x), demonstrated a detectable level of PUUV neutralizing antibody.

**Table 4. Cross-neutralization activity in serum from vaccinated monkeys**

| | | PRNT^{d} | | | | | | | |
|---|---|---|---|---|---|---|---|---|---|
| | | SEOV | | HTNV | | DOBV | | PUUV | |
| | Monkey^{e} | 80% | 50% | 80% | 50% | 80% | 50% | 80% | 50% |
| Serum source^{a} | | | | | | | | | |
| Vaccinated^{b} with pWRG/SEO-M(x) | CH03 | **640** | 1280 | * | * | 40 | 160 | * | * |
| | CH32 | **1280** | 1280 | * | 80 | 320 | 640 | * | 40 |
| Vaccinated^{b} with pWRG/HTN-M(x) | CH28 | * | * | **2560** | 2560 | * | 320 | * | * |
| | CH02 | * | * | **5120** | 10240 | 160 | 320 | * | * |
| | CH27 | * | 20 | **640** | 1280 | 80 | 320 | * | * |
| Vaccinated^{c} with rVV/HTN-M+S | 62F | * | * | **40** | 160 | * | 80 | * | * |
| | CH21 | * | * | **320** | 640 | * | 160 | * | * |
| | AA016 | * | * | **320** | 320 | * | 20 | * | * |

| | | | | | | | | | |
|---|---|---|---|---|---|---|---|---|---|
| ^{a} Monkey serum collected 3 weeks after the final vaccination. ^{b} Monkeys gene gun-vaccinated three times at 3 week intervals with the indicated DNA vaccine plasmid. ^{c} Monkeys s.c. injected with recombinant vaccinia virus vaccine two times at 42-day interval. ^{d} PRNT values are the reciprocal serum dilution that neutralizes the indicated virus plaque number by 80% or 50%. Homotypic titer are shown in bold. *, titer less than 20 | | | | | | | | | |

### Discussion

Transient expression of a cloned HTNV V gene was first accomplished by using recombinant vaccinia virus and baculovirus systems (Pensiero et al. 1988, J. Virol. 62, 696-702; Schmaljohn et al., 1989, supra). Both G1 and G2 proteins were expressed and appeared to be biochemically and antigenically identical to the authentic viral proteins. HTNV G1 and G2 were also transiently expressed from a plasmid containing HTNV gene controlled by a T7 promoter using the vaccinia virus/T7 RNA polymerase expression system (Kamrud and Schmaljohn, 1994, Virus Res. 31, 109-121). Problems in glycoprotein expression, however, arose when the HTNV M gene was cloned into plasmids utilizing a CMVpromoter. In most cases, G2 but not G1 was expressed as measured by RIPA (data not shown). For example, pcHTN-M, a plasmid used in a recent pseudotype study (Ma et al., 1999, Virus Res. 64, 23-32) was thought to express both G1 and G2; however, subsequent RIPA results performed in our laboratory indicate that only G2 was expressed correctly (unpublished data). It is possible that G1 is expressed but incorrectly processed (*e.g*., incorrectly folded, secreted from the cell, targeted to the nucleus, or targeted for degradation), or partially expressed (*e.g*., truncated).

We found that including a short sequence of extraneous DNA upstream of the SEOV or HTNV M gene allowed G1 expression from the DNA vaccine vector pWRG7077. In pWRG7077, the CMV promoter is followed by intron A. Intron A includes elements that function to increase levels of expression of the cloned gene (Chapman et al. 1991, Nucl. Acids. Res. 19, 3979-3986). After transcription, the intron A sequence is excised from the RNA and the spliced transcript is transported from the nucleus to the cytoplasm where it is expressed. We suspected that, if the splicing of intron A was aberrant and somehow resulted in disruption of the G1 sequence, then this might account for the observed defect in G1 expression, despite normal levels of G2 expression. The extraneous sequence, which was located between the intron A and hantavirus M gene, we hypothesized, might somehow prevent the hypothetical abberrant splicing and allow normal expression of G1 and G2. This hypothesis was bolstered when we noticed that a seven nucleotide sequence within the extraneous sequence was identical to the intron A splice acceptor site TCTGCAG (Stenberg *et al*., 1984, supra). However, mutation of the putative splice acceptor in the extraneous sequence had no effect on G1 expression (Fig. 9B). This does not rule out the possibility that some other splicing event influences G1 expression from the pWRG7077 plasmid. Further experimentation will be required to elucidate how a short run of nucleotides, GGATCTG, positioned upstream of the cloned SEOV and HTNV M gene is capable of affecting the expression of G1 but not G2. Regardless of the mechanism, this finding allowed us to develop a candidate DNA vaccine against HTNV.

Our previous results with the SEOV M DNA vaccine, coupled with the results reported here demonstrating immunogenicity and protective efficacy of the HTNV M DNA vaccine, make a strong case for the use of the full-length M gene in DNA vaccines against other hantaviruses. It remains unclear whether or not G1 alone, G2 alone, or fragments of the glycoproteins can elicit neutralizing antibody and protect against infection. Vaccination with recombinant baculovirus-infected cell lysates containing G1 or G2 alone, and recombinant vaccinia viruses expressing G1 or G2 alone, failed to elicit neutralizing antibody, and exhibited incomplete protection in a hamster infection model (Schmaljohn et al., 1990, J. Virol. 64, 3162-3170). These data suggest that a full-length M gene capable of expressing G1 and G2 may be required for protective immunity. In contrast, Bharadwai *et al.* reported low levels of neutralizing antibody after i.m. needle injection of mice with DNA vaccine plasmids containing short (∼166 amino acid) sections of the M gene of Sin Nombre virus (SNV), a HPS-associated hantavirus (Bharadwai et al., 1999, Vaccine 17, 2836-2843). This finding suggests that to elicit a neutralizing antibody response, it not only doesn't require a full-length M gene, but also occurs when only subunits of G1 or G2 are expressed. Presently, we are performing experiments designed to test the capacity of DNA vaccines that express HTNV G1 alone, G2 alone, or a combination of G1 and G2 (expressed from different plasmids) to elicit neutralizing antibody and protect hamsters from infection.

There are at least 10 hantaviruses known to cause either HFRS or HPS, so information on cross-neutralization and cross-protection among hantaviruses is important for the rational design of cross-protective vaccines. Investigators have evaluated the capacity of serum from various species (including humans) infected with hantaviruses to cross-neutralize other hantaviruses (Chu *et al*., 1995, supra; Niklasson et al., 1991, Am. J. Trop. Med. Hyg. 45, 660-665; Lee et al., 1985, J. Clin. Microbiol. 22, 940-944; Lundkvist et al. 1997, J. Med. Virol. 53, 51-59). Different species, and different individuals within a species, appear to exhibit differing levels of cross-neutralizing antibody. In general, data from these experiments indicate that serum from HTNV-, SEOV-, or DOBV-infected individuals share cross-neutralizing antibodies, albeit with about a fourfold difference in titer among the serotypes. Whereas, serum from PUUV-infected individuals exhibit little or no SEOV-, HTNV-, or DOBV- cross-neutralizing antibodies. Our data obtained with sera from infected, or DNA-vaccinated, hamsters are consistent with earlier findings. We observed a greater level of cross-neutralizing antibody in the pooled sera from the DNA-vaccinated hamsters than the pooled sera from infected hamsters. This may reflect a qualitative difference between the antibodies elicited after DNA vaccination and the antibodies elicited by infection, or it may simply reflect a quantitative difference in antibody levels. It is noteworthy that high homotypic neutralizing antibody levels in vaccinated hamsters did not necessarily correlate with cross-neutralizing activity (Fig 12).

The cross-neutralization tests with the hamster serum pools suggested that if neutralizing antibody could predict protective immunity, then vaccination with either pWRG/SEO-M or pWRG/HTN-M would protect against SEOV, HTNV, but not PUUV; and this we found, in general, to be true. Data in Fig. 12 indicate that the presence of cross-neutralizing antibodies correlated with a protective effect with a single exception. Hamster 2119 exhibited cross-neutralizing antibody, but was not protected by definition because there was a detectable anti-N response. However, the absence of detectable levels of cross-neutralizing antibody didn't necessarily predict a lack of protection. Examples of the latter case can be found in Fig. 12, hamsters 1437, 1434, 2122, and 2133. It is possible that cross-neutralizing antibodies are present, but not detected due to limitations of the assay, or that non-neutralizing responses also are protective.

In this study, we demonstrated that vaccination with either pWRG/SEO-M or pWRG/HTN-M cross-protected against SEOV, HTNV, and DOBV. Other experimental hantavirus vaccines have the capacity to cross-protect. For example a vaccinia recombinant expressing the HTNV G1, G2, and N (rW/HTN-M+S) cross-protected against infection with SEOV but not PUUV (Chu et al., 1995, J. Virol. 69, 6417-6423), and vaccinia recombinants expressing the SEOV G1 and G2, or N, cross-protected against HTNV (Xu et al., 1992, Am. J. Trop. Med. Hyg. 47, 397-404). Ours is the first report to demonstrate protection against DOBV.

The hantavirus M gene-based DNA vaccines not only elicited positive responses in rhesus macaques, but also elicited a level of neutralizing antibody that was relatively high. When we combined the serological data from the SEOV and HTNV M gene DNA thrice-vaccinated monkeys, the PRNT_{80%} GMT was 1470. This neutralizing antibody response was almost 10 times greater that elicited by the recombinant vaccinia virus vaccine, PRNT_{80%} GMT =160, PRNT_{50%} GMT = 320. The rW/HTN-M+S neutralizing antibody response was similar to the neutralizing antibody responses previously reported for humans, PRNT_{50%} GMT = 160 (McClain *et al*., 2000, supr). Inactivated hantavirus vaccines administered to humans also routinely elicit neutralizing antibody titers of -100 (Yu et al., 1999, In: Factors in the Emergence and Control of Rodent-borne Viral Diseases (Hantavirus and Arenal Diseases), J. F. Saluzzo and B. Dodet, Eds., pp. 157-161. Elsevier Press, Paris; Zhu et al. 1994, Chinese Med. J. 107, 167-170).

We found that the level of neutralizing antibody in the vaccinated monkeys dropped in the months after the final gene gun vaccination. Nevertheless, even after 6 months, neutralizing antibody could still be detected in all of the monkeys vaccinated with either pWRG/SEO-M(x) or pWRG/HTN-M(x). In comparison, monkeys vaccinated with rVV/HTN-M+S did not have detectable levels of neutralizing antibody after 4 months (Fig. 14).

In China, where several inactivated virus vaccines against hantaviruses have been developed and tested in humans, the PRNT assay is used to evaluate the potency of the vaccine (Zhu *et al*., 1994, supra, reviewed in Yu *et al*., 1999, supra). Most of the inactivated virus vaccines made in cell culture elicit neutralizing antibody in 90-100% of the vaccinees after three doses, GMT ≤100. The seropositive rate drops to -50% 6 months after the final boost, but can be restored by administering a booster vaccination. Although the hantavirus vaccines developed in Asia show promise in terms of efficacy, we have directed our efforts towards the development of recombinant hantavirus vaccines that avoid the safety concerns inherent to inactivated-virus vaccines. Moreover, we believe it might be possible to elicit a more robust neutralizing antibody response using a vaccine platform that entails expression of G1 and G2 within the cells of the vaccinee (*e.g*., DNA vaccine), rather than as exogenous proteins (*e.g*., beta-propiolactone-treated virions combined with adjuvant). The high neutralizing antibody response elicited in our DNA-vaccinated monkeys suggests that very high levels of neutralizing antibody are attainable by this vaccine approach.

### Example 12

**pWRG/HTN-M(x) partially protects against a lethal ANDV challenge.** We previously demonstrated that our HTNV M gene-based DNA vaccine, pWRG/HTN-M(x), could cross-protect against three of the four hantaviruses that cause HFRS. To determine if this vaccine could protect against the hantavirus that causes HPS, we vaccinated hamsters three times at 3-week intervals with pWRG/HTN-M(x) or a negative control plasmid and then challenged them with ANDV (250 LD₅₀, i.m.) 3 weeks after the last vaccination. Two independent experiments were performed and the combined results are shown in Fig. 15. The vaccine elicited HTNV NAb (50% neutralization geometric mean titers [GMT] = 226; range <20-5120) in all but one hamster. ANDV cross-NAb was not detected, with one exception (#514, titer= 20). After challenge, 15 of 24 hamsters vaccinated with pWRG/HTN-M(x) died and 14 of 15 negative controls died. The mean day-of-death was 13 for both groups. Thus, the protection against a lethal ANDV challenge afforded by vaccination with pWRG/HTN-M(x) was not statistically significant, but was suggestive (P= 0.0569). Hamsters that survived challenge were re-challenged with ANDV to ensure they were exposed to virus. At least one animal (ID#504) had no antibody response after two successive challenges but was clearly protected from lethal disease.

### Example 13

**ANDV M gene-based DNA vaccine expresses G1 and G2.** Because the level of protection afforded by pWRG/HTN-M(x) in our experiments was only 38%, we endeavored to construct an ANDV M gene-based DNA vaccine. The full-length M gene of ANDV, strain Chile-9717869, was RT-PCR cloned into pWRG7077 to yield pWRG/AND-M (SEQ ID NO:8). We sequenced the entire M gene open reading frame. The sequence of our cloned M gene was almost identical to the published M gene sequence of ANDV, GeneBank accession number AF291703, which is not surprising because the viral isolates were from the same rodent specimen (Meissner et al., 2002, Virus Res. 89, 131-143). There were two adenine to guanine nucleotide changes. The change at position 1504 was silent and the change at position 1840 resulted in a threonine to alanine substitution at amino acid 597.

Expression of G1 and G2 in transfected COS cells was evaluated by RIPA. Serum pools from convalescent HPS patients immunoprecipitated polypeptides with the predicted size of G1 and G2 from pWRG/AND-M-transfected cells or ANDV-infected cells (Fig. 17A-B). The sizes of the expression products were similar to the sizes of G1 and G2 of other hantaviruses; which range from 68-76 kDa for G1 and 52-58 kDa for G2 (Schmaljohn and Hooper, 2001, In: Fields Virology, 4th Edition, Lippincott, Williams, and Wilkins, Philadelphia, PA, pp. 1581-1602). We presume that the -46-kDa protein in the ANDV-infected cells but not the pWRG/AND-M transfected cells, was the ANDV nucleocapsid protein. To confirm that the polypeptides were G1 and G2, we screened a battery of HTNV G1- and G2-specific MAbs for capacity to immunoprecipitate the ANDV glycoproteins from cells transfected with pWRG/AND-M or pWRG/HTN-M(x). We were unable to identify HTNV G1-specific MAbs that cross-reacted with the ANDV G1 protein; MAb-2D5, MAb-6D4, MAb-8B6 (Fig. 17C), MAb-10F11, or MAb-3D5 (data not shown). HTNV G2-specific MAb-23G10, MAb-16E6, and MAb-HCO2 did not immunoprecipitate ANDV G2; however, HTNV G2-specific MAb-3D7 did immunoprecipitate ANDV G2 from the pWRG/AND-M transfected cells (Fig. 17C). By deduction, we concluded that the protein with mobility just under the 69-kDa marker that is precipitated by HPS patient sera but not the G2-specific MAb-3D7 is ANDV G1. The identity of the -70-kDa protein remains unknown.

### Example 14

**pWRG/AND-M DNA vaccine is neither immunogenic, nor protective in hamsters.** To test the immunogenicity of pWRG/AND-M, we vaccinated 24 hamsters three times at 3-week intervals using a gene gun, collected serum samples, and tested them for NAb by PRNT. ANDV-specific NAb were not detected (data not shown). Hamsters vaccinated with pWRG/HTN-M(x) on the same days did develop HTNV-specific NAb with titers as high as 10240 (data not shown). To determine if vaccination with pWRG/AND-M elicited ANDV G1- or G2-specific non-neutralizing antibodies, we performed IFAT with cells transfected with pWRG/AND-M. No antibody against G1 or G2 was detected in representative sera from hamsters vaccinated with pWRG/AND-M, but antibody was detected in positive control samples that were sera from hamsters previously infected with ANDV (data not shown). We repeated the entire vaccination experiment on a second group of seven hamsters and, again, no NAb response was detected (data not shown). Because there was a possibility the vaccinations with pWRG/AND-M elicited a nonhumoral, but nevertheless protective, immune responses, we challenged eight hamsters from the first experiment and seven hamsters from the second experiment with a 250 LD₅₀ of ANDV. Twelve of the 14 hamsters vaccinated with pWRG/AND-M developed HPS and died. Six of eight unvaccinated hamsters developed HPS and died. The mean-day-of-death was between 12 and 13 for both vaccinated and unvaccinated groups. From these negative data, we concluded that pWRG/AND-M was not immunogenic in Syrian hamsters.

### Example 15

**pWRG/AND-M elicits a high-titer NAb response in rhesus macaques.** To determine if the lack of immunogenicity of pWRG/AND-M in hamsters was a species-specific phenomenon, we tested this plasmid in a rhesus macaque. The monkey, ID# CH69, was vaccinated by gene gun with pWRG/AND-M four times at 3-week intervals. As positive controls, two rhesus macaques were vaccinated with pWRG/HTN-M(x), which we previously showed to be immunogenic in nonhuman primates. Serum collected before each vaccination and then 3 weeks after the fourth vaccination was tested for the presence of HTNV and ANDV NAbs (Fig. 18A,C). Monkey CH69 vaccinated with pWRG/AND-M and the positive control monkeys vaccinated with pWRG/HTN-M(x) developed NAb responses after the second vaccination and the NAb titers were extraordinarily high after the fourth vaccination (Fig. 18A,C).

We tested serum from the vaccinated monkeys for a capacity to cross-neutralize other HPS-associated hantaviruses. Serum from the monkey vaccinated with pWRG/AND-M, but not pWRG/HTN-M(x) neutralized SNV and BCCV (Fig. 18A). Serum from monkeys vaccinated with pWRG/AND-M or pWRG/HTN-M(x) did not cross-neutralize HTNV or ANDV, respectively.

Pre- and postvaccination (6 weeks after the last vaccination), serum was collected from monkey CH69 and tested by RIPA for a capacity to immunoprecipitate the ANDV glycoproteins. The post- but not prevaccination serum immunoprecipitated proteins similar to those immunoprecipitated by human convalescent sera: a strong G2 band, a weak G1 band, and a -70-kDa band of unknown identity (Fig. 18B).

To confirm that vaccination with pWRG/AND-M was immunogenic in rhesus macaques, we vaccinated a second animal four times at 3-week intervals. This monkey (ID# 90BD25) developed a PRNT₅₀ titer of 5120 after the second vaccination, and a titer of 10240 3 weeks after the fourth vaccination (Fig. 18C). Thus, the capacity of pWRG/AND-M to elicit high-titer NAb in nonhuman primates was reproducible.

To look at the duration of the NAb response in the monkeys vaccinated with the HPS or HFRS DNA vaccine, serum samples were periodically collected for approximately 6 months. The monkeys vaccinated with pWRG/AND-M still had PRNT₅₀ titers between 320 (monkey ID# 90BD25) and 2560 (monkey ID# CH69), 20 and 25 weeks after the last vaccine, respectively (Fig. 18C). The two positive control monkeys vaccinated with pWRG/HTN-M(x) still had homologous PRNT₅₀ titers ( 640 25 weeks after the last vaccination.

### Example 16

**Dual construct protects against HTNV and ANDV infection.** A construct comprising both the Hantaan M gene and the Andes M gene was prepared. The Hantaan M gene and the Andes M gene were cloned into pWRG7077 to produce pWRG/HA-M (SEQ ID NO:9). Each M gene was flanked by a cytomegalovirus promoter and intron A (CMV intron A) and a bovine growth hormone poly adenylation site. The entire Hantaan virus and Andes virus M gene open reading frames as well as most of the 5' and 3' noncoding sequences are included in the construct. In addition, the 24 bp sequence positioned between each hantavirus M sequence and its respective CMV intron A sequence. As discussed above, we have found, for unknown reasons, this 24 bp sequence (or a portion thereof) is required for expression of G1 glycoprotein.

When pWRG/HA-M was introduced into mammalian cells, the Hantaan virus and Andes virus M genes were expressed. The expression products consist of the Hantaan virus G1 and G2 glycoproteins and the Andes virus G1 and G2 glycoproteins.

The Hantaan/Andes dual-M gene hantavirus DNA vaccine, pWRG/HA-M, was tested for immunogenicity in rhesus macaques. The vaccine elicited an antibody response that neutralized Hantaan virus and Andes virus (see Table 8). In a single hamster experiment, we found that this plasmid was similar to the Andes virus plasmid, in that it did not elicit an antibody response in hamsters. However, the fact it elicits neutralizing antibodies in monkeys suggests it could elicit neutralizing antibodies in humans. This is the first DNA vaccine designed to protect against all hantaviruses, both HPRS-associated and HPS-associated, that cause severe disease.

### Example 17

### Passive transfer of serum from a monkey vaccinated with pWRG/AND-M 1 day before challenge delays or prevents HPS in hamsters.

Having successfully vaccinated rhesus macaques with a HPS DNA vaccine, we were interested in determining if the NAb response elicited by this vaccine could protect hamsters from a lethal ANDV infection. To test this, serum from a monkey vaccinated with pWRG/AND-M, or a monkey vaccinated with a negative control plasmid, was injected into groups of four hamsters. The next day the animals were challenged with ANDV (250 LD₅₀, i.m.) and observed daily for 4 weeks. All hamsters that received serum containing ANDV NAb survived and all hamsters that received negative control serum died between 10 and 13 days after challenge (Table 5). PRNT were performed on serum samples collected on the day of challenge (day 0) and collected from survivors on day 28. Hamsters that were injected with the protective monkey serum, which had an ANDV PRNT₅₀ titer of 20480, had PRNT₅₀ titers between 320 and 1280 at the time of challenge. Four weeks later, these hamsters had no detectable NAb and no nucleocapsid-specific antibody as measured by ELISA. Thus, these hamsters not only survived, but also the data suggest that they had sterile immunity to infection from the challenge.

In a second experiment, a different serum pool from a monkey vaccinated with pWRG/AND-M (ANDV PRNT₅₀ titer= 640) was injected into four hamsters. As negative controls, four hamsters were injected with serum from a monkey vaccinated with a negative control plasmid. The hamsters were challenged with ANDV (250 LD₅₀, i.m.) and observed daily for 14 weeks. As expected, all of the hamsters injected with the negative control monkey serum developed HPS and died between days 10 and 14 (Table 5). Whereas in the first experiment all of the hamsters injected with the serum from the monkey vaccinated with pWRG/AND-M were sterilely protected, in the second experiment two of the hamsters died on day 23, and the other two hamsters died on day 40. Together, the aforementioned experiments indicate that passive transfer of serum from a monkey vaccinated with pWRG/AND-M on the day before challenge can sterilely protect hamsters from a lethal ANDV challenge, or significantly delay disease onset and death.

**TABLE 5. Pre- and postchallenge passive transfer of sera from rhesus macaques vaccinated with pWRG/AND-M to hamsters**

| Antibody | | | | | | Terminal bleed^{f} | | % Protection | |
|---|---|---|---|---|---|---|---|---|---|
| Hamster ID# | Source^{a} | Day^{b} injected | Day 0 PRNT titer^{c} | Challenge virus^{d} | Day of death^{e} | PRNT titer^{g} | ELISA titer^{h} | From lethality | From infectionⁱ |
| 905 | AND-M | -1 | 320 | ANDV | - | <20 | <100 | 50 | 50 |
| 906 | | -1 | 640 | ANDV | - | <20 | <100 | | |
| 907 | | -1 | 320 | ANDV | - | <20 | <100 | | |
| 908 | | -1 | 1280 | ANDV | - | <20 | <100 | | |
| 105 | | -1 | 20 | ANDV | 23 | na | na | | |
| 106 | | -1 | 40 | ANDV | 23 | na | na | | |
| 107 | | -1 | 20 | ANDV | 40 | na | na | | |
| 108 | | -1 | 20 | ANDV | 40 | na | na | | |
| | | | | | | | | | |
| 901 | (-) | -1 | <20 | ANDV | 12 | na | na | 0 | 0 |
| 902 | | -1 | <20 | ANDV | 13 | na | na | | |
| 903 | | -1 | <20 | ANDV | 10 | na | na | | |
| 904 | | -1 | <20 | ANDV | 13 | na | na | | |
| 101 | | -1 | <20 | ANDV | 10 | na | na | | |
| 102 | | -1 | <20 | ANDV | 14 | na | na | | |
| 103 | | -1 | <20 | ANDV | 10 | na | na | | |
| 104 | | -1 | <20 | ANDV | 11 | na | na | | |
| | | | | | | | | | |
| 909 | AND-M | 3 | <20 | ANDV | - | <20 | <100 | 88 | 14 |
| 910 | | 3 | <20 | ANDV | - | <20 | 400 | | |
| 911 | | 3 | <20 | ANDV | - | 2560 | 12800 | | |
| | | | | | | | | | |

**TABLE 5 Continued. Pre- and postchallenge passive transfer of sera from rhesus macaques vaccinated with pWRG/AND-M to hamsters**

| Antibody | | | | | | Terminal bleed^{f} | | % Protection | |
|---|---|---|---|---|---|---|---|---|---|
| Hamster ID# | Source^{a} | Day^{b} injected | Day 0 PRNT titer^{c} | Challenge virus^{d} | Day of death^{e} | PRNT titer^{g} | ELISA titer^{h} | From lethality | From infectionⁱ |
| 912 | | 3 | <20 | ANDV | - | 160 | 800 | | |
| 109 | | 3 | <20 | ANDV | - | >640 | 1600 | | |
| 110 | | 3 | <20 | ANDV | - | 640 | 6400 | | |
| 111 | | 3 | <20 | ANDV | 12 | na | na | | |
| 112 | | 3 | <20 | ANDV | - | >640 | 3200 | | |
| | | | | | | | | | |
| 113 | AND-M | 4 | <20 | ANDV | - | 160 | 400 | 100 | 0 |
| 114 | | 4 | <20 | ANDV | - | 640 | 400 | | |
| 115 | | 4 | <20 | ANDV | - | >640 | 12800 | | |
| 116 | | 4 | <20 | ANDV | - | 320 | 1600 | | |
| | | | | | | | | | |
| 117 | AND-M | 5 | <20 | ANDV | - | 320 | 1600 | 100 | 0 |
| 118 | | 5 | <20 | ANDV | - | >640 | 1600 | | |
| 119 | | 5 | <20 | ANDV | - | 640 | 800 | | |
| 120 | | 5 | <20 | ANDV | - | 160 | 400 | | |
| | | | | | | | | | |
| 121 | AND-M | 6 | <20 | ANDV | 11 | na | na | 38 | 0 |
| 122 | | 6 | <20 | ANDV | - | 40 | 800 | | |
| 123 | | 6 | <20 | ANDV | 13 | na | na | | |
| 124 | | 6 | <20 | ANDV | 12 | na | na | | |
| | | | | | | | | | |

**TABLE 5 Continued. Pre- and postchallenge passive transfer of sera from rhesus macaques vaccinated with pWRG/AND-M to hamsters**

| Antibody | | | | | | Terminal bleed^{f} | | % Protection. | |
|---|---|---|---|---|---|---|---|---|---|
| Hamster ID# | Source^{a} | Day^{b} injected | Day 0 PRNT titer^{c} | Challenge virus^{d} | Day of death^{e} | PRNT titer^{g} | ELISA titer^{h} | From lethality | From infectionⁱ |
| 913 | | 6 | <20 | ANDV | 11 | na | na | | |
| 914 | | 6 | <20 | ANDV | - | 1280 | 12800 | | |
| 915 | | 6 | <20 | ANDV | 10 | na | na | | |
| 916 | | 6 | <20 | ANDV | - | 1280 | 6400 | | |
| | | | | | | | | | |
| 917 | AND-M | 9 | <20 | ANDV | 11 | na | na | 25 | 0 |
| 918 | | 9 | <20 | ANDV | 13 | na | na | | |
| 919 | | 9 | <20 | ANDV | - | 10240 | 12800 | | |
| 920 | | 9 | <20 | ANDV | 11 | na | na | | |
| | | | | | | | | | |
| 921 | AND-M | -1 | 160 | PBS | - | <20 | <100 | 100 | 100 |
| 922 | | -1 | 640 | PBS | - | <20 | <100 | | |
| 923 | | -1 | <20 | PBS | - | <20 | <100 | | |
| 924 | | -1 | 160 | PBS | - | <20 | <100 | | |

| | | | | | | | | | |
|---|---|---|---|---|---|---|---|---|---|
| ^{a}Serum from monkey vaccinated with pWRG/AND-M (AND-M) or negative control sera (-). ^{b} Day antibody injected relative to challenge (Day 0). ^{c} NAb titer in hamster serum at time of challenge. ^{d} 2000 PFU of ANDV (250 LD₅₀), or PBS was injected i.m. on day 0. ^{e} -, animal survived. ^{f} In exp 1 (ID# 909-924), terminal bleed on day 28. In exp 2 (ID# 109-124), terminal bleed on day 98. ^{g} Reciprocal of highest dilution neutralizing 50% of the plaques; na, not applicable. ^{h} Endpoint ELISA titer. ⁱ Sterile immunity indicates no detectable antibody response to nucleocapsid. | | | | | | | | | |

**TABLE 6. Passive transfer of sera from NHP vaccinated with pWRG/HTN-M(x) in a nonlethal infection model.**

| | | | | Terminal bleed^{f} | | |
|---|---|---|---|---|---|---|
| Hamster ID# | Antibody source^{a} | Day 0 PRNT titer^{b} | Challenge virus^{c} | PRNT titer^{d} | ELISA titer^{e} | % Sterile immunity^{g} |
| 492 | HTN-M | 640 | HTNV | 20 | <100 | Y |
| 493 | | 1280 | HTNV | 80 | <100 | Y |
| 494 | | 640 | HTNV | 20 | <100 | Y |
| 495 | | 1280 | HTNV | 20 | 200 | N |
| 496 | | 640 | HTNV | <20 | <100 | Y |
| 497 | | 1280 | HTNV | 1280 | <100 | Y |
| 498 | | <20 | HTNV | 5120 | 12800 | N |
| 499 | | 640 | HTNV | ND | <100 | Y |
| | | | | | | |
| 484 | (-) | <20 | HTNV | >640 | >12800 | N |
| 485 | | 20 | HTNV | >640 | >12800 | N |
| 486 | | <20 | HTNV | >640 | >12800 | N |
| 488 | | <20 | HTNV | >640 | >12800 | N |
| 489 | | <20 | HTNV | >640 | >12800 | N |
| 490 | | <20 | HTNV | >640 | 3200 | N |
| 491 | | <20 | HTNV | >640 | >12800 | N |

**TABLE 6 Continued. Passive transfer of sera from NHP vaccinated with pWRG/HTN-M(x) in a nonlethal infection model.**

| | | | | Terminal bleed^{f} | | |
|---|---|---|---|---|---|---|
| Hamster ID# | Antibody source^{a} | Day 0 PRNT titer^{b} | Challenge virus^{c} | PRNT titer^{d} | ELISA titer^{e} | % Sterile immunity^{g} |
| 476 | none | <20 | HTNV | >640 | >12800 | N |
| 477 | | <20 | HTNV | >640 | >12800 | N |
| 478 | | <20 | HTNV | >640 | >12800 | N |
| 479 | | <20 | HTNV | >640 | >12800 | N |
| 480 | | <20 | HTNV | >640 | >12800 | N |
| 481 | | <20 | HTNV | >640 | >12800 | N |
| 482 | | <20 | HTNV | >640 | >12800 | N |
| 483 | | <20 | HTNV | >640 | >12800 | N |

| | | | | | | |
|---|---|---|---|---|---|---|
| ^{a} HTN-M indicates serum from monkey vaccinated with pWRG/HTN-M(x); (-) indicates serum from negative control monkey vaccinated with irrelevant plasmid; none indicates no passive transfer. ^{b} NAb titer (50%) in hamster serum at time of challenge. ^{c} 2000 PFU of HTNV (1000 LD₅₀) was injected i.m. on day 0. ^{d} Reciprocal of highest dilution neutralizing 50% of the plaques; ND, not done. ^{e} Endpoint ELISA titer. ^{f} Terminal bleed was on day 35 after challenge. ^{g} Sterile immunity indicates no detectable antibody response to nucleocapsid. Y and N, yes and no sterile immunity, respectively. | | | | | | |

**TABLE 7. Pre- and postchallenge passive transfer of plasma from a Chilean HPS patient to hamsters**

| Antibody | | | | | | Terminal bleed^{f} | | % Protection | |
|---|---|---|---|---|---|---|---|---|---|
| Hamster ID# | Source^{a} | Day^{b} injected | Day 0 PRNT titer^{c} | Challenge virus^{d} | Day of death^{e} | PRNT titer^{g} | ELISA titer^{h} | From lethality | From infectionⁱ |
| 125 | Normal | -1 | <20 | ANDV | 12 | na | na | 0 | 0 |
| 126 | | | <20 | ANDV | 10 | na | na | | |
| 127 | | | <20 | ANDV | 11 | na | na | | |
| 128 | | | <20 | ANDV | 12 | na | na | | |
| | | | | | | | | | |
| 129 | HPS | -1 | 640 | ANDV | - | <20 | <100 | 50 | 50 |
| 130 | | | 1280 | ANDV | 57 | na | na | | |
| 131 | | | 640 | ANDV | 68 | na | na | | |
| 132 | | | 1280 | ANDV | - | <20 | <100 | | |
| | | | | | | | | | |
| 133 | HPS | 3 | <20 | ANDV | - | >640 | 3200 | 75 | 0 |
| 134 | | | <20 | ANDV | - | 160 | 800 | | |
| 135 | | | <20 | ANDV | - | 80 | 800 | | |
| 136 | | | <20 | ANDV | 12 | na | na | | |
| | | | | | | | | | |
| 137 | HPS | 4 | <20 | ANDV | 12 | na | na | 50 | 0 |
| 138 | | | <20 | ANDV | 15 | na | na | | |
| 139 | | | <20 | ANDV | - | 320 | 1600 | | |
| 140 | | | <20 | ANDV | - | 320 | 3200 | | |
| | | | | | | | | | |

**TABLE 7 Continued Pre- and postchallenge passive transfer of plasma from a Chilean HPS patient to hamsters**

| Antibody | | | | | | Terminal bleed^{f} | | % Protection | |
|---|---|---|---|---|---|---|---|---|---|
| Hamster ID# | Source^{a} | Day^{b} injected | Day 0 PRNT titer^{c} | Challenge virus^{d} | Day of death^{e} | PRNT titer^{g} | ELISA titer^{h} | From lethality | From infectionⁱ |
| 141 | HPS | 5 | <20 | ANDV | - | >640 | 6400 | 50 | 0 |
| 142 | | | <20 | ANDV | - | 40 | <100 | | |
| 143 | | | <20 | ANDV | 11 | na | na | | |
| 144 | | | <20 | ANDV | 11 | na | na | | |
| | | | | | | | | | |
| 145 | HPS | -1 | 40 | None | - | <20 | <100 | 100 | 100 |
| 146 | | | 640 | None | - | <20 | <100 | | |
| 147 | | | 320 | None | - | <20 | <100 | | |

| | | | | | | | | | |
|---|---|---|---|---|---|---|---|---|---|
| ^{a} HPS indicates plasma from a human HPS patient; normal indicates normal human serum. ^{b} Day antibody injected (1 ml i.p.) relative to challenge (Day 0). ^{c} NAb titer in hamster serum at time of challenge. ^{d} 2000 PFU of ANDV (250 LD₅₀) was injected i.m.(ANDV), or no treatment (None). ^{e} -, animal lived. ^{f} Terminal bleed on day 98. ^{g} Reciprocal of highest dilution neutralizing 50% of the plaques; na, not applicable. ^{h} Endpoint ELISA titer. ⁱ Protection from infection (sterile immunity) indicates no detectable antibody response to nucleocapsid. | | | | | | | | | |

### Example 18

**Passive transfer of serum from a monkey vaccinated with pWRG/AND-M to hamsters 4-5 days after ANDV challenge completely protects against lethal HPS.** We tested serum from a monkey vaccinated with pWRG/AND-M for protective efficacy when administered to hamsters 3, 4, 5, 6, or 9 days *after* ANDV challenge (Table 5). Fifteen of the 16 hamsters that received antibody on days 3, 4, or 5 after challenge survived. However, if antibody was administered 6 days after challenge, only three of eight hamsters survived; and if antibody was administered 9 days after challenge only one of four hamsters survived. All but one of the survivors from the postexposure experiments (ID#909, day 3) were infected with ANDV, as measured by PRNT and nucleocapsid-specific ELISA. Unlike the pre-challenge passive transfer experiments described above, in the postchallenge passive transfer experiments, we did not observe any late deaths. Thus, immunoprophlaxis with serum from monkeys vaccinated with pWRG/AND-M, up to 5 days after a lethal challenge with ANDV, protected from 88-100% of the ANDV-challenged hamsters.

### Example 19

**Passive transfer of sera from HTN-M DNA-vaccinated monkeys protects hamsters against HTNV infection.** In earlier studies we demonstrated that vaccinating hamsters with pWRG/HTN-M(x) elicited NAbs and protected against infection; however, we never tested whether or not the antibodies elicited were, by themselves, were sufficient to protect. To address this question, groups of hamsters were either not injected, or were injected with a pool of sera from monkeys vaccinated four times with pWRG/HTN-M(x) (PRNT₅₀ titer= 20480), or from a monkey vaccinated with a negative control plasmid. One day after passive transfer, the hamsters were bled and challenged with HTNV. Five weeks after challenge, the hamsters were terminally bled and serum from day 0 and day 35 were tested for HTNV NAbs by PRNT, and nucleocapsid-specific antibodies by ELISA (Table 6). All of the hamsters that were injected with the negative control serum or no serum were clearly infected as indicated by high PRNT and nucleocapsid-specific ELISA titers. In contrast, six of the eight hamsters injected with the serum from the monkeys vaccinated with pWRG/HTN-M(x) failed to develop detectable anti-nucleocapsid antibodies by ELISA. Hamster #495 had a low ELISA titer indicating a low level of infection. Although hamster #498 was in the group injected with immune serum, no NAb response was detected at the time of challenge, indicating antibody was not successfully transferred, which explains why this animal was not protected against HTNV infection. Thus, serum from nonhuman primates vaccinated with pWRG/HTN-M(x), administered 1 day before challenge, was sufficient to protect hamsters against infection with HTNV.

### Example 20

**Passive transfer of plasma from human HPS patient.** Having demonstrated that serum from monkeys vaccinated with ANDV M gene-based DNA vaccines could protect against a lethal ANDV challenge when administered pre- or postexposure, we investigated the capacity of human convalescent plasma from a Chilean HPS patient (human-HPS plasma) to protect in the ANDV/hamster lethal disease model. Human-HPS plasma, with an initial ANDV NAb PRNT₅₀ titer of 10240, was injected into different groups of hamsters on day -1, 3, 4, or 5. A control group was injected with normal human serum on day -1. On day 0, blood was collected from the hamsters and then the hamsters were challenged with ANDV (250 LD₅₀, i.m.). As a control for toxicity, three hamsters that were injected with the human-HPS plasma on day -1 were not challenged. Hamsters were monitored for 98 days, survivors were terminally bled, and PRNT and ELISA were performed on the day 0 and day 98 serum samples (Table 7). All of the hamsters receiving normal human sera died between 10 and 12 days after challenge. Two of the four hamsters that received human-HPS plasma on day -1 survived. The other two hamsters in the day -1 group developed HPS and died on days 57 and 68 after challenge. Seven of the 12 hamsters that received antibody on days 3, 4, or 5 after challenge survived. The hamsters that died in those groups did so between 11 and 15 days after challenge. The three hamsters in the toxicity control groups all remained healthy.

Serology results from the postchallenge sera indicate that the two hamsters that were injected with human-HPS plasma on day -1, and survived, were protected from infection (i.e., they did not produce anti-nucleocapsid antibodies)(Table 7). All but one of the hamsters that received the human-HPS plasma after challenge, and survived, were nevertheless infected because they all produced anti-nucleocapsid antibodies. Thus, human-HPS plasma protected at least half the challenged hamsters when administered on day -1, 3, 4, or 5. Hamsters receiving the plasma on day -1 were either protected from infection, or survived more than four times longer than the controls.

### DISCUSSION

Presently, there are no vaccines, effective antiviral drugs, or immunologics to protect against or treat HPS. This is disconcerting because HPS afflicts previously healthy individuals in all age groups, disease progression is rapid, and the case-fatality is one of the highest for any acute viral disease known. Reports of person-to-person transmission of ANDV-associated HPS in southern Argentina and Chile make development of countermeasures against this highly lethal disease more urgent (Padula et al., 1998, Virology 241, 323-330; Toro et al., 1997, Emerg. Infect. Dis. 4, 687-694; Wells et al., 1997, Emerg. Infect. Dis. 3, 171-174). Here, we report the development of a candidate HPS DNA vaccine that elicits high-titer NAb in nonhuman primates. Moreover, we report that the antibodies elicited by this vaccine can protect hamsters from lethal HPS, even when administered 5 days after challenge.

This is the first study in which a hantavirus disease model in an adult laboratory animal has been used to evaluate the protective efficacy of a candidate vaccine, drug, or immunoprophylactic. Previous studies used hantavirus infection models involving mice, hamsters, or bank voles to evaluate vaccines and immunoprophylactics, or a suckling mouse neurologic-disease model to evaluate drugs (Hooper and Li, 2001, In: Hantaviruses, p. 171-191, Springer-Verlag, Berlin, Germany; Schmaljohn and Hooper, 2001, supra) Here, we used a recently described ANDV/hamster lethal disease model (Hooper et al., 2001, Virology 289, 6-14). We confirmed that ANDV causes lethal HPS in adult Syrian hamsters and determined that the challenge had a relatively consistent outcome. Of the 35 hamsters that were negative controls in the active vaccination or passive transfer experiments, 100% of the ANDV-challenged hamsters were infected and 91% developed fatal HPS (mean-day-of-death= 12, range 10-15 days). Onset of symptoms (*e.g*., dyspnea) was rapid and death usually occurred within 24 h after onset.

In an earlier study, we demonstrated that previous infection with HTNV, SEOV, DOBV, PUUV, or SNV could protect hamsters against a lethal ANDV challenge (Hooper et al., 2001, supra). From this, we reasoned that it might be possible to use our HTNV M gene-based DNA vaccine, pWRG/HTN-M(x), to protect hamsters against ANDV challenge. We tested this possibility and found that pWRG/HTN-M(x) protected 9 of 24 hamsters from a lethal ANDV challenge. This level of protection was not statistically significant (P= 0.0569) but did suggest that the vaccine was eliciting some immunity against ANDV. It is possible that the pWRG/HTN-M(x) vaccine would afford a higher level of protection against a challenge dose less than 250 LD₅₀, or against a different challenge route (*e.g*., aerosol or oral route). There was essentially no ANDV cross-neutralizing antibodies in either the serum of pWRG/HTN-M(x)-vaccinated hamsters (Fig.14) or in the HTNV-infected hamsters that were protected in our earlier work (Hooper et al., 2001, supra). This suggests that the cross-protection we observed in previously infected hamsters was due to cell-mediated-immunity targeting both nucleocapsid and glycoproteins; whereas the partial cross-protection we observed in the pWRG/HTN-M(x)-vaccinated hamsters was likely due to cell-mediated-immunity targeting only the glycoproteins. It is theoretically possible that the product of the L genome segment could play a role in the protection observed in previously infected hamsters; however, there is presently no evidence that the viral polymerase elicits humoral- or cell-mediated-immunity. Cross-protection after infection with HTNV, but not after inoculation with the HTNV glycoproteins, suggests that vaccination with a plasmid(s) expressing both the HTNV nucleocapsid and glycoproteins might mimic HTNV infection and cross-protect against ANDV.

In an alternative approach to developing a HPS vaccine, we constructed a plasmid designed to specifically protect against viruses that cause HPS. The plasmid, pWRG/AND-M, contains the complete ANDV M gene open reading frame and expresses both the G1 and G2 glycoproteins in cell culture. To our knowledge, the BCCV M gene is the only other HPS-hantavirus complete M gene that has been successfully cloned and expressed (Ravkov et al., 1998, J. Virol. 72, 2865-2870). In that study the BCCV M gene was cloned into a Sindbis virus replicon system, SIN-rep5, and expression was detected in BHK21 cells by immunoprecipitation.

Vaccination with pWRG/AND-M elicited a potent NAb response in rhesus macaques. This is the first candidate HPS vaccine shown to unequivocally elicit a NAb response in any laboratory animal. Not only were NAbs produced in the monkeys, but also 1) NAbs were detected after only two vaccinations; 2) NAb titers were very high (*i.e*., titers as high as those found in HPS-patient convalescent serum); 3) the NAbs cross-neutralized at least two other HPS-associated hantaviruses, SNV and BCCV; and 4) NAbs were still detected 6 months after the last vaccination. The neutralization of SNV suggests that the pWRG/AND-M vaccine would protect against the major HPS-hantaviruses in South America and North America, ANDV and SNV, respectively. The neutralization of BCCV suggests that vaccination with pWRG/AND-M might protect against a wide range of HPS-associated hantaviruses (See Table 8). These data coupled with our HFRS DNA vaccine data indicate that a vaccine comprised of both pWRG/HTN-M(x) and pWRG/AND-M could conceivably protect against several major hantaviruses known to be highly lethal to humans.

**Table 8. Immunogenicity of hantavirus M gene-based DNA vaccines in nonhuman primates: response after 1 through 4 vaccinations.**

| | | | Titer 3 wks after vaccination^{b} | | | |
|---|---|---|---|---|---|---|
| Exp ^{a} | DNA vaccine plasmid | Monkey ID # | vacc1 | vacc2 | vacc3 | vacc4 |
| I | pWRG/SEO-M(x) | CH03 | <20 | 160 | 1280 | na |
| I | " | CH32 | <20 | 160 | 1280 | na |
| I | pWRG/HTN-M(x) | CH28 | 40 | 1280 | 2560 | na |
| I | " | CH02 | 40 | 1280 | 10240 | na |
| I | " | CH27 | <20 | 320 | 1280 | na |
| II | " | CH64 | <20 | 160 | 1280 | 20480 |
| II | " | CH85 | <20 | 160 | 320 | 20480 |
| II | pWRG/AND-M | CH69 | <20 | 2560 | 2560 | 20480 |
| III | " | 90BD25 | <20 | 5120 | 5120 | 10240 |
| III | pWRG/HA-M^{c} | CAA | HTN<20 | 160 | 80 | 320 |
| | | | AND<20 | <20 | <20 | 320 |
| | " | HJV | HTN<20 | 320 | 160 | >640 |
| | | | AND<20 | 40 | 80 | 640 |

| | | | | | | |
|---|---|---|---|---|---|---|
| ^{a} Three independent experiments were performed. Monkeys received 8 g.g. administrations per vacc. ^{b} PRNT₅₀ titer against homotypic virus. For pWRG/HA-M, Hantaan (HTN) and Andes (AND) titers are given. ^{c} pWRG/HA-M is a plasmid containing both the Andes virus and Hantaan virus M gene. | | | | | | |

All monkey vaccinated with our Hantaan, Seoul, Andes, or Hantaan/Andes M gene-based DNA vaccines elicited neutralizing antibody levels that were still detectable after six months. Two of three monkeys vaccinated with pWRG/HTN-M(x) still had detectable levels of neutralizing antibodies two years after the last vaccination (Table 9). A summary of duration of immunity data is shown in Table 10.

**Table 9. Immunogenicity of hantavirus M gene-based DNA vaccines in nonhuman primates: duration of immunity.**

| | | | PRNT₅₀ Titer | | | | |
|---|---|---|---|---|---|---|---|
| | | | Months after final vaccination | | | | |
| Exp ^{a} | DNA vaccine plasmid | Monkey ID # | ~1 | ~6 | ~12 | ~18 | ~24 |
| I | pWRG/SEO-M(x) | CH03 | 1280 | 160 | 320 | nd | |
| I | | CH32 | 1280 | 320 | 40 | nd | |
| I | pWRG/HTN-M(x) | CH28 | 2560 | 320 | 80 | 160 | 80 |
| I | | CH02 | 10240 | 320 | 80 | 640 | 80 |
| I | | CH27 | 1280 | 40 | <20 | <20 | <20 |
| II | | CH64 | 20480 | 640 | nd | | |
| II | | CH85 | 20480 | 1280 | nd | | |
| II | pWRG/AND-M | CH69 | 20480 | 2560 | nd | | |
| III | | 90BD25 | 10240 | 320 | nd | | |

| | | | | | | | |
|---|---|---|---|---|---|---|---|
| ^{a} Three independent exp. were performed. Monkeys received 8 g.g. administrations per vacc. ^{b} PRNT₅₀ titer against homotypic virus. | | | | | | | |

**Table 10. Immunogenicity of hantavirus M gene-based DNA**

| **vaccines in nonhuman primates: duration of immunity.** | | | | | |
|---|---|---|---|---|---|
| | | Geometeric Mean Titer^{a} | | | |
| | | Months after final vaccination | | | |
| DNA vaccine | ~1 | ~6 | ~12 | ~18 | ~24 |
| pWRG/SEO-M(x) | 1280 | 226 | 113 | nd | nd |
| pWRG/HTN-M(x) | 6756 | 320 | 40 | 101 | 40 |
| pWRG/AND-M | 14482 | 269 | nd | nd | nd |
| all | 5530 | 373 | nd | nd | nd |

| | | | | | |
|---|---|---|---|---|---|
| ^{a} PRNT₅₀ titer against homotypic virus | | | | | |

One of the more perplexing results we obtained during the course of this study was the failure of pWRG/AND-M to elicit a detectable immune response in hamsters. In two independent experiments, hamsters vaccinated three times with pWRG/AND-M failed to produce antibodies detectable by PRNT or IFAT, and were not protected against a lethal ANDV challenge. In contrast, rhesus macaques vaccinated as few as two times with pWRG/AND-M developed a detectable NAb response. Although unlikely, it is possible that the DNA vaccine was nonimmunogenic in hamsters for technical reasons. Before exploring the mechanism by which pWRG/AND-M might fail to generate an immune response in hamsters, and whether this phenomenon is related to the highly lethal nature of ANDV in Syrian hamsters, it would be prudent to evaluate the pWRG/AND-M vaccine in hamsters and nonhuman primates in a side-by-side experiment using the same vaccine lot and vaccination schedule in both species. For the current study, the important point is that pWRG/AND-M is highly immunogenic in nonhuman primates.

In previous studies we demonstrated that DNA vaccines expressing the glycoproteins of SEOV or HTNV elicited NAb in mice, hamsters, and nonhuman primates, and we demonstrated that the presence of NAb in vaccinated hamsters correlated with protection against infection (Hooper et al., 1999, Virology 255, 269-78; Kamrud et al., 1999, Virology 263, 209-219). Here, we demonstrate for the first time that the humoral response elicited by DNA vaccination with pWRG/HTN-M(x) is sufficient to protect against infection with HTNV. Specifically, we demonstrated that passive transfer of serum from a monkey vaccinated with pWRG/HTN-M(x) 1 day before challenge provided sterilizing protection in six of eight hamsters challenged with HTNV. The two hamsters that were not protected either had a limited infection (hamster #495) as determined by a very low postchallenge, anti-nucleocapsid titer; or did not receive a proper injection of serum (hamster #498) as determined by the absence of detectable NAb on the day of challenge. It is possible that a similar passive transfer assay could be used as part of a clinical study to evaluate sera from humans vaccinated with pWRG/HTN-M(x), and to predict efficacy of the vaccine.

Presently there is no treatment for individuals exposed, or potentially exposed, to HPS hantaviruses. Reports that high NAb titers in patients admitted to hospitals correlated with favorable clinical course suggested that NAbs might ameliorate disease, and therefore, immunotherapy could be a viable treatment option to persons exposed to HPS viruses (Bharadwaj et al., 2000, J. Infect. Dis. 182, 43-48). For HFRS-associated hantaviruses, passive transfer of infected-rat immune serum or mouse MAbs conferred protection against infection in hamsters or neurologic disease in newborn rats or suckling mice (Arikawa et al., 1989, J. Gen. Virol. 70, 615-24; Liang et al., 1996, Virology 217, 262-271; Schmaljohn et al., 1997, J. Virol. 71, 9563-9569; Zhang et al., 1989, Arch. Virol. 105, 235-246). For HPS hantaviruses, there have been no reports of the passive transfer of protective immunity. The discovery of the ANDV/hamster lethal disease model has allowed us, for the first time, to evaluate the capacity of antibodies to protect against a hantavirus disease that resembles the disease in humans. Using this model, we demonstrated that passive transfer of serum from a monkey vaccinated with pWRG/AND-M protected 100% of challenged hamsters from a lethal ANDV infection when administered on day 4 or 5 after exposure. This finding indicates that it should be possible to develop a product (*e.g*., polyclonal immunoglobulin from primates, including humans, vaccinated with pWRG/AND-M and/or pWRG/HTN-M(x), or MAbs that neutralize ANDV and/or HTNV) that could be used to treat laboratory workers exposed to hantaviruses, family members or other close contacts of HFRS or HPS patients, or medical personnel treating HPS cases, especially in South America where there is evidence of person-to-person transmission of HPS(Padula et al, 1998, Virology 241, 323-330; Toro et al., 1997, supra; Wells et al., 1997, Emerg. Infect. Dis. 3, 171-174).

HPS-patient convalescent plasma was essentially equivalent to the monkey serum when administered on day 3 postexposure (75% vs 88% protection) but only protected 50% of the hamsters when administered on day 4 or 5 postexposure. Due to low sample numbers, this difference could be an artifact. Alternatively, it is possible the human NAbs were cleared more rapidly than the rhesus macaque NAbs resulting in a lower effective NAb titer at the time of challenge. We have not evaluated the kinetics by which the monkey or human hantavirus-specific NAbs are cleared from hamsters.

Passive transfer of sera from monkeys vaccinated with pWRG/AND-M, or HPS-patient plasma, administered 1 day before challenge, either protected hamsters against a lethal ANDV challenge with sterile immunity, or delayed disease onset and death by two- to almost fourfold. In the experiments involving passive transfer of serum containing ANDV NAb (Table 5 and 7), the only hamsters that died late were those that were injected with serum 1 day before challenge. The six hamsters died on days 23, 23, 40, 40, 57, and 68 for a mean-day-of-death = 42. This was a significant delay in death because the 12 hamsters that were injected with negative control serum (monkey or human) all died between days 10 and 14, mean-day-of-death = 12. We surmise that the late deaths in the hamsters injected with antibody on day -1 were the result of clearance of the heterologous monkey or human ANDV-specific NAb, and subsequent virus amplification from virus introduced either "from without" (*e.g*., ANDV shed from hamsters in other cages in the same room) or "from within" (*e.g*., virus that escaped neutralization by the passively transferred antibody and infected a cell). We hypothesize that late deaths were not observed in hamsters that received serum postexposure because the challenged hamsters had mounted an active immune response to the challenge virus. This active immunity would normally be insufficient to protect the hamsters against ANDV; however, passively transferred immunity (*i.e*., NAbs in serum from a monkeys vaccinated with pWRG/AND-M) administered on day 3 thru 5 would tip the balance in favor of the hamster and allow survival (22 of 28 survived, see tables 6 and 7). The active immune response would persist and protect the hamster when, and if, the hamster was reexposed to ANDV after clearance of the passive immunity. In support of this, all but one (#902, Table 5) of the 26 survivors that received passively transferred antibody on day 3, 4, 5, 6, or 9, were nevertheless infected, as indicated by a anti-nucleocapsid and/or NAb response detectable several weeks after challenge. Regardless of whether the late deaths can be attributed to re-exposure from "within" or from "without," these data suggest that a post-hantavirus-exposure prophylaxis treatment regimen might require repeated administrations, or both passive transfer of antibodies and active vaccination. A passive/active vaccination approach for postexposure prophylaxis is routinely used to prevent other viral diseases including hepatitis A, hepatitis B, and rabies (Centers for Disease Control, Atlanta, Georgia, 1991, MMWR 40, 1-19, MMWR 48, 1-21, MMWR 48, 1-37).

Most of the hamsters that received the ANDV-specific NAb on day 6 or 9 died, indicating that immunoprophylaxis might become less effective as disease progresses. Others have reported that the small molecule drug ribavirin might have been ineffective for treating HPS because, for most patients, drug treatment was initiated after onset of clinical symptoms (Chapman et al, 1999, Antivir. Ther. 4, 211-219). The critical pathogenic juncture where damage caused by the infection is irreversible and prophylaxis is impossible has not been defined. Future studies characterizing the time course of HPS in the Andes/hamster lethal disease model will provide insights into the pathogenesis of HPS, and should allow us to formulate strategies for pre- and postexposure prophylaxis and therapy.

### SEQUENCE LISTING

Seq ID NO:1 SEOV M genome segment 
tagtagtaga ctccgcaaga aacagcagtt aaagaacaat 40
aggatcatgt ggagtttgct attactggcc gctttagttg 80
gccaaggctt tgcattaaaa aatgtatttg acatgagaat 120
tcagttgccc cactcagtca actttgggga aacaagtgtg 160
tcaggctata cagaatttcc cccactctca ttacaggagg 200
cagaacagct agtgccagag agctcatgca acatggacaa 240
ccaccagtca ctctcaacaa taaataaatt aaccaaggtc 280
atatggcgga aaaaagcaaa tcaggaatca gcaaaccaga 320
attcatttga agttgtggaa agtgaagtca gctttaaagg 360
gttgtgtatg ttaaagcata gaatggttga agaatcatat 400
agaaatagga gatcagtaat ctgttatgat ctagcctgta 440
atagtacatt ctgtaaacca actgtttata tgattgttcc 480
tatacatgct tgcaacatga tgaaaagctg tttgattggc 520
cttggcccct acagaatcca ggttgtctat gaaaggacat 560
actgcactac gggtatattg acagaaggaa aatgctttgt 600
ccctgacaag gctgttgtca gtgcattgaa aagaggcatg 640
tatgctatag caagcataga gacaatctgc ttttttattc 680
atcagaaagg gaatacatat aagatagtga ctgccattac 720
atcagcaatg ggctccaaat gtaataatac agatactaaa 760
gttcaaggat attatatctg tattattggt ggaaactccg 800
cccctgtata tgcccctgct ggtgaagact tcagagcaat 840
ggaggttttt tctgggatta ttacatcacc acatggagaa 880
gaccatgacc tacccggcga agaaatcgca acgtaccaga 920
tttcagggca gatagaggca aaaatccctc atacagtgag 960
ctccaaaaac ttaaaattga ctgcttttgc aggtattcca 1000
tcatactcat caactagtat attggctgct tcagaagatg 1040
gtcgtttcat atttagtcct ggtttatttc ctaacctaaa 1080
tcagtcagtc tgtgacaaca atgcactccc tttaatctgg 1120
aggggcctaa ttgatttaac gggatactat gaggcagtcc 1160
acccttgcaa tgtgttctgt gtcttatcag gaccaggtgc 1200
ttcatgtgag gccttttcag aaggaggtag ggcaatattt 1240
acttctccaa tgtgtctggt gtctaagcaa aatagattta 1280
gagcagctga gcagcagatt agctttgtct gccaaagagt 1320
tgatatggat attatagtgt actgtaatgg tcagaaaaaa 1360
acaatcctaa caaaaacatt agttataggc caatgtattt 1400
atactattac aagtctcttt tcactgttac caggggttgc 1440
ccattctatt gctattgagt tgtgtgttcc agggtttcat 1480
ggctgggcca cagctgcact tttgattaca ttctgcttcg 1520
gctgggtatt gattcctgca tgtacattag ctattctttt 1560
agtccttaag ttctttgcaa atatccttca tacaagcaat 1600
caagagaacc gattcaaagc cattctacgg aaaataaagg 1640
aggagtttga aaaaacaaag ggttccatgg tttgtgagat 1680
ctgtaagtat gagtgtgaaa cattaaagga attgaaggca 1720
cataacctat catgtgttca aggagagtgc ccatattgct 1760
ttacccactg tgaaccgaca gaaactgcaa ttcaggcaca 1800
ttacaaagtt tgtcaagcca cccaccgatt cagagaagat 1840
ttaaaaaaga ctgtaactcc tcaaaatatt gggccaggct 1880
gttaccgaac actaaatctt tttaggtata aaagtaggtg 1920
 
ttatattctg acaatgtgga ctcttcttct cattattgaa 1960
tccatcctct gggcagcaag tgcagcagaa atcccccttg 2000
tccctctctg gacagataat gctcatggcg ttgggagtgt 2040
tcctatgcat acggatcttg aattagactt ctctttgcca 2080
tccagttcta agtacacata caaaagacat ctcacaaacc 2120
cagttaatga ccaacagagt gtctcattgc atatagaaat 2160
tgaaagtcaa ggcattggtg ctgctgttca tcatcttgga 2200
cattggtatg atgcaagatt gaatctaaaa acctcatttc 2240
attgttatgg tgcctgcaca aaatatcaat acccatggca 2280
cactgcaaaa tgccattttg agaaagatta tgagtatgaa 2320
aatagctggg cttgcaaccc cccagattgc ccaggggttg 2360
gtacaggttg tactgcttgt ggattatatc tagatcaatt 2400
gaagccggta ggaacagcct ttaaaattat aagtgtaaga 2440
tacagtagaa aagtgtgcgt gcagtttggt gaagaacacc 2480
tttgtaaaac aattgatatg aatgattgct ttgtgactag 2520
gcatgccaaa atatgtataa ttgggactgt atctaagttt 2560
tctcaaggtg acactctact atttctgggg cccatggaag 2600
gaggtggtat aatctttaaa cactggtgta catctacctg 2640
tcactttgga gaccctggtg atgtcatggg tccaaaagat 2680
aaaccattta tttgccctga atttccaggg caatttagga 2720
aaaaatgtaa ctttgccaca actccagttt gtgaatatga 2760
tggaaacatt atatcaggct ataagaaagt acttgcaaca 2800
attgattctt tccaatcatt taacacaagc aatatacact 2840
tcactgatga gagaattgaa tggagagacc ctgatggcat 2880
gcttcgggat catattaata ttgttatttc taaagatatt 2920
gattttgaaa atttggctga gaatccttgt aaagtagggc 2960
tccaggcagc aaacatagaa ggtgcctggg gttcaggtgt 3000
cgggtttaca ctcacatgca aggtgtctct cacagaatgc 3040
ccaacatttc ttacatcaat aaaggcctgt gacatggcaa 3080
tttgttatgg tgcagaaagt gtgacactct cacgaggaca 3120
aaatactgtc aaaattaccg ggaaaggtgg ccatagtggt 3160
tcttcattca aatgctgtca tgggaaagaa tgttcatcaa 3200
ctggcctcca agccagtgca ccacatctgg ataaggtaaa 3240
tggtatctct gagttagaaa acgagaaagt ttatgatgac 3280
ggtgcacctg aatgtggcat tacttgttgg tttaaaaaat 3320
caggtgaatg ggttatgggt ataatcaatg ggaactgggt 3360
tgtcctaatt gtcttgtgtg tactgctgct cttttctctt 3400
atcctgttga gcatcttgtg tcctgttaga aagcataaaa 3440
aatcataaat cccacctaac aatcttcaca tcatgtatcg 3480
attttcaaac actttatcat ttagaactta acttggcact 3520
actatctgat aactgacttt catttttatt tttatatgga 3560
ttaattacta aaaaaaatac tctcttctat ctcccaatct 3600
tttattgatt caccggggtg ctgtcttgac atctggcggc 3640
gtctactact a 3651
SEQ ID NO:2 SEOV S genome segment 
tagtagtaga ctccctaaag agctactaca ctaacaagaa 40
aaatggcaac tatggaagaa atccagagag aaatcactgc 80
tcacgagggg cagcttgtga tagcacgcca gaaggtcaag 120
gatgcagaaa agcagtatga gaaggatcct gatgacttaa 160
acaagagggc actgcatgat cgggagagtg tcgcagcttc 200
aatacaatca aaaattgatg aattgaagcg ccaacttgcc 240
 
gacagattgc agcagggaag aacatccggg caggaccggg 280
atcctacagg ggtagagcca ggtgatcatc ttaaggaaag 320
atcagcacta agctacggga atacactgga cctgaatagt 360
cttgacattg atgaacctac aggacagaca gctgattggc 400
tgaccataat tgtctatctg acatcattcg tggtcccgat 440
catcttgaag gcactgtaca tgttaacaac acgaggtagg 480
cagacttcaa aggacaacaa ggggatgagg atcagattca 520
aggatgacag ctcatatgag gatgtcaatg gaatcagaaa 560
gcccaaacat ctgtatgtgt caatgccaaa cgcccaatcc 600
agcatgaagg ctgaagagat aacaccagga agattccgca 640
ctgcagtatg tggactatat cctgcacaga taaaggcaag 680
gaatatggta agccctgtca tgagtgtagt tgggttcttg 720
gcactggcaa aagactggac atcgagaatt gaagaatggc 760
tcggtgcacc ctgcaaattc atggcggagt ctcttattgc 800
cgggagttta tctgggaatc ctgtgaatcg tgactatatc 840
agacagagac aaggtgcact tgcagggatg gagccaaagg 880
aatttcaagc cctcaggcaa cattcaaagg atgctggatg 920
tacactagtt gaacatattg agtcaccatc atcaatatgg 960
gtgtttgctg gggcccctga taggtgtcca ccaacatgct 1000
tgtttgtcgg agggatggct gaattaggtg ccttcttttc 1040
tatacttcag gatatgagga acacaatcat ggcttcaaaa 1080
actgtgggca cagctgatga aaagcttcga aagaaatcat 1120
cattctatca atcatacctc agacgcacac aatcaatggg 1160
aatacaactg gaccagagga taattgttat gtttatggtt 1200
gcctggggaa aggaggcagt ggacaacttt catctcggtg 1240
atgacatgga tccagagctt cgtagcctgg ctcagatctt 1280
gattgaccag aaagtgaagg aaatctcaaa ccaggaacct 1320
atgaaattat aagtacataa atatataatc aatactaact 1360
ataggttaag aaatactaat cattagttaa taagaatata 1400
gatttattga ataatcatat taaataatta ggtaagttaa 1440
ctagtattta gttaagttag ctaattgatt tatatgattg 1480
tcacaattaa atgtaatcat aagcacaatc actgccatgt 1520
ataatcacgg gtatacgggt ggttttcata tggggaacag 1560
ggtgggctta gggccaggtc accttaagtg accttttttt 1600
gtatatatgg atgtagattt caattgatcg aatactaatc 1640
ctactgtcct cttttctttt cctttctcct tctttactaa 1680
caacaacaaa ctacctcaca ccttaatata tactacttta 1720
ttaagttgtt aagttgtgtc tttttgggga gtaagggagt 1760
ctactacta 1769
SEQ ID NO:3 note="M genome segment from SEOV, strain SR-11, subcloned into DNA vector pWRG7077" 
gggggggggg ggcgctgagg tctgcctcgt gaagaaggtg 40
ttgctgactc ataccaggcc tgaatcgccc catcatccag 80
ccagaaagtg agggagccac ggttgatgag agctttgttg 120
taggtggacc agttggtgat tttgaacttt tgctttgcca 160
cggaacggtc tgcgttgtcg ggaagatgcg tgatctgatc 200
cttcaactca gcaaaagttc gatttattca acaaagccga 240
cgtcccgtca agtcagcgta atgctctgcc agtgttacaa 280
ccaattaacc aattctgatt agaaaaactc atcgagcatc 320
aaatgaaact gcaatttatt catatcagga ttatcaatac 360
 
catatttttg aaaaagccgt ttctgtaatg aaggagaaaa 400
ctcaccgagg cagttccata ggatggcaag atcctggtat 440
cggtctgcga ttccgactcg tccaacatca atacaaccta 480
ttaatttccc ctcgtcaaaa ataaggttat caagtgagaa 520
atcaccatga gtgacgactg aatccggtga gaatggcaaa 560
agcttatgca tttctttcca gacttgttca acaggccagc 600
cattacgctc gtcatcaaaa tcactcgcat caaccaaacc 640
gttattcatt cgtgattgcg cctgagcgag acgaaatacg 680
cgatcgctgt taaaaggaca attacaaaca ggaatcgaat 720
gcaaccggcg caggaacact gccagcgcat caacaatatt 760
ttcacctgaa tcaggatatt cttctaatac ctggaatgct 800
gttttcccgg ggatcgcagt ggtgagtaac catgcatcat 840
caggagtacg gataaaatgc ttgatggtcg gaagaggcat 880
aaattccgtc agccagttta gtctgaccat ctcatctgta 920
acatcattgg caacgctacc tttgccatgt ttcagaaaca 960
actctggcgc atcgggcttc ccatacaatc gatagattgt 1000
cgcacctgat tgcccgacat tatcgcgagc ccatttatac 1040
ccatataaat cagcatccat gttggaattt aatcgcggcc 1080
tcgagcaaga cgtttcccgt tgaatatggc tcataacacc 1120
ccttgtatta ctgtttatgt aagcagacag ttttattgtt 1160
catgatgata tatttttatc ttgtgcaatg taacatcaga 1200
gattttgaga cacaacgtgg ctttcccccc ccccccggca 1240
tgcctgcagg tcgacataaa tcaatattgg ctattggcca 1280
ttgcatacgt tgtatctata tcataatatg tacatttata 1320
ttggctcatg tccaatatga ccgccatgtt gacattgatt 1360
attgactagt tattaatagt aatcaattac ggggtcatta 1400
gttcatagcc catatatgga gttccgcgtt acataactta 1440
cggtaaatgg cccgcctcgt gaccgcccaa cgacccccgc 1480
ccattgacgt caataatgac gtatgttccc atagtaacgc 1520
caatagggac tttccattga cgtcaatggg tggagtattt 1560
acggtaaact gcccacttgg cagtacatca agtgtatcat 1600
atgccaagtc cggcccccta ttgacgtcaa tgacggtaaa 1640
tggcccgcct ggcattatgc ccagtacatg accttacggg 1680
actttcctac ttggcagtac atctacgtat tagtcatcgc 1720
tattaccatg gtgatgcggt tttggcagta caccaatggg 1760
cgtggatagc ggtttgactc acggggattt ccaagtctcc 1800
accccattga cgtcaatggg agtttgtttt ggcaccaaaa 1840
tcaacgggac tttccaaaat gtcgtaataa ccccgccccg 1880
ttgacgcaaa tgggcggtag gcgtgtacgg tgggaggtct 1920
atataagcag agctcgttta gtgaaccgtc agatcgcctg 1960
gagacgccat ccacgctgtt ttgacctcca tagaagacac 2000
cgggaccgat ccagcctccg cggccgggaa cggtgcattg 2040
gaacgcggat tccccgtgcc aagagtgacg taagtaccgc 2080
ctatagactc tataggcaca cccctttggc tcttatgcat 2120
gctatactgt ttttggcttg gggcctatac acccccgctc 2160
cttatgctat aggtgatggt atagcttagc ctataggtgt 2200
gggttattga ccattattga ccactcccct attggtgacg 2240
atactttcca ttactaatcc ataacatggc tctttgccac 2280
aactatctct attggctata tgccaatact ctgtccttca 2320
gagactgaca cggactctgt atttttacag gatggggtcc 2360
catttattat ttacaaattc acatatacaa caacgccgtc 2400
ccccgtgccc gcagttttta ttaaacatag cgtgggatct 2440
ccacgcgaat ctcgggtacg tgttccggac atgggctctt 2480
ctccggtagc ggcggagctt ccacatccga gccctggtcc 2520
 
catgcctcca gcggctcatg gtcgctcggc agctccttgc 2560
tcctaacagt ggaggccaga cttaggcaca gcacaatgcc 2600
caccaccacc agtgtgccgc acaaggccgt ggcggtaggg 2640
tatgtgtctg aaaatgagct cggagattgg gctcgcaccg 2680
tgacgcagat ggaagactta aggcagcggc agaagaagat 2720
gcaggcagct gagttgttgt attctgataa gagtcagagg 2760
taactcccgt tgcggtgctg ttaacggtgg agggcagtgt 2800
agtctgagca gtactcgttg ctgccgcgcg cgccaccaga 2840
cataatagct gacagactaa cagactgttc ctttccatgg 2880
gtcttttctg cagtcaccgt ccaagcttgc ggccgcggat 2920
ctgcaggaat tcggcacgag agtagtagac tccgcaagaa 2960
acagcagtta aagaacaata ggatcatgtg gagtttgcta 3000
ttactggccg ctttagttgg ccaaggcttt gcattaaaaa 3040
atgtatttga catgagaatt cagttgcccc actcagtcaa 3080
ctttggggaa acaagtgtgt caggctatac agaatttccc 3120
ccactctcat tacaggaggc agaacagcta gtgccagaga 3160
gctcatgcaa catggacaac caccagtcac tctcaacaat 3200
aaataaatta accaaggtca tatggcggaa aaaagcaaat 3240
caggaatcag caaaccagaa ttcatttgaa gttgtggaaa 3280
gtgaagtcag ctttaaaggg ttgtgtatgt taaagcatag 3320
aatggttgaa gaatcatata gaaataggag atcagtaatc 3360
tgttatgatc tagcctgtaa tagtacattc tgtaaaccaa 3400
ctgtttatat gattgttcct atacatgctt gcaacatgat 3440
gaaaagctgt ttgattggcc ttggccccta cagaatccag 3480
gttgtctatg aaaggacata ctgcactacg ggtatattga 3520
cagaaggaaa atgctttgtc cctgacaagg ctgttgtcag 3560
tgcattgaaa agaggcatgt atgctatagc aagcatagag 3600
acaatctgct tttttattca tcagaaaggg aatacatata 3640
agatagtgac tgccattaca tcagcaatgg gctccaaatg 3680
taataataca gatactaaag ttcaaggata ttatatctgt 3720
attattggtg gaaactccgc ccctgtatat gcccctgctg 3760
gtgaagactt cagagcaatg gaggtttttt ctgggattat 3800
tacatcacca catggagaag accatgacct acccggcgaa 3840
gaaatcgcaa cgtaccagat ttcagggcag atagaggcaa 3880
aaatccctca tacagtgagc tccaaaaact taaaattgac 3920
tgcttttgca ggtattccat catactcatc aactagtata 3960
ttggctgctt cagaagatgg tcgtttcata tttagtcctg 4000
gtttatttcc taacctaaat cagtcagtct gtgacaacaa 4040
tgcactccct ttaatctgga ggggcctaat tgatttaacg 4080
ggatactatg aggcagtcca cccttgcaat gtgttctgtg 4120
tcttatcagg accaggtgct tcatgtgagg ccttttcaga 4160
aggaggtatt ttcaatatta cttctccaat gtgtctggtg 4200
tctaagcaaa atagatttag agcagctgag cagcagatta 4240
gctttgtctg ccaaagagtt gatatggata ttatagtgta 4280
ctgtaatggt cagaaaaaaa caatcctaac aaaaacatta 4320
gttataggcc aatgtattta tactattaca agtctctttt 4360
cactgttacc aggggttgcc cattctattg ctattgagtt 4400
gtgtgttcca gggtttcatg gctgggccac agctgcactt 4440
ttgattacat tctgcttcgg ctgggtattg attcctgcat 4480
gtacattagc tattctttta gtccttaagt tctttgcaaa 4520
tatccttcat acaagcaatc aagagaaccg attcaaagcc 4560
attctacgga aaataaagga ggagtttgaa aaaacaaagg 4600
gttccatggt ttgtgagatc tgtaagtatg agtgtgaaac 4640
attaaaggaa ttgaaggcac ataacctatc atgtgttcaa 4680
 
ggagagtgcc catattgctt tacccactgt gaaccgacag 4720
aaactgcaat tcaggcacat tacaaagttt gtcaagccac 4760
ccaccgattc agagaagatt taaaaaagac tgtaactcct 4800
caaaatattg ggccaggctg ttaccgaaca ctaaatcttt 4840
ttaggtataa aagtaggtgt tatattctga caatgtggac 4880
tcttcttctc attattgaat ccatcctctg ggcagcaagt 4920
gcagcagaaa tcccccttgt ccctctctgg acagataatg 4960
ctcatggcgt tgggagtgtt cctatgcata cggatcttga 5000
attagacttc tctttgccat ccagttctaa gtacacatac 5040
aaaagacatc tcacaaaccc agttaatgac caacagagtg 5080
tctcattgca tatagaaatt gaaagtcaag gcattggtgc 5120
tgctgttcat catcttggac attggtatga tgcaagattg 5160
aatctaaaaa cctcatttca ttgttatggt gcctgcacaa 5200
aatatcaata cccatggcac actgcaaaat gccattttga 5240
gaaagattat gagtatgaaa atagctgggc ttgcaacccc 5280
ccagattgcc caggggttgg tacaggttgt actgcttgtg 5320
gattatatct agatcaattg aagccggtag gaacagcctt 5360
taaaattata agtgtaagat acagtagaaa agtgtgcgtg 5400
cagtttggtg aagaacacct ttgtaaaaca attgatatga 5440
atgattgctt tgtgactagg catgccaaaa tatgtataat 5480
tgggactgta tctaagtttt ctcaaggtga cactctacta 5520
tttctggggc ccatggaagg aggtggtata atctttaaac 5560
actggtgtac atctacctgt cactttggag accctggtga 5600
tgtcatgggt ccaaaagata aaccatttat ttgccctgaa 5640
ttcccagggc aatttaggaa aaaatgtaac tttgccacaa 5680
ctccagtttg tgaatatgat ggaaacatta tatcaggcta 5720
taagaaagta cttgcaacaa ttgattcttt ccaatcattt 5760
aacacaagca atatacactt cactgatgag agaattgaat 5800
ggagagaccc tgatggcatg cttcgggatc atattaatat 5840
tgttatttct aaagatattg attttgaaaa tttggctgag 5880
aatccttgta aagtagggct ccaggcagca aacatagaag 5920
gtgcctgggg ttcaggtgtc gggtttacac tcacatgcaa 5960
ggtgtctctc acagaatgcc caacatttct tacatcaata 6000
aaggcctgtg acatggcaat ttgttatggt gcagaaagtg 6040
tgacactctc acgaggacaa aatactgtca aaattaccgg 6080
gaaaggtggc catagtggtt cttcattcaa atgctgtcat 6120
gggaaagaat gttcatcaac tggcctccaa gccagtgcac 6160
cacatctgga taaggtaaat ggtatctctg agttagaaaa 6200
cgagaaagtt tatgatgacg gtgcacctga atgtggcatt 6240
acttgttggt ttaaaaaatc aggtgaatgg gttatgggta 6280
taatcaatgg gaactgggtt gtcctaattg tcttgtgtgt 6320
actgctgctc ttttctctta tcctgttgag catcttgtgt 6360
cctgttagaa agcataaaaa atcataaatc ccacctaaca 6400
atcttcacat catgtatcga ttttcaaaca ctttatcatt 6440
tagaacttaa cttggcacta ctatctgata actgactttc 6480
atttttattt ttatatggat taattactaa aaaaaatact 6520
ctctcgtgcc gaattcgata tcaagcttat cgataccgtc 6560
gacctcgagg gggggcccgg tacccgggat cctcgcaatc 6600
cctaggagga ttaggcaagg gcttgagctc acgctcttgt 6640
gagggacaga aatacaatca ggggcagtat atgaatactc 6680
catggagaaa cccagatcta cgtatgatca gcctcgactg 6720
tgccttctag ttgccagcca tctgttgttt gcccctcccc 6760
cgtgccttcc ttgaccctgg aaggtgccac tcccactgtc 6800
ctttcctaat aaaatgagga aattgcatcg cattgtctga 6840
 
gtaggtgtca ttctattctg gggggtgggg tggggcagga 6880
cagcaagggg gaggattggg aagacaatag caggcatgct 6920
ggggatgcgg tgggctctat ggcttctgag gcggaaagaa 6960
ccagctgggg ctcgacagct cgactctaga attgcttcct 7000
cgctcactga ctcgctgcgc tcggtcgttc ggctgcggcg 7040
agcggtatca gctcactcaa aggcggtaat acggttatcc 7080
acagaatcag gggataacgc aggaaagaac atgtgagcaa 7120
aaggccagca aaaggccagg aaccgtaaaa aggccgcgtt 7160
gctggcgttt ttccataggc tccgcccccc tgacgagcat 7200
cacaaaaatc gacgctcaag tcagaggtgg cgaaacccga 7240
caggactata aagataccag gcgtttcccc ctggaagctc 7280
cctcgtgcgc tctcctgttc cgaccctgcc gcttaccgga 7320
tacctgtccg cctttctccc ttcgggaagc gtggcgcttt 7360
ctcaatgctc acgctgtagg tatctcagtt cggtgtaggt 7400
cgttcgctcc aagctgggct gtgtgcacga accccccgtt 7440
cagcccgacc gctgcgcctt atccggtaac tatcgtcttg 7480
agtccaaccc ggtaagacac gacttatcgc cactggcagc 7520
agccactggt aacaggatta gcagagcgag gtatgtaggc 7560
ggtgctacag agttcttgaa gtggtggcct aactacggct 7600
acactagaag gacagtattt ggtatctgcg ctctgctgaa 7640
gccagttacc ttcggaaaaa gagttggtag ctcttgatcc 7680
ggcaaacaaa ccaccgctgg tagcggtggt ttttttgttt 7720
gcaagcagca gattacgcgc agaaaaaaag gatctcaaga 7760
agatcctttg atcttttcta cggggtctga cgctcagtgg 7800
aacgaaaact cacgttaagg gattttggtc atcagattat 7840
caaaaaggat cttcacctag atccttttaa attaaaaatg 7880
aagttttaaa tcaatctaaa gtatatatga gtaaacttgg 7920
tctgacagtt accaatgctt aatcagtgag gcacctatct 7960
cagcgatctg tctatttcgt tcatccatag ttgcctgact 8000
c 8001
SEQ ID NO:4
   S genome segment from SEOV, strain SR-11, subcloned into DNA vector pWRG7077 
gggggggggg ggcgctgagg tctgcctcgt gaagaaggtg 40
ttgctgactc ataccaggcc tgaatcgccc catcatccag 80
ccagaaagtg agggagccac ggttgatgag agctttgttg 120
taggtggacc agttggtgat tttgaacttt tgctttgcca 160
cggaacggtc tgcgttgtcg ggaagatgcg tgatctgatc 200
cttcaactca gcaaaagttc gatttattca acaaagccgc 240
cgtcccgtca agtcagcgta atgctctgcc agtgttacaa 280
ccaattaacc aattctgatt agaaaaactc atcgagcatc 320
aaatgaaact gcaatttatt catatcagga ttatcaatac 360
catatttttg aaaaagccgt ttctgtaatg aaggagaaaa 400
ctcaccgagg cagttccata ggatggcaag atcctggtat 440
cggtctgcga ttccgactcg tccaacatca atacaaccta 480
ttaatttccc ctcgtcaaaa ataaggttat caagtgagaa 520
atcaccatga gtgacgactg aatccggtga gaatggcaaa 560
agcttatgca tttctttcca gacttgttca acaggccagc 600
cattacgctc gtcatcaaaa tcactcgcat caaccaaacc 640
gttattcatt cgtgattgcg cctgagcgag acgaaatacg 680
 
cgatcgctgt taaaaggaca attacaaaca ggaatcgaat 720
gcaaccggcg caggaacact gccagcgcat caacaatatt 760
ttcacctgaa tcaggatatt cttctaatac ctggaatgct 800
gttttcccgg ggatcgcagt ggtgagtaac catgcatcat 840
caggagtacg gataaaatgc ttgatggtcg gaagaggcat 880
aaat[?]ccgtc agccagttta gtctgaccat ctcatctgta 920
acatcattgg caacgctacc tttgccatgt ttcagaaaca 960
actctggcgc atcgggcttc ccatacaatc gatagattgt 1000
cgcacctgat tgcccgacat tatcgcgagc ccatttatac 1040
ccatataaat cagcatccat gttggaattt aatcgcggcc 1080
tcgagcaaga cgtttcccgt tgaatatggc tcataacacc 1120
ccttgtatta ctgtttatgt aagcagacag ttttattgtt 1160
catgatgata tatttttatc ttgtgcaatg taacatcaga 1200
gattttgaga cacaacgtgg ctttcccccc ccccccggca 1240
tgcctgcagg tcgacataaa tcaatattgg ctattggcca 1280
ttgcatacgt tgtatctata tcataatatg tacatttata 1320
ttggctcatg tccaatatga ccgccatgtt gacattgatt 1360
attgactagt tattaatagt aatcaattac ggggtcatta 1400
gttcatagcc catatatgga gttccgcgtt acataactta 1440
cggtaaatgg cccgcctcgt gaccgcccaa cgacccccgc 1480
ccattgacgt caataatgac gtatgttccc atagtaacgc 1520
caatagggac tttccattga cgtcaatggg tggagtattt 1560
acggtaaact gcccacttgg cagtacatca agtgtatcat 1600
atgccaagtc cggcccccta ttgacgtcaa tgacggtaaa 1640
tggcccgcct ggcattatgc ccagtacatg accttacggg 1680
actttcctac ttggcagtac atctacgtat tagtcatcgc 1720
tattaccatg gtgatgcggt tttggcagta caccaatggg 1760
cgtggatagc ggtttgactc acggggattt ccaagtctcc 1800
accccattga cgtcaatggg agtttgtttt ggcaccaaaa 1840
tcaacgggac tttccaaaat gtcgtaataa ccccgccccg 1880
ttgacgcaaa tgggcggtag gcgtctacgg tgggaggtct 1920
atataagcag agctcgttta gtgaaccgtc agatcgcctg 1960
gagacgccat ccacgctgtt ttgacctcca tagaagacac 2000
cgggaccgat ccagcctccg cggccgggaa cggtgcattg 2040
gaacgcggat tccccgtgcc aagagtgacgt aagtaccgc 2080
ctatagactc tataggcaca cccctttggc tcttatgcat 2120
gctatactgt ttttggcttg gggcctatac acccccgctc 2160
cttatgctat aggtgatggt atagcttagc ctataggtgt 2200
gggttattga ccattattga ccactcccct attggtgacg 2240
atactttcca ttactaatcc ataacatggc tctttgccac 2280
aactatctct attggctata tgccaatact ctgtccttca 2320
gagactgaca cggactctgt atttttacag gatggggtcc 2360
catttattat ttacaaattc acatatacaa caacgccgtc 2400
ccccgtgccc gcagttttta ttaaacatag cgtgggatct 2440
ccacgcgaat ctcgggtacg tgttccggac atgggctctt 2480
ctccggtagc ggcggagctt ccacatccga gccctggtcc 2520
catgcctcca gcggctcatg gtcgctcggc agctccttgc 2560
tcctaacagt ggaggccaga cttaggcaca gcacaatgcc 2600
caccaccacc agtgtgccgc acaaggccgt ggcggtaggg 2640
tatgtgtctg aaaatgagct cggagattgg gctcgcaccg 2680
tgacgcagat ggaagactta aggcagcggc agaagaagat 2720
gcaggcagct gagttgttgt attctgataa gagtcagagg 2760
taactcccgt tgcggtgctg ttaacggtgg agggcagtgt 2800
agtctgagca gtactcgttg ctgccgcgcg cgccaccaga 2840
 
cataatagct gacagactaa cagactgttc ctttccatgg 2880
gtcttttctg cagtcaccgt ccaagcttgc ggccaattcg 2920
gcacgagaga gtagtagact ccctaaagag ctactacact 2960
aacaagaaaa atggcaacta tggaagaaat ccagagagaa 3000
atcagtgctc acgaggggca gcttgtgata gcacgccaga 3040
aggtcaagga tgcagaaaag cagtatgaga aggatcctga 3080
tgacttaaac aagagggcac tgcatgatcg ggagagtgtc 3120
gcagcttcaa tacaatcaaa aattgatgaa ttgaagcgcc 3160
aacttgccga cagattgcag cagggaagaa catccgggca 3200
ggaccgggat cctacagggg tagagccagg tgatcatctt 3240
aaggaaagat cagcactaag ctacgggaat acactggacc 3280
tgaatagtct tgacattgat gaacctacag gacagacagc 3320
tgat[?]ggctg accataattg tctatctgac atcattcgtg 3360
gtcccgatca tcttgaaggc actgtacatg ttaacaacac 3400
gaggtaggca gacttcaaag gacaacaagg ggatgaggat 3440
cagattcaag gatgacagct catatgagga tgtcaatgga 3480
atcagaaagc ccaaacatct gtatgtgtca atgccaaacg 3520
cccaatccag catgaaggct gaagagataa caccaggaag 3560
attccgcact gcagtatgtg gactatatcc tgcacagata 3600
aaggcaagga atatggtaag ccctgtcatg agtgtagttg 3640
ggttcttggc actggcaaaa gactggacat cgagaattga 3680
agaatggctc ggtgcaccct gcaaattcat ggcggagtct 3720
cttattgccg ggagtttatc tgggaatcct gtgaatcgtg 3760
actatatcag acagagacaa ggtgcacttg cagggatgga 3800
gccaaaggaa tttcaagccc tcaggcaaca ttcaaaggat 3840
gctggatgta cactagttga acatattgag tcaccatcat 3880
caatatgggt gtttgctggg gcccctgata ggtgtccacc 3920
aacatgcttg tttgtcggag ggatggctga attaggtgcc 3960
ttcttttcta tacttcagga tatgaggaac acaatcatgg 4000
cttcaaaaac tgtgggcaca gctgatgaaa agcttcgaaa 4040
gaaatcatca ttctatcaat catacctcag acgcacacaa 4080
tcaatgggaa tacaactgga ccagaggata attgttatgt 4120
ttatggttgc ctggggaaag gaggcagtgg acaactttca 4160
tctcggtgat gacatggatc cagagcttcg tagcctggct 4200
caga[?]cttga ttgaccagaa agtgaaggaa atctcaaacc 4240
aggaacctat gaaattataa gtacataaat atataatcaa 4280
tactaactat aggttaagaa atactaatca ttagttaata 4320
agaatataga tttattgaat aatcatatta aataattagg 4360
taagttaact agtatttagt taagttagct aattgattta 4400
tatgattgtc acaattaaat gtaatcataa gcacaatcac 4440
tgccatgtat aatcacgggt atacgggtgg ttttcatatg 4480
gggaacaggg tgggcttagg gccaggtcac cttaagtgac 4520
ctttttttgt atatatggat gtagatttca attgatcgaa 4560
tactaatcct actgtcctct tttcttttcc tttctccttc 4600
tttactaaca aactacctcg tgccgaattg gccgcggatc 4640
ctcgcaatcc ctaggaggat taggcaaggg cttgagctca 4680
cgctcttgtg agggacagaa atacaatcag gggcagtata 4720
tgaatactcc atggagaaac ccagatctac gtatgatcag 4760
cctcgactgt gccttctagt tgccagccat ctgttgtttg 4800
cccctccccc gtgccttcct tgaccctgga aggtgccact 4840
cccactgtcc tttcctaata aaatgaggaa attgcatcgc 4880
attgtctgag taggtgtcat tctattctgg ggggtggggt 4920
ggggcaggac agcaaggggg aggattggga agacaatagc 4960
aggcatgctg gggatgcggt gggctctatg gcttctgagg 5000
 
cggaaagaac cagctggggc tcgacagctc gactctagaa 5040
ttgcttcctc gctcactgac tcgctgcgct cggtcgttcg 5080
gctgcggcga gcggtatcag ctcactcaaa ggcggtaata 5120
cggttatcca cagaatcagg ggataacgca ggaaagaaca 5160
tgtgagcaaa aggccagcaa aaggccagga accgtaaaaa 5200
ggccgcgttg ctggcgtttt tccataggct ccgcccccct 5240
gacgagcatc acaaaaatcg acgctcaagt cagaggtggc 5280
gaaacccgac aggactataa agataccagg cgtttccccc 5320
tggaagctcc ctcgtgcgct ctcctgttcc gaccctgccg 5360
cttaccggat acctgtccgc ctttctccct tcgggaagcg 5400
tggcgctttc tcaatgctca cgctgtaggt atctcagttc 5440
ggtgtaggtc gttcgctcca agctgggctg tgtgcacgaa 5480
ccccccgttc agcccgaccg ctgcgcctta tccggtaact 5520
atcgtcttga gtccaacccg gtaagacacg acttatcgcc 5560
actggcagca gccactggta acaggattag cagagcgagg 5600
tatgtaggcg gtgctacaga gttcttgaag tggtggccta 5640
actacggcta cactagaagg acagtatttg gtatctgcgc 5680
tctgctgaag ccagttacct tcggaaaaag agttggtagc 5720
tcttgatccg gcaaacaaac caccgctggt agcggtggtt 5760
tttttgtttg caagcagcag attacgcgca gaaaaaaagg 5800
atctcaagaa gatcctttga tcttttctac ggggtctgac 5840
gctcagtgga acgaaaactc acgttaaggg attttggtca 5880
tgagattatc aaaaaggatc ttcacctaga tccttttaaa 5920
ttaaaaatga agttttaaat caatctaaag tatatatgag 5960
taaacttggt ctgacagtta ccaatgctta atcagtgagg 6000
cacctatctc agcgatctgt ctatttcgtt catccatagt 6040
tgcctgactc 6050
SEQ ID NO:5
   Sequence from plasmid pWRG/SEO-M in Figure 9A 
gcggccgc ggatctgcaggaattcggcacgag
agtagtagactccgcaagaaacagcagttaaagaacaataggatcatg
SEQ ID NO:6
   Sequence of 24 bp extraneous sequence
   ggatctgcaggaattcggcacgag
SEQ ID NO:7
   pWRG/HTN-M(x) sequence 
GGGGGGGGGGGCGCTGAGGTCTGCCTCGTGAAGAAGGTGTTGCTGACTCATACCAGGCCTGAATCGCCCCATCATCCAG
CCAGAAAGTGAGGGAGCCACGGTTGATGAGAGCTTTGTTGTAGGTGGACCAGTTGGTGATTTTGAACTTTTGCTTTGCCA
CGGAACGGTCTGCGTTGTCGGGAAGATGCGTGATCTGATCCTTCAACTCAGCAAAAGTTCGATTTATTCAACAAAGCCGC
CGTCCCGTCAAGTCAGCGTAATGCTCTGCCAGTGTTACAACCAATTAACCAATTCTGATTAGAAAAACTCATCGAGCATC
AAATGAAACTGCAATTTATTCATATCAGGATTATCAATACCATATTTTTGAAAAAGCCGTTTCTGTAATGAAGGAGAAAA
CTCACCGAGGCAGTTCCATAGGATGGCAAGATCCTGGTATCGGTCTGCGATTCCGACTCGTCCAACATCAATACAACCTA
TTAATTTCCCCTCGTCAAAAATAAGGTTATCAAGTGAGAAATCACCATGAGTGACGACTGAATCCGGTGAGAATGGCAAA
AGCTTATGCATTTCTTTCCAGACTTGTTCAACAGGCCAGCCATTACGCTCGTCATCAAAATCACTCGCATCAACCAAACC
GTTATTCATTCGTGATTGCGCCTGAGCGAGACGAAATACGCGATCGCTGTTAAAAGGACAATTACAAACAGGAATCGAAT
GCAACCGGCGCAGGAACACTGCCAGCGCATCAACAATATTTTCACCTGAATCAGGATATTCTTCTAATACCTGGAATGCT
GTTTTCCCGGGGATCGCAGTGGTGAGTAACCATGCATCATCAGGAGTACGGATAAAATGCTTGATGGTCGGAAGAGGCAT
 
AAATTCCGTCAGCCAGTTTAGTCTGACCATCTCATCTGTAACATCATTGGCAACGCTACCTTTGCCATGTTTCAGAAACA
ACTCTGGCGCATCGGGCTTCCCATACAATCGATAGATTGTCGCACCTGATTGCCCGACATTATCGCGAGCCCATTTATAC
CCATATAAATCAGCATCCATGTTGGAATTTAATCGCGGCCTCGAGCAAGACGTTTCCCGTTGAATATGGCTCATAACACC
CCTTGTATTACTGTTTATGTAAGCAGACAGTTTTATTGTTCATGATGATATATTTTTATCTTGTGCAATGTAACATCAGA
GATTTTGAGACACAACGTGGCTTTCCCCCCCCCCCCGGCATGCCTGCAGGTCGACAATATTGGCTATTGGCCATTGCATA
CGTTGTATCTATATCATAATATGTACATTTATATTGGCTCATGTCCAATATGACCGCCATGTTGACATTGATTATTGACT
AGTTATTAATAGTAATCAATTACGGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTACATCAAGTGTATCATATGCCA
AGTCCGCCCCCTATTGACGTCAATGACGGTAAATGGCCCGCCTGGCATTATGCCCAGTACATGACCTTACGGGACTTTCC
TACTTGGCAGTACATCTACGTATTAGTCATCGCTATTACCATGGTGATGCGGTTTTGGCAGTACACCAATGGGCGTGGAT
AGCGGT[?]TGACTCACGGGGATTTCCAAGTCTCCACCCCATTGACGTCAATGGGAGTTTGTTTTGGCACCAAAATCAACGG
GACTTTCCAAAATGTCGTAATAACCCCGCCCCGTTGACGCAAATGGGCGGTAGGCGTGTACGGTGGGAGGTCTATATAAG
CAGAGCTCGTTTAGTGAACCGTCAGATCGCCTGGAGACGCCATCCACGCTGTTTTGACCTCCATAGAAGACACCGGGACC
GATCCAGCCTCCGCGGCCGGGAA[?]GGTGCATTGGAACGCGGATTCCCCGTGCCAAGAGTGACGTAAGTACCGCCTATAGA
CTCTATAGGCACACCCCTTTGGCTCTTATGCATGCTATACTGTTTTTGGCTTGGGGCCTATACACCCCCGCTTCCTTATG
CTATAGGTGATGGTATAGCTTAGCCTATAGGTGTGGGTTATTGACCATTATTGACCACTCCCCTATTGGTGACGATACTT
TCCATTACTAATCCATAACATGGCTCTTTGCCACAACTATCTCTATTGGCTATATGCCAATACTCTGTCCTTCAGAGACT
GACACGGACTCTGTATTTTTACAGGATGGGGTCCCATTTATTATTTACAAATTCACATATACAACAACGCCGTCCCCCGT
GCCCGCAGTTTTTATTAAACATAGCGTGGGATCTCCACGCGAATCTCGGGTACGTGTTCCGGACATGGGCTCTTCTCCGG
TAGCGGCGGAGCTTCCACATCCGAGCCCTGGTCCCATGCCTCCAGCGGCTCATGGTCGCTCGGCAGCTCCTTGCTCCTAA
CAGTGGAGGCCAGACTTAGGCACAGCACAATGCCCACCACCACCAGTGTGCCGCACAAGGCCGTGGCGGTAGGGTATGTG
TCTGAAAATGAGCTCGGAGATTGGGCTCGCACCGCTGACGCAGATGGAAGACTTAAGGCAGCGGCAGAAGAAGATGCAGG
CAGCTGAGTTGTTGTATTCTGATAAGAGTCAGAGGTAACTCCCGTTGCGGTGCTGTTAACGGTGGAGGGCAGTGTAGTCT
GAGCAGTACTCGTTGCTGCCGCGCGCGCCACCAGACATAATAGCTGACAGACTAACAGACTGTTCCTTTCCATGGGTCTT
TTCTGCAGTCACCGTCCAAGCTTGCGGCCGCGGATCTGCAGGAATTCGGCACGAGAGTAGTAGACTCCGCAAGAAACAGC
AGTCAATCAGCAACATGGGGATATGGAAGTGGCTAGTGATGGCCAGTTTAGTATGGCCTGTTTTGACACTGAGAAATGTC
TATGACATGAAAATTGAGTGCCCCCATACAGTAAGTTTTGGGGAAAACAGTGTGATAGGTTATGTAGAATTACCCCCCGT
GCCATTGGCCGACACAGCACAGATGGTGCCTGAGAGTTCTTGTAGCATGGATAATCACCAATCGTTGAATACAATAACAA
AATATACCCAAGTAAGTTGGAGAGGAAAGGCTGATCAGTCACAGTCTAGTCAAAATTCATTTGAGACAGTGTCCACTGAA
GTTGACTTGAAAGGAACATGTGCTCTAAAACACAAAATGGTGGAAGAATCATACCGTAGTAGGAAATCAGTAACCTGTTA
CGACCTGTCTTGCAATAGCACTTACTGCAAGCCAACACTATACATGATTGTACCAATTCATGCATGCAATATGATGAAAA
GCTGTTTGATTGCATTGGGACCATACAGAGTACAGGTGGTTTATGAGAGATCTTATTGCATGACAGGAGTCCTGATTGAA
GGGAAATGCTTTGTCCCAGATCAAAGTGTGGTCAGTATTATCAAGCATGGGATCTTTGATATTGCAAGTGTTCATATTGT
ATGTTTCTTTGTTGCAGTTAAAGGGAATACTTATAAAATTTTTGAACAGGTTAAGAAATCCTTTGAATCAACATGCAATG
ATACAGAGAATAAAGTGCAAGGATATTATATTTGTATTGTAGGGGGAAACTCTGCACCAATATATGTTCCAACACTTGAT
GATTTCAGATCCATGGAAGCATTTACAGGAATCTTCAGATCACCACATGGGGAAGATCATGATCTGGCTGGAGAAGAAAT
TGCATCTTATTCTATAGTCGGACCTGCCAATGCAAAAGTTCCTCATAGTGCTAGCTCAGATACATTGAGCTTGATTGCCT
ATTCAGGTATACCATCTTATTCTTCCCTTAGCATCCTAACAAGTTCAACAGAAGCTAAGCATGTATTCAGCCCTGGGTTG
TTCCCAAAACTTAATCACACAAATTGTGATAAAAGTGCCATACCACTCATATGGACTGGGATGATTGATTTACCTGGATA
CTACGAAGCTGTCCACCCTTGTACAGTTTTTTGCGTATTATCAGGTCCTGGGGCATCATGTGAAGCCTTTTCTGAAGGCG
GGATTTTCAACATAACCTCTCCCATGTGCTTAGTGTCAAAACAAAATCGATTCCGGTTAACAGAACAGCAAGTGAATTTT
GTGTGTCAGCGAGTGGACATGGACATTGTTGTGTACTGCAACGGGCAGAGGAAAGTAATATTAACAAAAACTCTAGTTAT
TGGACAGTGTATATATACTATAACAAGCTTATTCTCATTACTACCTGGAGTAGCACATTCTATTGCTGTTGAATTGTGTG
TACCTGGGTTCCATGGTTGGGCCACAGCTGCTCTGCTTGTTACATTCTGTTTCGGATGGGTTCTTATACCAGCAATTACA
TTTATCATACTAACAGTCCTAAAGTTCATTGCTAATATTTTTCACACAAGTAATCAAGAGAATAGGCTAAAATCAGTACT
TAGAAAGATAAAGGAAGAGTTTGAAAAAACAAAAGGCTCAATGGTATGTGATGTCTGCAAGTATGAGTGTGAAACCTATA
AAGAATTAAAGGCACACGGGGTATCATGCCCCCAATCTCAATGTCCTTACTGTTTTACTCATTGTGAACCCACAGAAGCA
GCATTCCAAGCTCATTACAAGGTATGCCAAGTTACTCACAGATTCAGGGATGATCTAAAGAAAACTGTTACTCCTCAAAA
TTTTACACCAGGATGTTACCGGACACTAAATTTATTTAGATACAAAAGCAGGTGCTACATCTTTACAATGTGGATATTTC
TTCTTGTCTTAGAATCCATACTGTGGGCTGCAAGTGCATCAGAGACACCATTAACTCCTGTCTGGAATGACAATGCCCAT
GGGGTAGGTTCTGTTCCTATGCATACAGATTTAGAGCTTGATTTCTCTTTAACATCCAGTTCCAAGTATACATACCGTAG
GAAGTTAACAAACCCACTTGAGGAAGCACAATCCATTGACCTACATATTGAAATAGAAGAACAGACAATTGGTGTTGATG
TGCATGCTCTAGGACACTGGTTTGATGGTCGTCTTAACCTTAAAACATCCTTTCACTGTTATGGTGCTTGTACAAAGTAT
GAATACCCTTGGCATACTGCAAAGTGCCACTATGAAAGAGATTACCAATATGAGACGAGCTGGGGTTGTAATCCATCAGA
TTGTCCTGGGGTGGGCACAGGCTGTACAGCATGTGGTTTATACCTAGATCAACTGAAACCAGTTGGTAGTGCTTATAAAA
TTATCACAATAAGGTACAGCAGGAGAGTCTGTGTTCAGTTTGGGGAGGAAAACCTTTGTAAGATAATAGACATGAATGAT
TGTTTTGTATCTAGGCATGTTAAGGTCTGCATAATTGGTACAGTATCTAAATTCTCTCAGGGTGATACCTTATTGTTTTT
 
TGGACCGCTTGAAGGTGGTGGTCTAATATTTAAACACTGGTGTACATCCACATGTCAATTTGGTGACCCAGGAGATATCA
TGAGTCCAAGAGACAAAGGTTTTTATGCCCTGAGTTTCCAGGTAGTTTCAGGAAGAAATGCAACTTTGCTACTACCCCT
ATTTGTGAGTATGATGGAAATATGGTCTCAGGTTACAAGAAAGTGATGGCGACAATTGATTCCTTCCAATCTTTTAATAC
AAGCACTATGCACTTCACTGATGAAAGGATAGAGTGGAAAGACCCTGATGGAATGCTAAGGACCATATAAACATTTTAG
TAACGAAGGACATTGACTTTGATAACCTTGGTGAAAATCCTTGCAAAATTGGCCTACAAACATCTTCTATTGAGGGGGCC
TGGGGTTCTGGTGTGGGGTTCACATTAACATGTCTGGTATCACTAACAGAATGTCCTACCTTTTTGACCTCAATAAAGGC
TTGTGATAAGGCTATCTGTTATGGTGCAGAGAGTGTAACATTGACAAGAGGACAAAATACAGTCAAGGTATCAGGGAAAG
GTGGCCATAGTGGTTCAACATTTAGGTGTTGCCATGGGAGGACTGTTCACAAATTGGACTCCATGCTGCTGCACCTCAC
CTTGACAAGGTAAATGGGATTTCTGAGATAGAAAATAGTAAAGTATATGATGATGGGCACCGCAATGTGGATAAAATG
TTGGTTTGTTAAATCAGGGGAATGGATTTCAGGGATATTCAGTGGTAATTGGATTGTACTCATTGTCCTCTGTGTATTTC
TATTGTTCTCCTTGGTTTTACTAAGCATTCTCTGTCCCGTAAGGAAGCATAAAAAATCATAGCTAAATTCTGTGACTATC
CTGTTCTTATGTATAGCTTTAACATATATACTAATTTTTATATTCCAGTATACTCTATCTAACACACTAAAAAAAATAGT
AGCTTTCTAACCACAAAACGGATCTACGTATGATCAGCCTCGACTGTGCCTTCTAGTTGCCAGCCATCTGTTGTTTGCCC
CTCCCCCGTGCCTTCCTTGACCCTGGAAGGTGCCACTCCCACTGTCCTTTCCTAATAAAATGAGGAAATTGCATCGCATT
GTCTGAGTAGGTGTCATTCTATTCTGGGGGGTGGGGTGGGGCAGGACAGCAAGGGGGAGGATTGGGAAGACAATAGCAGG
CATGCTGGGGATGCGGTGGGCTCTATGGCTTCTGAGGCGGAAAGAACCAGCTGGGGCTCGACAGCTCGACTCTAGAATTG
CTTCCTCGCTCACTGACTCGCTGCGCTCGGTCGTTCGGCTGCGGCGAGCGGTATCAGCTCACTCAAAGGCGGTAATACGG
TTATCCACAGAATCAGGGGATAACGCAGGAAAGAACATGTGAGCAAAAGGCCAGCAAAAGGCCAGGAACCGTAAAAAGGC
CGCGTT[?]CTGGCGTTTTTCCATAGGCTCCGCCCCCCTGACGAGCATCACAAAAATCGACGCTCAAGTCAGAGGTGGCGAA
ACCCGACAGGACTATAAAGATACCAGGCGTTTCCCCCTGGAAGCTCCCTCGTGCGCTCTCCTGTTCCGACCCTGCCGCTT
ACCGGATACCTGTCCGCCTTTCTCCCTTCGGGAAGCGTGGCGCTTTCTCATAGCTCACGCTGTAGGTATCTCAGTTCGGT
GTAGGTCGTTCGCTCCAAGCTGGGCTGTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACTGGTAACAGGATTAGCAGAGCGAGGTAT
GTAGGCGGTGCTACAGAGTTCTTGAAGTGGTGGCCTAACTACGGCTACACTAGAAGAACAGTATTTGGTATCTGCGCTCT
GCTGAAGCCAGTTACCTTCGGAAAAAGAGTTGGTAGCTCTTGATCCGGCAAACAAACCACCGCTGGTAGCGGTGGTTTTT
TTGTTTGCAAGCAGCAGATTACGCGCAGAAAAAAAGGATCTCAAGAAGATCCTTTGATCTTTTCTACGGGGTCTGACGCT
CAGTGGAACGAAAACTCACGTTAAGGGATTTTGGTCATGAGATTATCAAAAAGGATCTTCACCTAGATCCTTTTAAATTA
AAAATGAAGTTTTAAATCAATCTAAAGTATATATGAGTAAACTTGGTCTGACAGTTACCAATGCTTAATCAGTGAGGCAC
CTATCTCAGCGATCTGTCTATTTCGTTCATCCATAGTTGCCTGACTC
SEQ ID NO:8
   pWRG/AND-M(x) sequence 
GGGGGGGGGGGCGCTGAGGTCTGCCTCGTGAAGAAGGTGTTGCTGACTCATACCAGGCCTGAATCGCCCCATCATCCAG
CCAGAAAGTGAGGGAGCCACGGTTGATGAGAGCTTTGTTGTAGGTGGACCAGTTGGTGATTTTGAACTTTTGCTTTGCCA
CGGAACGGTCTGCGTTGTCGGGAAGATGCGTGATCTGATCCTTCAACTCAGCAAAAGTTCGATTTATTCAACAAAGCCGC
CGTCCCGTCAAGTCAGCGTAATGCTCTGCCAGTGTTACAACCAATTAACCAATTCTGATTAGAAAAACTCATCGAGCATC
AAATGAAACTGCAATTTATTCATATCAGGATTATCAATACCATATTTTTGAAAAAGCCGTTTCTGTAATGAAGGAGAAAA
CTCACCGAGGCAGTTCCATAGGATGGCAAGATCCTGGTATCGGTCTGCGATTCCGACTCGTCCAACATCAATACAACCTA
TTAATTTCCCCTCGTCAAAAATAAGGTTATCAAGTGAGAAATCACCATGAGTGACGACTGAATCCGGTGAGAATGGCAAA
AGCTTATGCATTTCTTTCCAGACTTGTTCAACAGGCCAGCCATTACGCTCGTCATCAAAATCACTCGCATCAACCAAACC
GTTATTCATTCGTGATTGCGCCTGAGCGAGACGAAATACGCGATCGCTGTTAAAAGGACAATTACAAACAGGAATCGAAT
GCAACCGGCGCAGGAACACTGCCAGCGCATCAACAATATTTTCACCTGAATCAGGATATTCTTCTAATACCTGGAATGCT
GTTTTCCCGGGGATCGCAGTGGTGAGTAACCATGCATCATCAGGAGTACGGATAAAATGCTTGATGGTCGGAAGAGGCAT
AAATTCCGTCAGCCAGTTTAGTCTGACCATCTCATCTGTAACATCATTGGCAACGCTACCTTTGCCATGTTTCAGAAACA
ACTCTGGCGCATCGGGCTTCCCATACAATCGATAGATTGTCGCACCTGATTGCCCGACATTATCGCGAGCCCATTTATAC
CCATATAAATCAGCATCCATGTTGGAATTTAATCGCGGCCTCGAGCAAGACGTTTCCCGTTGAATATGGCTCATAACACC
CCTTGTATTACTGTTTATGTAAGCAGACAGTTTTATTGTTCATGATGATATATTTTTATCTTGTGCAATGTAACATCAGA
GATTTTGAGACACAACGTGGCTTTCCCCCCCCCCCCGGCATGCCTGCAGGTCGACAATATTGGCTATTGGCCATTGCATA
CGTTGTATCTATATCATAATATGTACATTTATATTGGCTCATGTCCAATATGACCGCCATGTTGACATTGATTATTGACT
AGTTATTAATAGTAATCAATTACGGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTACATCAAGTGTATCATATGCCA
AGTCCGCCCCCTATTGACGTCAATGACGGTAAATGGCCCGCCTGGCATTATGCCCAGTACATGACCTTACGGGACTTTCC
TACTTGGCAGTACATCTACGTATTAGTCATCGCTATTACCATGGTGATGCGGTTTTGGCAGTACACCAATGGGCGTGGAT
AGCGGTTTGACTCACGGGGATTTCCAAGTCTCCACCCCATTGACGTCAATGGGAGTTTGTTTTGGCACCAAAATCAACGG
 
GACTTTCCAAAATGTCGTAATAACCCCGCCCCGTTGACGCAAATGGGCGGTAGGCGTGTACGGTGGGAGGTCTATATAAG
CAGAGCTCGTTTAGTGAACCGTCAGATCGCCTGGAGACGCCATCCACGCTGTTTTGACCTCCATAGAAGACACCGGGACC
GATCCAGCCTCCGCGGCCGGGAACGGTGCATTGGAACGCGGATTCCCCGTGCCAAGAGTGACGTAAGTACCGCCTATAGA
CTCTATAGGCACACCCCTTTGGCTCTTATGCATGCTATACTGTTTTTGGCTTGGGGCCTATACACCCCCGCTTCCTTATG
CTATAGGTGATGGTATAGCTTAGCCTATAGGTGTGGGTTATTGACCATTATTGACCACTCCCCTATTGGTGACGATACTT
TCCATTACTAATCCATAACATGGCTCTTTGCCACAACTATCTCTATTGGCTATATGCCAATACTCTGTCCTTCAGAGACT
GACACGGACTCTGTATTTTTACAGGATGGGGTCCCATTTATTATTTACAAATTCACATATACAACAACGCCGTCCCCCGT
GCCCGCAGTTTTTATTAAACATAGCGTGGGATCTCCACGCGAATCTCGGGTACGTGTTCCGGACATGGGCTCTTCTCCGG
TAGCGGCGGAGCTTCCACATCCGAGCCCTGGTCCCATGCCTCCAGCGGCTCATGGTCGCTCGGCAGCTCCTTGCTCCTAA
CAGTGGAGGCCAGACTTAGGCACAGCACAATGCCCACCACCACCAGTGTGCCGCACAAGGCCGTGGCGGTAGGGTATGTG
TCTGAAAATGAGCTCGGAGATTGGGCTCGCACCGCTGACGCAGATGGAAGACTTAAGGCAGCGGCAGAAGAAGATGCAGG
CAGCTGAGTTGTTGTATTCTGATAAGAGTCAGAGGTAACTCCCGTTGCGGTGCTGTTAACGGTGGAGGGCAGTGTAGTCT
GAGCAGTACTCGTTGCTGCCGCGCGCGCCACCAGACATAATAGCTGACAGACTAACAGACTGTTCCTTTCCATGGGTCTT
TTCTGCAGTCACCGTCCAAGCTTGCGGCCGCGGATCTGCAGGAATTCGGCACGAGAGTAGTAGACTCCGCACGAAGAAGC
AAAAAATTAAAGAAGTGAGTTTAAAATGGAAGGGTGGTATCTGGTTGTTCTTGGAGTCTGCTATACGCTGACACTGGCAA
TGCCCAAGACCATTTATGAGCTTAAAATGGAATGCCCGCACACTGTGGGTCTCGGTCAAGGTTACATCATTGGCTCAACA
GAACTAGGTTTGATCTCAATTGAGGCTGCATCTGATATAAAGCTCGAGAGCTCTTGCAATTTTGATCTTCATACAACATC
TATGGCCCAGAAGAGTTTCACCCAAGTTGAATGGAGAAAGAAAAGTGACACAACTGATACCACAAATGCTGCGTCCACTA
CCTTTGAAGCACAAACTAAAACTGTTAACCTTAGAGGGACTTGTATACTGGCACCTGAACTCTATGATACATTGAAGAAA
GTAAAAAAGACAGTCCTGTGCTATGATCTAACATGTAATCAAACACATTGTCAGCCAACTGTCTATCTGATTGCACCTGT
ATTGACATGCATGTCAATAAGAAGTTGTATGGCTAGTGTGTTTACAAGCAGGATTCAGGTGATTTATGAAAAGACACATT
GTGTAACAGGTCAGCTGATTGAGGGTCAGTGTTTCAACCCAGCACACACATTGACATTATCTCAGCCTGCTCACACTTAT
GATACTGTCACCCTTCCTATCTCTTGTTTTTCACACCAAAGAAGTCGGAGCAACTAAAAGTTATAAAAACATTTGAAGG
AATTCTGACGAAGACAGGTTGCACGGAGAATGCATTGCAGGGTTATTATGTGTGTTTTTTAGGGAGTCATTCAGAACCTT
TAATTGTTCCGAGTTTGGAGGACATACGGTCTGCTGAAGTTGTTAGTAGGATGCTTGTACACCCTAGGGGAGAAGACCAT
GATGCCATACAGAATTCACAAAGTCACTTAAGAATAGTGGACCTATCACAGCAAAAGTGCCATCAACTAGTTCCACAGA
TACCCTAAAGGGGACAGCCTTTGCAGGCGTCCCAATGTATAGCTCTTTATCTACACTAGTCAGAAATGCAGACCCAGAAT
TTGTATTTTCTCCAGGTATAGTACCTGAATCTAATCACAGTACATGTGATAAGAAGACAGTACCTATCACATGGACAGGC
TACCTACCAATATCAGGTGAGATGGAAAAAGTGACTGGATGTACAGTTTTTTGTACACTAGCAGGACCTGGTGCTAGTTG
TGAGGCCTATTCTGAAAATGGTATATTTAACATCAGTTCTCCAACATGTCTTGTAAACAAAGTCCAAAGATTTCGTGGAT
CTGAACAGAAAATAAATTTTATCTGTCAGCGGGTAGATCAGGATGTTGTTGTATACTGCAATGGGCAAAAGAAAGTCATA
TTAACCAAAACTTTGGTTATTGGGCAGTGTATTTATACATTCACAAGCCTATTTTCATTGATGCCTGATGTAGCCCACTC
ATTGGCTGTAGAATTATGTGTCCCGGGATTACATGGGTGGGCCACTGTCATGCTTCTATCAACATTCTGCTTTGGTGGG
TCTTGATTCCTGCGGTCACATTAATAATATTAAAGTGTCTAAGGGTTTTGACGTTTTCTTGTTCCCATTACACTAATGAG
TCAAAATTTAAATTCATCCTGGAAAAAGTTAAAATTGAATACCAAAAGACTATGGGATCAATGGTGTGCGATGTATGTCA
TCATGAGTGTGAAACAGCAAAAGAACTTGAATCACATAGACAGAGTTGTATCAATGGACAATGTCCTTATTGCATGACAA
TAACTGAAGCAACTGAAAGTGCCTTGCAAGCCCATTATTCCATTTGTAAATTGGCAGGAAGATTTCAGGAGGCACTGAAA
AAGTCACTTAAAAAGCCAGAGGTAAAAAAAGGTTGTTACAGAACACTCGGGGTATTTAGATATAAAAGTAGATGTTATGT
GGGTTTGGTATGGTGCCTATTGTTGACATGTGAAATTGTTATTTGGCCGCAAGTGCAGAGACTCCACTAATGGAGTCAG
GCTGGTCAGATACGGCTCATGGTGTTGGTGAGATTCCAATGAAGACAGACCTCGAGCTGGACTTTTCACTGCCTTCTTCA
TCCTCTTACAGTTATAGGAGAAAGCTCACAAACCCAGCCAATAAAGAAGAGTCTATTCCCTTCCACTTCCAGATGGAAAA
ACAAGTAATTCATGCTGAAATCCAACCCCTGGGTCATTGGATGGATGCGACATTTAATATTAAGACTGCATTTCATTGTT
ATGGTGCATGCCAGAAATACTCTTATCCATGGCAGACATCTAAGTGCTTCTTTGAAAAGGACTACCAGTATGAAACAGGC
TGGGGCTGTAATCCTGGTGACTGCCCAGGGGTTGGGACTGGATGCACTGCTTGTGGTGTTTATCTCGATAAACTAAAATC
TGTTGGGAAGGCCTATAAGATAATTTCTTTAAAATATACCAGAAAGGTTTGTATTCAGTTAGGAACAGAACAAACTTGCA
AGCATATTGATGCAAATGATTGTTTAGTGACACCATCTGTGAAAGTTTGCATAGTGGGCACAGTTTCAAAACTTCAACCA
TCTGATACTCTTTTGTTCTTAGGTCCACTAGAACAAGGGGGAATCATTCTTAAGCAATGGTGCACAACATCATGTGCATT
TGGGGACCCTGGTGATATCATGTCCACTCCCAGTGGTATGAGGTGTCCAGAGCACACTGGATCATTTAGGAAAATTTGCG
GTTTTGCTACTACACCAGTTTGTGAATATCAAGGAAATACCATTTCTGGATATAAAAGAATGATGGCAACAAAAGATTCA
TTCCAATCATTTAACTTAACAGAACCTCACATCACAACAAACAAGCTTGAATGGATCGACCCAGATGGGAATACAAGAGA
CCACGTAAACCTTGTCTTAAATAGAGATGTCTCATTTCAGGATTTAAGTGATAACCCCTGTAAAGTAGACCTACACACAC
AAGCAATAGAAGGGGCATGGGGTTCTGGTGTAGGGTTTACACTCACATGTACTGTCGGATTAACAGAGTGCCCAAGTTTT
ATGACATCAATTAAGGCATGTGACCTAGCTATGTGTTATGGATCAACAGTAACAAACCTTGCCAGGGGCTCTAATACAGT
GAAAGTAGTTGGTAAAGGAGGCCATTCAGGGTCCTCATTTAAATGCTGTCATGATACAGATTGCTCCTCTGAAGGTTTAC
TTGCATCAGCCCCTCATCTTGAGAGGGTAACAGGATTCAATCAAATTGATTCAGATAAGGTTTATGATGATGGTGCACCA
CCTTGCACATTCAAATGCTGGTTCACTAAGTCAGGTGAGTGGCTTCTTGGGATCTTAAACGGAATTGGATTGTTGTTGT
AGTGCTTGTTGTGATACTCATTCTCTCTATCATAATGTTCAGTGTTTTGTGTCCCAGGAGAGGGCACAAGAAAACTGTCT
AAGCATTGACCTCAACTCCTACATTAGATCATATACATTTATGCACTTCCTCATATTTAGCTGCACTAAGATATTAATAA
 
ACTCTAGTTATTGACTTTATAAGATTATTATGGAACTAACCTCACTTAAAAAAACAAATACTTTACTCATATATAACTC
CATATTCTCTTACCGAGGATTTTGTTCCTGCGGAGCATACTACTAGGATCTACGTATGATCAGCCTCGACTGTGCCTTCT
AGTTGCCAGCCATCTGTTGTTTGCCCCTCCCCCGTGCCTTCCTTGACCCTGGAAGGTGCCACTCCCACTGTCCTTTCCTA
ATAAAATGAGGAAATTGCATCGCATTGTCTGAGTAGGTGTCATTCTATTCTGGGGGGTGGGGTGGGGCAGGACAGCAAGG
GGGAGGATTGGGAAGACAATAGCAGGCATGCTGGGGATGCGGTGGGCTCTATGGCTTCTGAGGCGGAAAGAACCAGCTGG
GGCTCGACAGCTCGACTCTAGAATTGCTTCCTCGCTCACTGACTCGCTGCGCTCGGTCGTTCGGCTGCGGCGAGCGGTAT
CAGCTCACTCAAAGGCGGTAATACGGTTATCCACAGAATCAGGGGATAACGCAGGAAAGAACATGTGAGCAAAAGGCCAG
CAAAAGGCCAGGAACCGTAAAAAGGCCGCGTTGCTGGCGTTTTTCCATAGGCTCCGCCCCCCTGACGAGCATCACAAAAA
TCGACG[?]TCAAGTCAGAGGTGGCGAAACCCGACAGGACTATAAAGATACCAGGCGTTTCCCCCTGGAAGCTCCCTCGTGC
GCTCTCCTGTTCCGACCCTGCCGCTTACCGGATACCTGTCCGCCTTTCTCCCTTCGGGAAGCGTGGCGCTTTCTCATAGC
TCACGCTGTAGGTATCTCAGTTCGGTGTAGGTCGTTCGCTCCAAGCTGGGCTGTGTGCACGAACCCCCCGTTCAGCCCGA
CCGCTGCGCCTTATCCGGTAACTATCGTCTTGAGTCCAACCCGGTAAGACACGACTTATCGCCACTGGCAGCAGCCACTG
GTAACAGGATTAGCAGAGCGAGGTATGTAGGCGGTGCTACAGAGTTCTTGAAGTGGTGGCCTAACTACGGCTACACTAGA
AGAACAGTATTTGGTATCTGCGCTCTGCTGAAGCCAGTTACCTTCGGAAAAAGAGTTGGTAGCTCTTGATCCGGCAAACA
AACCACCGCTGGTAGCGGTGGTTTTTTTGTTTGCAAGCAGCAGATTACGCGCAGAAAAAAAGGATCTCAAGAAGATCCTT
TGATCTTTTCTACGGGGTCTGACGCTCAGTGGAACGAAAACTCACGTTAAGGGATTTTGGTCATGAGATTATCAAAAAGG
ATCTTCACCTAGATCCTTTTAAATTAAAAATGAAGTTTTAAATCAATCTAAAGTATATATGAGTAAACTTGGTCTGACAG
TTACCAATGCTTAATCAGTGAGGCACCTATCTCAGCGATCTGTCTATTTCGTTCATCCATAGTTGCCTGACTC
SEQ ID NO:9
   pWRG/HA-M sequence 
GGGGGGGGGGGCGCTGAGGTCTGCCTCGTGAAGAAGGTGTTGCTGACTCATACCAGGCCTGAATCGCCCCATCATCCAG
CCAGAAAGTGAGGGAGCCACGGTTGATGAGAGCTTTGTTGTAGGTGGACCAGTTGGTGATTTTGAACTTTTGCTTTGCCA
CGGAACGGTCTGCGTTGTCGGGAAGATGCGTGATCTGATCCTTCAACTCAGCAAAAGTTCGATTTATTCAACAAAGCCGC
CGTCCCGTCAAGTCAGCGTAATGCTCTGCCAGTGTTACAACCAATTAACCAATTCTGATTAGAAAAACTCATCGAGCATC
AAATGAAACTGCAATTTATTCATATCAGGATTATCAATACCATATTTTTGAAAAAGCCGTTTCTGTAATGAAGGAGAAAA
CTCACCGAGGCAGTTCCATAGGATGGCAAGATCCTGGTATCGGTCTGCGATTCCGACTCGTCCAACATCAATACAACCTA
TTAATTTCCCCTCGTCAAAAATAAGGTTATCAAGTGAGAAATCACCATGAGTGACGACTGAATCCGGTGAGAATGGCAAA
AGCTTATGCATTTCTTTCCAGACTTGTTCAACAGGCCAGCCATTACGCTCGTCATCAAAATCACTCGCATCAACCAAACC
GTTATTCATTCGTGATTGCGCCTGAGCGAGACGAAATACGCGATCGCTGTTAAAAGGACAATTACAAACAGGAATCGAAT
GCAACCGGCGCAGGAACACTGCCAGCGCATCAACAATATTTTCACCTGAATCAGGATATTCTTCTAATACCTGGAATGCT
GTTTTCCCGGGGATCGCAGTGGTGAGTAACCATGCATCATCAGGAGTACGGATAAAATGCTTGATGGTCGGAAGAGGCAT
AAATTCCGTCAGCCAGTTTAGTCTGACCATCTCATCTGTAACATCATTGGCAACGCTACCTTTGCCATGTTTCAGAAACA
ACTCTGGCGCATCGGGCTTCCCATACAATCGATAGATTGTCGCACCTGATTGCCCGACATTATCGCGAGCCCATTTATAC
CCATATAAATCAGCATCCATGTTGGAATTTAATCGCGGCCTCGAGCAAGACGTTTCCCGTTGAATATGGCTCATAACACC
CCTTGTATTACTGTTTATGTAAGCAGACAGTTTTATTGTTCATGATGATATATTTTTATCTTGTGCAATGTAACATCAGA
GATTTTGAGACACAACGTGGCTTTCCCCCCCCCCCCGGCATGCCTGCAGGTCGACAATATTGGCTATTGGCCATTGCATA
CGTTGTATCTATATCATAATATGTACATTTATATTGGCTCATGTCCAATATGACCGCCATGTTGACATTGATTATTGACT
AGTTATTAATAGTAATCAATTACGGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTACATCAAGTGTATCATATGCCA
AGTCCGCCCCCTATTGACGTCAATGACGGTAAATGGCCCGCCTGGCATTATGCCCAGTACATGACCTTACGGGACTTTCC
TACTTGGCAGTACATCTACGTATTAGTCATCGCTATTACCATGGTGATGCGGTTTTGGCAGTACACCAATGGGCGTGGAT
AGCGGTTTGACTCACGGGGATTTCCAAGTCTCCACCCCATTGACGTCAATGGGAGTTTGTTTTGGCACCAAAATCAACGG
GACTTTCCAAAATGTCGTAATAACCCCGCCCCGTTGACGCAAATGGGCGGTAGGCGTGTACGGTGGGAGGTCTATATAAG
CAGAGCTCGTTTAGTGAACCGTCAGATCGCCTGGAGACGCCATCCACGCTGTTTTGACCTCCATAGAAGACACCGGGACC
GATCCAGCCTCCGCGGCCGGGAACGGTGCATTGGAACGCGGATTCCCCGTGCCAAGAGTGACGTAAGTACCGCCTATAGA
CTCTATAGGCACACCCCTTTGGCTCTTATGCATGCTATACTGTTTTTGGCTTGGGGCCTATACACCCCCGCTTCCTTATG
CTATAGGTGATGGTATAGCTTAGCCTATAGGTGTGGGTTATTGACCATTATTGACCACTCCCCTATTGGTGACGATACTT
TCCATTACTAATCCATAACATGGCTCTTTGCCACAACTATCTCTATTGGCTATATGCCAATACTCTGTCCTTCAGAGACT
GACACGGACTCTGTATTTTTACAGGATGGGGTCCCATTTATTATTTACAAATTCACATATACAACAACGCCGTCCCCCGT
GCCCGCAGTTTTTATTAAACATAGCGTGGGATCTCCACGCGAATCTCGGGTACGTGTTCCGGACATGGGCTCTTCTCCGG
TAGCGGCGGAGCTTCCACATCCGAGCCCTGGTCCCATGCCTCCAGCGGCTCATGGTCGCTCGGCAGCTCCTTGCTCCTAA
CAGTGGAGGCCAGACTTAGGCACAGCACAATGCCCACCACCACCAGTGTGCCGCACAAGGCCGTGGCGGTAGGGTATGTG
TCTGAAAATGAGCTCGGAGATTGGGCTCGCACCGCTGACGCAGATGGAAGACTTAAGGCAGCGGCAGAAGAAGATGCAGG
CAGCTGAGTTGTTGTATTCTGATAAGAGTCAGAGGTAACTCCCGTTGCGGTGCTGTTAACGGTGGAGGGCAGTGTAGTCT
 
GAGCAGTACTCGTTGCTGCCGCGCGCGCCACCAGACATAATAGCTGACAGACTAACAGACTGTTCCTTTCCATGGGTCTT
TTCTGCAGTCACCGTCCAAGCTTGCGGCCGCGGATCTGCAGGAATTCGGCACGAGAGTAGTAGACTCCGCAAGAAACAGC
AGTCAATCAGCAACATGGGATATGGAAGTGGCTAGTGATGGCCAGTTTAGTATGGCCTGTTTTGACACTGAGAAATGTC
TATGACATGAAAATTGAGTGCCCCCATACAGTAAGTTTTGGGAAAACAGTGTGATAGGTTATGTAGAATTACCCCCCGT
GCCATTGGCCGACACAGCACAGATGGTGCCTGAGAGTTCTTGTAGCATGGATAATCACCAATCGTTGAATACAATAACAA
AATATACCCAAGTAAGTTGGAGAGGAAAGGCTGATCAGTCACAGTCTAGTCAAAATTCATTTGAGACAGTGTCCACTGAA
GTTGACTTGAAAGGAACATGTGCTCTAAAACACAAAATGGTGGAAGAATCATACCGTAGTAGGAAATCAGTAACCTGTTA
CGACCTGTCTTGCAATAGCACTTACTGCAAGCCAACACTATACATGATTGTACCAATTCATGCATGCAATATGATGAAAA
GCTGTTTGATTGCATTGGGACCATACAGAGTACAGGTGGTTTATGAGAGATCTTATTGCATGACAGGAGTCCTGATTGAA
GGGAAATGCTTTGTCCCAGATCAAAGTGTGGTCAGTATTATCAAGCATGGGATCTTTGATATTGCAAGTGTTCATATTGT
ATGTTTCTTTGTTGCAGTTAAAGGGAATACTTATAAAATTTTTGAACAGGTTAAGAAATCCTTTGAATCAACATGCAATG
ATACAGAGAATAAAGTGCAAGGATATTATATTTGTATTGTAGGGGGAAACTCTGCACCAATATATGTTCCAACACTTGAT
GATTTCAGATCCATGGAAGCATTTACAGGAATCTTCAGATCACCACATGGGGAAGATCATGATCTGGCTGGAGAAGAAAT
TGCATCTTATTCTATAGTCGGACCTGCCAATGCAAAAGTTCCTCATAGTGCTAGCTCAGATACATTGAGCTTGATTGCCT
ATTCAGGTATACCATCTTATTCTTCCCTTAGCATCCTAACAAGTTCAACAGAAGCTAAGCATGTATTCAGCCCTGGGTTG
TTCCCAAAACTTAATCACACAAATTGTGATAAAAGTGCCATACCACTCATATGGACTGGGATGATTGATTTACCTGGATA
CTACGAAGCTGTCCACCCTTGTACAGTTTTTTGCGTATTATCAGGTCCTGGGGCATCATGTGAAGCCTTTTCTGAAGGCG
GGATTTTCAACATAACCTCTCCCATGTGCTTAGTGTCAAAACAAAATCGATTCCGGTTAACAGAACAGCAAGTGAATTTT
GTGTGTCAGCGAGTGGACATGGACATTGTTGTGTACTGCAACGGGCAGAGGAAAGTAATATTAACAAAAACTCTAGTTAT
TGGACAGTGTATATATACTATAACAAGCTTATTCTCATTACTACCTGGAGTAGCACATTCTATTGCTGTTGAATTGTGTG
TACCTGGGTTCCATGGTTGGCCACAGCTGCTCTGCTTGTTACATTCTGTTTCGGATGGGTTCTTATACCAGCAATTACA
TTTATCATACTAACAGTCCTAAAGTTCATTGCTAATATTTTTCACACAAGTAATCAAGAGAATAGGCTAAAATCAGTACT
TAGAAAGATAAAGGAAGAGTTTGAAAAAACAAAAGGCTCAATGGTATGTGATGTCTGCAAGTATGAGTGTGAAACCTATA
AAGAATTAAAGGCACACGGGGTATCATGCCCCCAATCTCAATGTCCTTACTGTTTTACTCATTGTGAACCCACAGAAGCA
GCATTCCAAGCTCATTACAAGGTATGCCAAGTTACTCACAGATTCAGGGATGATCTAAAGAAAACTGTTACTCCTCAAAA
TTTTACACCAGGATGTTACCGGACACTAAATTTATTTAGATACAAAAGCAGGTGCTACATCTTTACAATGTGGATATTTC
TTCTTGTCTTAGAATCCATACTGTGGGCTGCAAGTGCATCAGAGACACCATTAACTCCTGTCTGGAATGACAATGCCCAT
GGGGTAGGTTCTGTTCCTATGCATACAGATTTAGAGCTTGATTTCTCTTTAACATCCAGTTCCAAGTATACATACCGTAG
GAAGTTAACAAACCCACTTGAGGAAGCACAATCCATTGACCTACATATTGAAATAGAAGAACAGACAATTGGTGTTGATG
TGCATGCTCTAGGACACTGGTTTGATGGTCGTCTTAACCTTAAAACATCCTTTCACTGTTATGGTGCTTGTACAAAGTAT
GAATACCCTTGGCATACTGCAAAGTGCCACTATGAAAGAGATTACCAATATGAGACGAGCTGGGGTTGTAATCCATCAGA
TTGTCCTGGGGTGGGCACAGGCTGTACAGCATGTGGTTTATACCTAGATCAACTGAAACCAGTTGGTAGTGCTTATAAAA
TTATCACAATAAGGTACAGCAGGAGAGTCTGTGTTCAGTTTGGGGAGGAAAACCTTTGTAAGATAATAGACATGAATGAT
TGTTTTGTATCTAGGCATGTTAAGGTCTGCATAATTGGTACAGTATCTAAATTCTCTCAGGGTGATACCTTATTGTTTTT
TGGACCGCTTGAAGGTGGTGGTCTAATATTTAAACACTGGTGTACATCCACATGTCAATTTGGTGACCCAGGAGATATCA
TGAGTCCAAGAGACAAAGGTTTTTTATGCCCTGAGTTTCCAGGTAGTTTCAGGAAGAAATGCAACTTTGCTACTACCCCT
ATTTGTGAGTATGATGGAAATATGGTCTCAGGTTACAAGAAAGTGATGGCGACAATTGATTCCTTCCAATCTTTTAATAC
AAGCACTATGCACTTCACTGATGAAAGGATAGAGTGGAAAGACCCTGATGGAATGCTAAGGGACCATATAAACATTTTAG
TAACGAAGGACATTGACTTTGATAACCTTGGTGAAAATCCTTGCAAAATTGGCCTACAAACATCTTCTATTGAGGGGGCC
TGGGGTTCTGGTGTGGGGTTCACATTAACATGTCTGGTATCACTAACAGAATGTCCTACCTTTTTGACCTCAATAAAGGC
TTGTGATAAGGCTATCTGTTATGGTGCAGAGAGTGTAACATTGACAAGAGGACAAAATACAGTCAAGGTATCAGGGAAAG
GTGGCCATAGTGGTTCAACATTTAGGTGTTGCCATGGGGAGGACTGTTCACAAATTGGACTCCATGCTGCTGCACCTCAC
CTTGACAAGGTAAATGGGATTTCTGAGATAGAAAATAGTAAAGTATATGATGATGGGGCACCGCAATGTGGGATAAAATG
TTGGTTTGTTAAATCAGGGGAATGGATTTCAGGGATATTCAGTGGTAATTGGATTGTACTCATTGTCCTCTGTGTATTTC
TATTGTTCTCCTTGGTTTTACTAAGCATTCTCTGTCCCGTAAGGAAGCATAAAAAATCATAGCTAAATTCTGTGACTATC
CTGTTCTTATGTATAGCTTTAACATATATACTAATTTTTATATTCCAGTATACTCTATCTAACACACTAAAAAAAATAGT
AGCTTTCTAACCACAAAACGGATCTACGTATGATCAGCCTCGACTGTGCCTTCTAGTTGCCAGCCATCTGTTGTTTGCCC
CTCCCCCGTGCCTTCCTTGACCCTGGAAGGTGCCACTCCCACTGTCCTTTCCTAATAAAATGAGGAAATTGCATCGCATT
GTCTGAGTAGGTGTCATTCTATTCTGGGGGGTGGGGTGGGGCAGGACAGCAAGGGGGAGGATTGGGAAGACAATAGCAGG
CATGCTGGGGATGCGGTGGGCTCTATGGCTTCTGAGGCGGAAAGAACCAGCTGGGGCTCGACAGCTCGACtctagagcag
gtcgacaatattggctatTGGCCATTGCATACGTTGTATCTATATCATAATATGTACATTTATATTGGCTCATGTCCAAT
ATGACCGCCATGTTGACATTGATTATTGACTAGTTATTAATAGTAATCAATTACGGGGTCATTAGTTCATAGCCCATATA
TGGAGTTCCGCGTTACATAACTTACGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTGGAGTATTTACGGTAAACTGCCCA
CTTGGCAGTACATCAAGTGTATCATATGCCAAGTCCGCCCCCTATTGACGTCAATGACGGTAAATGGCCCGCCTGGCATT
ATGCCCAGTACATGACCTTACGGGACTTTCCTACTTGGCAGTACATCTACGTATTAGTCATCGCTATTACCATGGTGATG
CGGTTTTGGCAGTACACCAATGGGCGTGGATAGCGGTTTGACTCACGGGGATTTCCAAGTCTCCACCCCATTGACGTCAA
TGGGAGTTTGTTTTGGCACCAAAATCAACGGGACTTTCCAAAATGTCGTAATAACCCCGCCCCGTTGACGCAAATGGGCG
 
GTAGGCGTGTACGGTGGAGGTCTATATAAGCAGAGCTCGTTTAGTGAACCGTCAGATCGCCTGGAGACGCCATCCACGC
TGTTTTGACCTCCATAGAAGACACCGGGACCGATCCAGCCTCCGCGGCCGGGAACGGTGCATTGGAACGCGGATTCCCCG
TGCCAAGAGTGACGTAAGTACCGCCTATAGACTCTATAGGCACACCCCTTTGGCTCTTATGCATGCTATACTGTTTTTGG
CTTGGGGCCTATACACCCCCGCTTCCTTATGCTATAGGTGATGGTATAGCTTAGCCTATAGGTGTGGGTTATTGACCATT
ATTGACCACTCCCCTATTGGTGACGATACTTTCCATTACTAATCCATAACATGGCTCTTTGCCACAACTATCTCTATTGG
CTATATGCCAATACTCTGTCCTTCAGAGACTGACACGGACTCTGTATTTTTACAGGATGGGGTCCCATTTATTATTTACA
AATTCACATATACAACAACGCCGTCCCCCGTGCCCGCAGTTTTTATTAAACATAGCGTGGGATCTCCACGCGAATCTCGG
GTACGTGTTCCGGACATGGGCTCTTCTCCGGTAGCGGCGGAGCTTCCACATCCGAGCCCTGGTCCCATGCCTCCAGCGGC
TCATGGTCGCTCGGCAGCTCCTTGCTCCTAACAGTGGAGGCCAGACTTAGGCACAGCACAATGCCCACCACCACCAGTGT
GCCGCACAAGGCCGTGGCGGTAGGGTATGTGTCTGAAAATGAGCTCGGAGATTGGGCTCGCACCGCTGACGCAGATGGAA
GACTTAAGGCAGCGGCAGAAGAAGATGCAGGCAGCTGAGTTGTTGTATTCTGATAAGAGTCAGAGGTAACTCCCGTTGCG
GTGCTGTTAACGGTGGAGGGCAGTGTAGTCTGAGCAGTACTCGTTGCTGCCGCGCGCGCCACCAGACATAATAGCTGACA
GACTAACAGACTGTTCCTTTCCATGGGTCTTTTCTGCAGTCACCGTCCAAGCTTGCGGCCGCGGATCTGCAGGAATTCGG
CACGAGAGTAGTAGACTCCGCACGAAGAAGCAAAAAATTAAAGAAGTGAGTTTAAAATGGAAGGGTGGTATCTGGTTGTT
CTTGGAGTCTGCTATACGCTGACACTGGCAATGCCCAAGACCATTTATGAGCTTAAAATGGAATGCCCGCACACTGTGGG
TCTCGGTCAAGGTTACATCATTGGCTCAACAGAACTAGGTTTGATCTCAATTGAGGCTGCATCTGATATAAAGCTCGAGA
GCTCTTGCAATTTTGATCTTCATACAACATCTATGGCCCAGAAGAGTTTCACCCAAGTTGAATGGAGAAAGAAAAGTGAC
ACAACTGATACCACAAATGCTGCGTCCACTACCTTTGAAGCACAAACTAAAACTGTTAACCTTAGAGGGACTTGTATACT
GGCACCTGAACTCTATGATACATTGAAGAAAGTAAAAAAGACAGTCCTGTGCTATGATCTAACATGTAATCAAACACATT
GTCAGCCAACTGTCTATCTGATTGCACCTGTATTGACATGCATGTCAATAAGAAGTTGTATGGCTAGTGTGTTTACAAGC
AGGATTCAGGTGATTTATGAAAAGACACATTGTGTAACAGGTCAGCTGATTGAGGGTCAGTGTTTCAACCCAGCACACAC
ATTGACATTATCTCAGCCTGCTCACACTTATGATACTGTCACCCTTCCTATCTCTTGTTTTTCACACCAAAGAAGTCGG
AGCAACTAAAAGTTATAAAAACATTTGAAGGAATTCTGACGAAGACAGGTTGCACGGAGAATGCATTGCAGGGTTATTAT
GTGTGTTTTTTAGGAAGTCATTCAGAACCTTTAATTGTTCCGAGTTTGGAGGACATACGGTCTGCTGAAGTTGTTAGTAG
GATGCTTGTACACCCTAGGGGAGAAGACCATGATGCCATACAGAATTCACAAAGTCACTTAAGAATAGTGGGACCTATCA
CAGCAAAAGTGCCATCAACTAGTTCCACAGATACCCTAAAGGGGACAGCCTTTGCAGGCGTCCCAATGTATAGCTCTTTA
TCTACACTAGTCAGAAATGCAGACCCAGAATTTGTATTTTCTCCAGGTATAGTACCTGAATCTAATCACAGTACATGTGA
TAAGAAGACAGTACCTATCACATGGACAGGCTACCTACCAATATCAGGTGAGATGGAAAAAGTGACTGGATGTACAGTTT
TTTGTACACTAGCAGGACCTGGTGCTAGTTGTGAGGCCTATTCTGAAAATGGTATATTTAACATCAGTTCTCCAACATGT
CTTGTAAACAAAGTCCAAAGATTTCGTGGATCTGAACAGAAAATAAATTTTATCTGTCAGCGGGTAGATCAGGATGTTGT
TGTATACTGCAATGGGCAAAAGAAAGTCATATTAACCAAAACTTTGGTTATTGGGCAGTGTATTTATACATTCACAAGCC
TATTTTCATTGATGCCTGATGTAGCCCACTCATTGGCTGTAGAATTATGTGTCCCGGGATTACATGGGTGGGCCACTGTC
ATGCTTCTATCAACATTCTGCTTTGGGTGGGTCTTGATTCCTGCGGTCACATTAATAATATTAAAGTGTCTAAGGGTTTT
GACGTTTTCTTGTTCCCATTACACTAATGAGTCAAAATTTAAATTCATCCTGGAAAAAGTTAAAATTGAATACCAAAAGA
CTATGGGATCAATGGTGTGCGATGTATGTCATCATGAGTGTGAAACAGCAAAAGAACTTGAATCACATAGACAGAGTTGT
ATCAATGGACAATGTCCTTATTGCATGACAATAACTGAAGCAACTGAAAGTGCCTTGCAAGCCCATTATTCCATTTGTAA
ATTGGCAGGAAGATTTCAGGAGGCACTGAAAAAGTCACTTAAAAAGCCAGAGGTAAAAAAAGGTTGTTACAGAACACTCG
GGGTATTTAGATATAAAAGTAGATGTTATGTGGGTTTGGTATGGTGCCTATTGTTGACATGTGAAATTGTTATTTGGGCC
GCAAGTGCAGAGACTCCACTAATGGAGTCAGGCTGGTCAGATACGGCTCATGGTGTTGGTGAGATTCCAATGAAGACAGA
CCTCGAGCTGGACTTTTCACTGCCTTCTTCATCCTCTTACAGTTATAGGAGAAAGCTCACAAACCCAGCCAATAAAGAAG
AGTCTATTCCCTTCCACTTCCAGATGGAAAAACAAGTAATTCATGCTGAAATCCAACCCCTGGGTCATTGGATGGATGCG
ACATTTAATATTAAGACTGCATTTCATTGTTATGGTGCATGCCAGAAATACTCTTATCCATGGCAGACATCTAAGTGCTT
CTTTGAAAAGGACTACCAGTATGAAACAGGCTGGGGCTGTAATCCTGGTGACTGCCCAGGGGTTGGGACTGGATGCACTG
CTTGTGGTGTTTATCTCGATAAACTAAAATCTGTTGGGAAGGCCTATAAGATAATTTCTTTAAAATATACCAGAAAGGTT
TGTATTCAGTTAGGAACAGAACAAACTTGCAAGCATATTGATGCAAATGATTGTTTAGTGACACCATCTGTGAAAGTTTG
CATAGTGGGCACAGTTTCAAAACTTCAACCATCTGATACTCTTTTGTTCTTAGGTCCACTAGAACAAGGGGGAATCATTC
TTAAGCAATGGTGCACAACATCATGTGCATTTGGGGACCCTGGTGATATCATGTCCACTCCCAGTGGTATGAGGTGTCCA
GAGCACACTGGATCATTTAGGAAAATTTGCGGTTTTGCTACTACACCAGTTTGTGAATATCAAGGAAATACCATTTCTGG
ATATAAAAGAATGATGGCAACAAAAGATTCATTCCAATCATTTAACTTAACAGAACCTCACATCACAACAAACAAGCTTG
AATGGATCGACCCAGATGGGAATACAAGAGACCACGTAAACCTTGTCTTAAATAGAGATGTCTCATTTCAGGATTTAAGT
GATAACCCCTGTAAAGTAGACCTACACACACAAGCAATAGAAGGGGCATGGGGTTCTGGTGTAGGGTTTACACTCACATG
TACTGTCGGATTAACAGAGTGCCCAAGTTTTATGACATCAATTAAGGCATGTGACCTAGCTATGTGTTATGGATCAACAG
TAACAAACCTTGCCAGGGGCTCTAATACAGTGAAAGTAGTTGGTAAAGGAGGCCATTCAGGGTCCTCATTTAAATGCTGT
CATGATACAGATTGCTCCTCTGAAGGTTTACTTGCATCAGCCCCTCATCTTGAGAGGGTAACAGGATTCAATCAAATTGA
TTCAGATAAGGTTTATGATGATGGTGCACCACCTTGCACATTCAAATGCTGGTTCACTAAGTCAGGTGAGTGGCTTCTTG
GGATCTTAAACGGGAATTGGATTGTTGTTGTAGTGCTTGTTGTGATACTCATTCTCTCTATCATAATGTTCAGTGTTTTG
TGTCCCAGGAGAGGGCACAAGAAAACTGTCTAAGCATTGACCTCAACTCCTACATTAGATCATATACATTTATGCACTTC
CTCATATTTAGCTGCACTAAGATATTAATAAACTCTAGTTATTGACTTTATAAGATTATTATGGAACTAACCTCACTTAA
 
AAAAAACAAATACTTTACTCATATATAACTCCATATTCTCTTACCGAGGATTTGTTCCTGCGGAGCATACTACTAGGAT
CTACGTATGATCAGCCTCGACTGTGCCTTCTAGTTGCCAGCCATCTGTTGTTTGCCCCTCCCCCGTGCCTTCCTTGACCC
TGGAAGGTGCCACTCCCACTGTCCTTTCCTAATAAAATGAGGAAATTGCATCGCATTGTCTGAGTAGGTGTCATTCTATT
CTGGGGGGTGGGGTGGGGCAGGACAGCAAGGGGGAGGATTGGGAAGACAATAGCAGGCATGCTGGGGATGCGGTGGGCTC
TATGGCTTCTGAGGCGGAAAGAACCAGCTGGGGCTCGACAGCTCGACtctagaATTGCTTCCTCGCTCACTGACTCGCTG
CGCTCGGTCGTTCGGCTGCGGCGAGCGGTATCAGCTCACTCAAAGGCGGTAATACGGTTATCCACAGAATCAGGGGATAA
CGCAGGAAAGAACATGTGAGCAAAAGGCCAGCAAAAGGCCAGGAACCGTAAAAAGGCCGCGTTGCTGGCGTTTTTCCATA
GGCTCCGCCCCCCTGACGAGCATCACAAAAATCGACGCTCAAGTCAGAGGTGGCGAAACCCGACAGGACTATAAAGATAC
CAGGCGTTTCCCCCTGGAAGCTCCCTCGTGCGCTCTCCTGTTCCGACCCTGCCGCTTACCGGATACCTGTCCGCCTTTCT
CCCTTCGGGAAGCGTGGCGCTTTCTCATAGCTCACGCTGTAGGTATCTCAGTTCGGTGTAGGTCGTTCGCTCCAAGCTGG
GCTGTGTGCACGAACCCCCCGTTCAGCCCGACCGCTGCGCCTTATCCGGTAACTATCGTCTTGAGTCCAACCCGGTAAGA
CACGACTTATCGCCACTGGCAGCAGCCACTGGTAACAGGATTAGCAGAGCGAGGTATGTAGGCGGTGCTACAGAGTTCTT
GAAGTGGTGGCCTAACTACGGCTACACTAGAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGTGGTTTTTTTGTTTGCAAGCAGCAGATTACG
CGCAGAAAAAAAGGATCTCAAGAAGATCCTTTGATCTTTTCTACGGGGTCTGACGCTCAGTGGAACGAAAACTCACGTTA
AGGGATTTTGGTCATGAGATTATCAAAAAGGATCTTCACCTAGATCCTTTTAAATTAAAAATGAAGTTTTAAATCAATCT
AAAGTATATATGAGTAAACTTGGTCTGACAGTTACCAATGCTTAATCAGTGAGGCACCTATCTCAGCGATCTGTCTATTT
CGTTCATCCATAGTTGCCTGACTC
SEQ ID NO:10
   primer0,
   5'-GCGCGCGGCCGCAGTAGTAGACTCCGCAAGAAAC
SEQ ID NO:11
   BACK,
   5'-GCGCGGATCCCGGGTACCGGGCCCCCCCTCG
SEQ ID NO:12 
primer1-24,
5' GGCCGCGGCCGCGGATCTGCAGGAATTCGGCACGAGAGTAGTAGACTCC
GCAAGAAACAGCA
SEQ ID NO:13 
primer24-1,
5'-GGCCGCGGCCGCGAGCACGGCTTAAGGACGTCTAGGAGTAGTAGTC
TCCGCAAGAAACAGCA
SEQ ID NO:14 
primer1-12,
5'-GGCCGCGGCCGCATTCGGCACGAGAGTAGTAGACTCCGCAAGAA
ACAGCA
SEQ ID NO:15 
primer13-24,
5' GGCCGCGGCCGCGGATCTGCAGGAAGTAGTAGACT
CCGCAAGAAACAGCA
SEQ ID NO:16 
primer1-24*,
5' GGCCGCGGCCGCGGATCTGCCCGAATTCGGCACGAGAGTAGTAGACTC
CGCAAGAAACAGCA
SEQ ID NO:17
   primer1-8,
   5'GGCCGCGGCCGCGGATCTGCAGTAGTAGACTCCGCAAGAAACAGCA
SEQ ID NO:18
   SEOMX,
   5'-GCGCGGATCCAGATTGGGAGATAGAAGAGAG
SEQ ID NO:19
   M5B,
   5'-TCAGGACTCCTGTCATGCAATAAGATCTC
SEQ ID NO:20
   HTNMX,
   5'-GCGCGGATCCGTTTGTGGTTAGAAAGCTAC
SEQ ID NO:21 
SN-Fj,
5'-GGCCGCGGCCGCGGATCTGCAGGAATTCGGCACGAGAGTAG
TAGACTCCGCACGAAGAAGC
SEQ ID NO:22
   PUUM-R,
   5'-GCGCGGATCCTAGTAGTATGCTCCGCAGGAAC

## Claims

1. An isolated nucleotide sequence selected from the group consisting of:
nucleotides 1-8 of SEQ ID NO. 6,
nucleotides 1-12 of SEQ ID NO. 6, and
nucleotides 1-24 of SEQ ID NO. 6.

2. A DNA fragment which encodes a Hantaan hantavirus (HTNV) M gene segment and the sequence according to claim 1.

3. A recombinant DNA construct comprising:
(i) a vector,
(ii) at least one hantavirus Hantaan M gene nucleic acid fragment, and
(iii) the nucleotide sequence according to claim 1.

4. A construct according to claim 3 wherein said construct is pWRG/HTN-M(x) set forth in SEQ ID NO:7.

5. A construct according to claim 3, wherein said construct comprises two said hantavirus M gene nucleic acid fragments.

6. A construct according to claim 5, wherein said construct is pWRG/HA-M set forth in SEQ NO. 9.

7. An immunogenic composition against hantavirus infection, comprising a nucleic acid comprising a promoter operative in the cells of a mammal, the nucleotide sequence according to claim 1, and a hantavirus polynucleotide M segment encoding G1 and G2 from the Hantaan hantavirus and, optionally, further hantaviruses.

8. A composition according to claim 7, further comprising inert particles, the nucleic acid being coated onto the inert particles producing nucleic acid coated particles.

9. A composition according to claim 8, wherein said further hantavirus is chosen from the group consisting of Seoul virus (SEOV), Dobrava virus (DOBV), Pumuula virus (PUUV), Sin Nombre virus (SNV), Black Creek Canal virus (BCCV), Bayou virus (BAYV), New York virus (NYV), Andes virus (ANDV), and Laguna Negra virus (LNV).

10. A composition according to claim 9 wherein said further hantavirus is SEOV.

11. A composition according to claim 7, wherein said nucleic acid is the construct according to claim 4 (pWRG/HTN-M(x) set forth in SEQ ID NO:7).

12. A composition according to claim 9, wherein said further hantavirus is ANDV.

13. A composition according to claim 7, wherein the nucleic acid comprises M segment from two hantaviruses.

14. A composition according to claim 13, wherein said two hantaviruses are HTNV and ANDV.

15. A composition according to claim 14 wherein said nucleic acid is the construct according to claim 6 (pWRG/HA-M, as set forth in SEQ ID NO:9).

16. A vaccine against hantavirus infection comprising the construct according to any of claims 3-6 or a composition according to any of claims 7-15.

17. A vaccine according to claim 16, the vaccine being against infection with SEOV, DOBV, and HTNV hantavirus, said vaccine comprising the composition according to any of claims 8, 9 or 10.

18. A vaccine according to claim 16, the vaccine being against infection with SEOV, HTNV, DOBV, ANDV, SNV, and BCCV, said vaccine comprising the composition of claim 15.

19. A vaccine according to claim 16, which is a multivalent vaccine for protection against infection with more than one hemorrhagic fever with renal syndrome (HFRS) hantavirus, the vaccine comprising a composition of matter comprising a nucleic acid comprising the nucleotide sequence according to claim 1 and two or more hantavirus M segments from different hantaviruses, at least one of which is a Hantaan hantavirus, each M segment encoding G1 and G2 from its respective hantavirus, operatively linked to a promoter active in cells of a mammal.

20. A vaccine according to claim 19, wherein the nucleic acid is coated onto an inert particle.

21. A vaccine according to claim 20, comprising two or more inert particles, each said particle having the nucleic acid coated thereon.

22. A multivalent vaccine according to claims 19 to 21, wherein said hantaviruses are chosen from the group consisting of HTNV, DOBV, PUUV and SEOV.

23. A vaccine according to claim 16, which is a multivalent vaccine for protection against infection with more than one HFRS and hantavirus pulmonary syndrome (HPS) hantavirus comprising a composition of matter comprising two or more nucleic acids, each said nucleic acids comprising the nucleotide sequence according to claim 1 and a hantavirus M segment encoding G 1 and G2 and operatively linked to a promoter active in cells of a mammal, wherein said M segment is chosen from different hantaviruses including at least one HPS-associated hantavirus and at least one HFRS-associated hantavirus, at least one of which is a Hantaan hantavirus.

24. A vaccine according to claim 23, comprising an inert particle having two or more of said nucleic acids coated thereon.

25. A vaccine according to claim 24, comprising two or more inert particles, each said particle having the nucleic acid coated thereon.

26. A multivalent vaccine according to any of claims 23-25, wherein said hantaviruses are chosen from the group consisting of ANDV, HTNV, and SEOV.

27. A nucleotide sequence according to claim 1 for use in inducing a protective immune response against Hantaan hantavirus infection in a mammal.

28. A nucleotide sequence according to claim 27, wherein the nucleotide sequence is comprised within the DNA fragment of claim 2.

29. A nucleotide sequence according to claim 27, wherein the nucleotide sequence is comprised within the recombinant DNA construct according to any of claims 3-6.

30. A nucleotide sequence according to claim 27, wherein the nucleotide sequence is comprised within the of composition according to any of claims 8-15.

31. A nucleotide sequence according to claim 27, wherein the nucleotide sequence is comprised within the vaccine according to any of claims 16-26.

32. A nucleotide sequence for use according to any of claims 27-31, where in the protective immune response is induced by physically accelerating the composition into epidermal cells of the mammal *in vivo* such that said nucleic acid is expressed.

## Patentansprüche

1. Isolierte Nukleotidsequenz, die aus der Gruppe ausgewählt ist, bestehend aus:
Nukleotiden 1-8 von SEQ ID NO: 6,
Nukleotiden 1-12 von SEQ ID NO: 6 und
Nukleotiden 1-24 von SEQ ID NO: 6.

2. DNA-Fragment, das für ein M-Gensegment des Hantavirus Hantaan (HTNV) und die Sequenz nach Anspruch 1 kodiert.

3. Rekombinantes DNA-Konstrukt, umfassend:
(i) einen Vektor,
(ii) mindestens ein Nukleinsäurefragment des Hantavirus Hantaan M-Gen und
(iii) die Nukleotidsequenz nach Anspruch 1.

4. Konstrukt nach Anspruch 3, wobei das in SEQ ID NO: 7 dargelegte Konstrukt pWRG/HTN-M(x) ist.

5. Konstrukt nach Anspruch 3, wobei das Konstrukt zwei Nukleinsäurefragmente des Hantavirus M-Gen umfasst.

6. Konstrukt nach Anspruch 5, wobei das in SEQ ID NO: 9 dargelegte Konstrukt pWRG/HA-M ist.

7. Immunogene Zusammensetzung gegen eine Hantavirus-Infektion, umfassend eine Nukleinsäure, die einen in den Zellen eines Säugers operativen Promotor, die Nukleotidsequenz nach Anspruch 1 und ein M-Segment des Hantavirus-Polynukleotids, das für G1 und G2 aus dem Hantavirus Hantaan kodiert, und gegebenenfalls weitere Hantaviren umfasst.

8. Zusammensetzung nach Anspruch 7, ferner umfassend inerte Partikel, wobei die Nukleinsäure auf die inerten Partikel geschichtet ist, wobei mit Nukleinsäure beschichtete Partikel hergestellt werden.

9. Zusammensetzung nach Anspruch 8, wobei das weitere Hantavirus aus der Gruppe ausgewählt ist, bestehend aus dem Seoul-Virus (SEOV), Dobrava-Virus (DOBV), Pumuula-Virus (PUUV), Sin Nombre-Virus (SNV), Black Creek Canal-Virus (BCCV), Bayou-Virus (BAYV), New York-Virus (NYV), Andes-Virus (ANDV) und Laguna Negra-Virus (LNV).

10. Zusammensetzung nach Anspruch 9, wobei das weitere Hantavirus SEOV ist.

11. Zusammensetzung nach Anspruch 7, wobei die Nukleinsäure das Konstrukt nach Anspruch 4 (pWRG/HTN-M(x), dargelegt in SEQ ID NO: 7) ist.

12. Zusammensetzung nach Anspruch 9, wobei das weitere Hantavirus ANDV ist.

13. Zusammensetzung nach Anspruch 7, wobei die Nukleinsäure das M-Segment aus zwei Hantaviren umfasst.

14. Zusammensetzung nach Anspruch 13, wobei die beiden Hantaviren HTNV und ANDV sind.

15. Zusammensetzung nach Anspruch 14, wobei die Nukleinsäure das Konstrukt nach Anspruch 6 (pWRG/HA-M, wie in SEQ ID NO: 9 dargelegt) ist.

16. Impfstoff gegen eine Hantavirus-Infektion, der das Konstrukt nach einem der Ansprüche 3-6 oder eine Zusammensetzung nach einem der Ansprüche 7-15 umfasst.

17. Impfstoff nach Anspruch 16, wobei der Impfstoff gegen eine Infektion mit dem Hantavirus SEOV, DOBV und HTNV ist, wobei der Impfstoff die Zusammensetzung nach einem der Ansprüche 8, 9 oder 10 umfasst.

18. Impfstoff nach Anspruch 16, wobei der Impfstoff gegen eine Infektion mit SEOV, HTNV, DOBV, ANDV, SNV und BCCV ist, wobei der Impfstoff die Zusammensetzung von Anspruch 15 umfasst.

19. Impfstoff nach Anspruch 16, bei dem es sich um einen multivalenten Impfstoff zum Schutz gegen eine Infektion mit mehr als einem hämorrhagisches Fieber mit renalem Syndrom (HFRS) verursachenden Hantavirus handelt, wobei der Impfstoff eine Zusammensetzung aus einem Stoff umfasst, umfassend eine Nukleinsäure, umfassend die Nukleotidsequenz nach Anspruch 1 und zwei oder mehr Hantavirus M-Segmente von verschiedenen Hantaviren, von denen mindestens eines ein Hantavirus Hantaan ist, wobei jedes M-Segment für G 1 and G2 aus seinem entsprechenden Hantavirus kodiert, das operativ mit einem in den Zellen eines Säugers aktiven Promotor verknüpft ist.

20. Impfstoff nach Anspruch 19, wobei die Nukleinsäure auf ein inertes Partikel geschichtet ist.

21. Impfstoff nach Anspruch 20, umfassend zwei oder mehr inerte Partikel, wobei jedes Partikel mit der Nukleinsäure darauf beschichtet ist.

22. Multivalenter Impfstoff nach den Ansprüchen 19 bis 21, wobei die Hantaviren aus der Gruppe ausgewählt sind, bestehend aus HTNV, DOBV, PUUV und SEOV.

23. Impfstoff nach Anspruch 16, bei dem es sich um einen multivalenten Impfstoff zum Schutz gegen eine Infektion mit mehr als einem HFRS und Hantavirus pulmonales Syndrom (HPS) verursachenden Hantavirus handelt, umfassend eine Zusammensetzung aus einem Stoff, umfassend zwei oder mehr Nukleinsäuren, wobei jede Nukleinsäure die Nukleotidsequenz nach Anspruch 1 und ein Hantavirus M-Segment umfasst, das für G1 and G2 kodiert und mit einem in den Zellen eines Säugers aktiven Promotor operativ verknüpft ist, wobei das M-Segment aus verschiedenen Hantaviren ausgewählt ist, die mindestens ein HPS-assoziiertes Hantavirus und mindestens ein HFRS-assoziiertes Hantavirus einschließen, von denen mindestens eines ein Hantavirus Hantaan ist.

24. Impfstoff nach Anspruch 23, umfassend ein inertes Partikel mit zwei oder mehr der darauf geschichteten Nukleinsäuren.

25. Impfstoff nach Anspruch 24, umfassend zwei oder mehr inerte Partikel, wobei jedes Partikel mit der Nukleinsäure darauf beschichtet ist.

26. Multivalenter Impfstoff nach einem der Ansprüche 23-25, wobei die Hantaviren aus der Gruppe ausgewählt sind, bestehend aus ANDV, HTNV und SEOV.

27. Nukleotidsequenz nach Anspruch 1 zur Verwendung bei der Induktion einer schützenden Immunantwort gegen eine Infektion mit dem Hantavirus Hantaan in einem Säuger.

28. Nukleotidsequenz nach Anspruch 27, wobei sich die Nukleotidsequenz im DNA-Fragment von Anspruch 2 befindet.

29. Nukleotidsequenz nach Anspruch 27, wobei sich die Nukleotidsequenz im rekombinanten DNA-Konstrukt nach einem der Ansprüche 3-6 befindet.

30. Nukleotidsequenz nach Anspruch 27, wobei sich die Nukleotidsequenz in der Zusammensetzung nach einem der Ansprüche 8-15 befindet.

31. Nukleotidsequenz nach Anspruch 27, wobei sich die Nukleotidsequenz im Impfstoff nach einem der Ansprüche 16-26 befindet.

32. Nukleotidsequenz zur Verwendung nach einem der Ansprüche 27-31, wobei die schützende Immunantwort durch physikalische Beschleunigung der Zusammensetzung in die Epidermalzellen des Säugers in vivo dergestalt induziert wird, dass die Nukleinsäure exprimiert wird.

## Revendications

1. Séquence de nucléotides isolée choisie dans le groupe constitué de:
nucléotides 1-8 de la SEQ ID NO. 6,
nucléotides 1-12 de la SEQ ID NO. 6, et
nucléotides 1-24 de la SEQ ID NO. 6.

2. Fragment d'ADN qui code pour un segment de gène M d'hantavirus Hantaan (HTNV) et la séquence selon la revendication 1.

3. Construction d'ADN recombinant comprenant:
(i) un vecteur,
(ii) au moins un fragment d'acide nucléique de gène M d'hantavirus Hantaan, et
(iii) la séquence de nucléotides selon la revendication 1.

4. Construction selon la revendication 3, où ladite construction est pWRG/HTN-M(x) présentée dans la SEQ ID NO:7.

5. Construction selon la revendication 3, où ladite construction comprend deux desdits fragments d'acide nucléique de gène M d'hantavirus.

6. Construction selon la revendication 5, où ladite construction est pWRG/HA-M présentée dans la SEQ ID NO:9.

7. Composition immunogène contre une infection d'hantavirus, comprenant un acide nucléique comprenant un promoteur fonctionnant dans les cellules d'un mammifère, la séquence de nucléotides selon la revendication 1 et un segment M polynucléotidique d'hantavirus codant pour G1 et G2 provenant de l'hantavirus Hantaan et, éventuellement, d'hantavirus supplémentaires.

8. Composition selon la revendication 7, comprenant en outre des particules inertes, l'acide nucléique étant revêtu sur les particules inertes produisant des particules revêtues d'acide nucléique.

9. Composition selon la revendication 8, dans laquelle ledit hantavirus supplémentaire est choisi dans le groupe constitué du virus de Séoul (SEOV), du virus de Dobrava (DOBV), du virus de Pumuula (PUUV), du virus de Sin Nombre (SNV), du virus de Black Creek Canal (BCCV), du virus de Bayou (BAYV), du virus de New York (NYV), du virus d'Andes (ANDV) et du virus de Laguna Negra (LNV).

10. Composition selon la revendication 9, dans laquelle ledit hantavirus supplémentaire est SEOV.

11. Composition selon la revendication 7, dans laquelle ledit acide nucléique est la construction selon la revendication 4 (pWRG/HTN-M(x) présentée dans la SEQ ID NO:7).

12. Composition selon la revendication 9, dans laquelle ledit hantavirus supplémentaire est ANDV.

13. Composition selon la revendication 7, dans laquelle l'acide nucléique comprend un segment M provenant de deux hantavirus.

14. Composition selon la revendication 13, dans laquelle lesdits deux hantavirus sont HTNV et ANDV.

15. Composition selon la revendication 14, dans laquelle ledit acide nucléique est la construction selon la revendication 6 (pWRG/HA-M présentée dans la SEQ ID NO:9).

16. Vaccin contre une infection d'hantavirus comprenant la construction selon l'une quelconque des revendications 3-6 ou une composition selon l'une quelconque des revendications 7-15.

17. Vaccin selon la revendication 16, le vaccin étant contre une infection avec les hantavirus SEOV, DOBV et HTNV, ledit vaccin comprenant la composition selon l'une quelconque des revendications 8, 9 ou 10.

18. Vaccin selon la revendication 16, le vaccin étant contre une infection avec les hantavirus SEOV, HTNV, DOBV, ANDV, SNV et BCCV, ledit vaccin comprenant la composition selon la revendication 15.

19. Vaccin selon la revendication 16, qui est un vaccin multivalent pour une protection contre une infection avec plus d'un hantavirus de fièvre hémorragique avec un syndrome rénal (HFRS), le vaccin comprenant une composition de matière comprenant un acide nucléique comprenant la séquence de nucléotides selon la revendication 1 et deux ou plus segments M d'hantavirus provenant de différents hantavirus, dont au moins un est un hantavirus Hantaan, chaque segment M codant pour G1 et G2 provenant de son hantavirus respectif, lié de manière fonctionnelle à un promoteur actif dans des cellules d'un mammifère.

20. Vaccin selon la revendication 19, dans lequel l'acide nucléique est revêtu sur une particule inerte.

21. Vaccin selon la revendication 20, comprenant deux ou plus particules inertes, chacune desdites particules ayant l'acide nucléique revêtu sur celle-ci.

22. Vaccin multivalent selon les revendications 19 à 21, dans lequel lesdits hantavirus sont choisis dans le groupe constitué de HTNV, DOBV, PUUV et SEOV.

23. Vaccin selon la revendication 16, qui est un vaccin multivalent pour une protection contre une infection avec plus d'un HFRS et hantavirus de syndrome pulmonaire d'hantavirus (HPS) comprenant une composition de matière comprenant deux ou plus acides nucléiques, chacun desdits acides nucléiques comprenant la séquence de nucléotides selon la revendication 1 et un segment M d'hantavirus codant pour G1 et G2 et lié de manière fonctionnelle à un promoteur actif dans des cellules d'un mammifère, dans lequel ledit segment M est choisi parmi différents hantavirus incluant au moins un hantavirus associé à HPS et au moins un hantavirus associé à HFRS, dont au moins un est un hantavirus Hantaan.

24. Vaccin selon la revendication 23, comprenant une particule inerte ayant deux ou plus desdits acides nucléiques revêtus sur celle-ci.

25. Vaccin selon la revendication 24, comprenant deux ou plus particules inertes, chacune desdites particules ayant l'acide nucléique revêtu sur celle-ci.

26. Vaccin multivalent selon l'une quelconque des revendications 23-25, dans lequel lesdits hantavirus sont choisis dans le groupe constitué de ANDV, HTNV et SEOV.

27. Séquence de nucléotides selon la revendication 1 pour une utilisation dans l'induction d'une réponse immunitaire protectrice contre une infection d'hantavirus Hantaan chez un mammifère.

28. Séquence de nucléotides selon la revendication 27, où la séquence de nucléotides est comprise dans le fragment d'ADN selon la revendication 2.

29. Séquence de nucléotides selon la revendication 27, où la séquence de nucléotides est comprise dans la construction d'ADN recombinant selon l'une quelconque des revendications 3-6.

30. Séquence de nucléotides selon la revendication 27, où la séquence de nucléotides est comprise dans la composition selon l'une quelconque des revendications 8-15.

31. Séquence de nucléotides selon la revendication 27, où la séquence de nucléotides est comprise dans le vaccin selon l'une quelconque des revendications 16-26.

32. Séquence de nucléotides pour une utilisation selon l'une quelconque des revendications 27-31, dans laquelle la réponse immunitaire protectrice est induite par une accélération physique de la composition dans des cellules épidermiques du mammifère *in vivo* de sorte que ledit acide nucléique est exprimé.
